# EUROPEAN PATENT SPECIFICATION

(11) **EP 3 225 691 B1**
(45) Date of publication and mention of the grant of the patent: **28.10.2020**
(21) Application number: 15863232.3
(22) Date of filing: 27.11.2015
(51) Int. Cl.: C12P 5/00, C12P 5/02, C12N 15/09, C08F 36/08

(54) **METHOD FOR PRODUCING ISOPRENOID COMPOUND**
VERFAHREN ZUR HERSTELLUNG EINER ISOPRENOIDVERBINDUNG
PROCÉDÉ DE PRODUCTION D'UN COMPOSÉ D'ISOPRÉNOÏDE

(30) Priority: 28.11.2014 RU 2014148144; 25.09.2015 RU 2015141007
(43) Date of publication of application: 04.10.2017
(73) Proprietor: Ajinomoto Co., Inc., Tokyo, 104-8315 (JP)
(72) Inventor: TAJIMA, Yoshinori, Kawasaki-shi Kanagawa 210-8681 (JP); RACHI, Hiroaki, Kawasaki-shi Kanagawa 210-8681 (JP); NISHIO, Yosuke, Kawasaki-shi Kanagawa 210-8681 (JP); KATASHKINA, Zhanna Iosifovna, Moscow 117545 (RU); OLEG, Valerievich Bylino, Moscow 117545 (RU)
(74) Representative: Grünecker Patent- und Rechtsanwälte PartG mbB
(86) International application number: PCT/JP2015/083498
(87) International publication number: WO 2016/084963

(56) References cited:
- WO-A1-2014/025941
- WO-A2-2010/003007
- WO-A2-2013/052914
- JP-A- 2005 270 115
- JP-A- 2005 528 106
- JP-A- 2011 526 790
- JP-A- 2012 500 648
- KEN'ICHI KAI ET AL: "Effective production of recombinant protein by using inducible pho promoter", SEIBUTSU KOGAKU KAISHI = JOURNAL OF THE SOCIETY FOR BIOSCIENCE AND BIOENGINEERING, JAPAN, NIPPON SEIBUTSU KOGAKKAI, SUITA, JP, vol. 71, no. 5, 1993, pages 317-323, XP009503208, ISSN: 0919-3758
- KEN'ICHI KAI ET AL.: 'Effective production of recombinant protein by using inducible pho promoter' JOURNAL OF THE SOCIETY FOR BIOSCIENCE AND BIOENGINEERING vol. 71, no. 5, 1993, JAPAN, pages 317 - 323, XP009503208

## Description

### TECHNICAL FIELD

The present invention relates to a method for producing an isoprenoid compound such as an isoprene monomer.

### BACKGROUND ART

Natural rubber is a very important raw material in the industries for production of tire and rubber. While its demand will be expanded in future due to motorization mainly in emerging countries, it is not easy to increase agricultural farms in view of regulation for deforestation and competition with palm plantations. Thus, it is difficult to anticipate a yield increase of the natural rubber, and the balance of the demand and supply of the natural rubber is predicted to become tight. Synthesized polyisoprene is available as a material in place of the natural rubber. Its raw material is an isoprene monomer (herein also referred to as "isoprene"). The isoprene (2-methyl-1,3-butadiene) is mainly obtained by extracting from a C5 fraction obtained by cracking of naphtha. However in recent years, with the use of light feed crackers, an amount of produced isoprene tends to decrease and its supply is concerned. Also in recent years, since variation of oil price influences greatly, it is requested to establish a system in which isoprene derived from non-oil sources is produced inexpensively in order to ensure the stable supply of isoprene.

Concerning such a request, a method in which isoprene is produced by a fermentation method using an inducer and a microorganism to which an ability to produce isoprene was given has been known (Patent Literature 1).

A method utilizing an environmental factor such as light or temperature in place of the inducer has been also known. For example, a method of producing isoprene utilizing the light (Non-Patent Literature 1) and a method of producing an objective protein (e.g., interferon-y, insulin) utilizing the temperature (Non-Patent Literature 2) have been known.

### PRIOR ART REFERENCES

### PATENT LITERATURES

Patent Literature 1: International Publication WO2009/076676

### NON-PATENT LITERATURES

Non-Patent Literature 1: Pia Lindberg, Sungsoon Park, Anastasios Melis, Metabolic Engineering 12 (2010): 70-79
Non-Patent Literature 2: Norma A Valdez-Cruz, Luis Caspeta, Néstor O Pérez, Octavio T Ramirez, Mauricio A Trujillo-Roldán, Microbial Cell Factories 2010, 9: 18
Non-Patent Literature 3: TL Sivy, R Fall, TN Rosenstiel, Biosci. Biotechnol. Biochem., Jan 2011; 75(12): 2376-83
Non-Patent Literature 4: Martin V, Pitera D, Withers S, Newman J, Keasling J, Nat. Biotechnol., 21, 796-802, 2003

WO 2010/003007 A2 discloses a process for producing isoprene. WO 2013/052914 A2 discloses a method for improving the efficiency of the production of isoprene. WO 2014/025941 A1 discloses a method for producing an isoprenoid compound.

### DISCLOSURE OF INVENTION

### PROBLEM TO BE SOLVED BY THE INVENTION

As described in Patent Literature 1 and Non-Patent Literatures 1 to 4, when isoprenoid compounds including isoprene are produced in a microorganism, a mevalonate (MVA) pathway or a methylerythritol (MEP) pathway is utilized. If a metabolic flux in the MVA pathway is increased in order to enhance a quantity of production of isoprenoid compounds, growth of a microorganism may be inhibited (Non-Patent Literatures 3 and 4). A method of separating a growth phase of the microorganism from a formation phase of a substance can be effective to avoid growth of the microorganism. A concept that a period for the growth of a microorganism is separated from a period for producing a substance has been known in the production of the substance by a fermentation method (Fermentation Handbook edited by Working Group for Fermentation and Metabolism, Japan Bioindustry Association, 2001). To accomplish this concept, a way to transfer from the growth phase of the microorganism to the formation phase of the substance and a way to continue to produce the substance in the formation phase of the substance are required. An example in which a microorganism that produces isoprene by the addition of an inducer is grown under a condition with no addition of the inducer and a sufficient amount of microbial cells are acquired followed by adding the inducer to form isoprene has been known in isoprene fermentation (Patent Literature 1). IPTG (Isopropyl β-D-1-thiogalactopyranoside), and tetracycline can be utilized as the inducer. When IPTG or tetracycline is present in a culture tank, a microorganism continues to have an activity to produce isoprene, but before long, IPTG or tetracycline is decomposed and consequently the microorganism becomes less able to keep the activity to produce isoprene. Thus, IPTG or tetracycline must be added continuously. However, these inducers are expensive and increase production cost of isoprene. Therefore, it is desirable to construct a production process not using the inducer.

A technique for inducing the production of isoprene by using a promoter, a transcription activity of which is increased under strong light and increasing light intensity has been reported in blue-green algae (Non-Patent Literature 1) as a method of transferring to the formation phase of isoprene without using the inducer. Microorganisms in which this method is available are limited to microorganisms having a metabolic switch function of a light response type and microorganisms that can perform photosynthesis.

As an example of producing a substance without using the inducer, which is the case different from isoprene, it has been reported that when a protein such as interferon-y or insulin is produced in *Escherichia coli* by the fermentation method, elevation of culture temperature (from 30°C to 42°C) enables the transfer from the growth phase of the microorganism to the formation phase of the protein (Non-Patent Literature 2). While this method is effective for the production of the protein, the culture temperature at 42°C differs from the temperature at which an optimal metabolic rate of *E. coli* is obtained thus reducing a consumption rate of a substrate such as glucose. If this technique is applied to the isoprene fermentation, a fermentation process uses two culture temperature conditions. It is required therefore to culture under a condition that differs from microbial cell growth and the optimal metabolic rate of a host at which isoprene fermentation is utilized. As a result, throughout fermentation production processes, it is concerned that isoprene productivity under the culture condition at high temperature is reduced compared with a fermentation production process in which the microorganism is cultured at temperature at which the optimal metabolic rate is obtained.

As described above, a method of transferring the microorganism to the formation phase of isoprene without using the inducer and a method of keeping the activity to produce the isoprene in the formation phase thereof has been required.

### MEANS FOR SOLVING PROBLEM

As a result of an extensive study for solving the above problem, the present inventors have found that an isoprenoid compound such as an isoprene can efficiently be formed by first growing an isoprenoid compound-forming microorganism under the sufficient concentration of a growth promoting agent and then reducing the concentration of the growth promoting agent. The present inventors have also found that the formation of the isoprenoid compound can be attained under a culture control condition where an oxygen consumption rate of a fermenting microorganism is nearly equal to an oxygen supply rate to a fermentation jar, and when phosphate is present at a certain concentration or below in a culture medium, and have completed the present invention.

The invention is defined by the following embodiments:
1. A method of producing an isoprenoid compound comprising:
   1) culturing an isoprenoid compound-forming microorganism in the presence of a growth promoting agent at a sufficient concentration to grow the isoprenoid compound-forming microorganism;
   2) decreasing the sufficient concentration of the growth promoting agent to induce formation of the isoprenoid compound by the isoprenoid compound-forming microorganism; and
   3) culturing the isoprenoid compound-forming microorganism to form the isoprenoid compound, wherein the isoprenoid compound-forming microorganism has an ability to form the isoprenoid compound depending on a promoter which is inversely dependent on the growth promoting agent, and wherein the isoprenoid compound-forming microorganism contains a gene encoding an isoprenoid compound-synthetic enzyme, wherein the gene is under the control of the promoter.
2. The method according to embodiment 1, wherein the growth promoting agent is oxygen.
3. The method according to embodiment 2, wherein the isoprenoid compound-forming microorganism is an aerobic microorganism.
4. The method according to any one of embodiments 2 or 3, wherein the promoter is a microaerobically inducible promoter.
5. The method according to embodiment 4, wherein the microaerobically inducible promoter is a promoter of the gene encoding a lactate dehydrogenase, or a promoter of the gene encoding an α-acetolactate decarboxylase.
6. The method according to any one of embodiments 1-5, wherein
   a) the isoprenoid compound is a monoterpene or
   b) the isoprenoid compound is a limonene or
   c) the isoprenoid compound is a linanool or
   d) the isoprenoid compound is an isoprene monomer.
7. A method of producing an isoprenoid compound, comprising:
   1') supplying oxygen into a liquid phase containing the isoprenoid compound-forming microorganism in a system comprising a gas phase and the liquid phase to grow the isoprenoid compound-forming microorganism in the presence of dissolved oxygen at a sufficient concentration;
   2') decreasing the dissolved oxygen concentration in the liquid phase to induce formation of the isoprenoid compound by the isoprenoid compound-forming microorganism in the liquid phase;
   3') culturing the isoprenoid compound-forming microorganism in the liquid phase to form the isoprenoid compound; and
   4') collecting the isoprenoid compound, wherein the isoprenoid compound-forming microorganism has an ability to form the isoprenoid compound depending on a promoter which is inversely dependent on the dissolved oxygen concentration, and wherein the isoprenoid compound-forming microorganism contains a gene encoding an isoprenoid compound-synthetic enzyme, wherein the gene is under the control of the promoter.
8. The method according to embodiment 7, wherein an oxygen concentration in the gas phase is controlled depending on the dissolved oxygen concentration in the liquid phase, and wherein the dissolved oxygen concentration in the liquid phase is decreased to avoid isoprene burst in the gas phase.
9. The method according to embodiment 7 or 8, wherein the dissolved oxygen concentration in the liquid phase is 0.34 ppm or less in 2') and 3').
10. The method according to embodiment 1, wherein the growth promoting agent is a phosphorus compound.
11. The method according to embodiment 10, wherein the promoter is a phosphorus deficiency-inducible promoter.
12. The method according to embodiment 11, wherein the phosphorus deficiency-inducible promoter is a promoter of the gene encoding an acid phosphatase, or a promoter of the gene encoding a phosphorus uptake carrier.
13. The method according to embodiment 11 or 12, wherein formation of the isoprenoid by the isoprenoid-forming microorganism is induced in the presence of a total phosphorus at concentration of 50 mg/L or less.
14. The method according to any one of embodiments 7 to 13, wherein the isoprenoid-forming rate in the induction phase is 600 ppm/vvm/h or less, preferably 80 ppm/vvm/h or less.
15. The method according to any one of embodiment 7 to 14, wherein the isoprenoid compound is an isoprene.
16. The method according to any one of embodiments 1 to 15, wherein the isoprenoid compound-forming microorganism has an ability to synthesize dimethylallyl diphosphate via a methylerythritol phosphate pathway.
17. The method according to any one of embodiments 1 to 16, wherein the isoprenoid compound-forming microorganism has the ability to synthesize dimethylallyl diphosphate via a mevalonate pathway.
18. The method according to any one of embodiments 1 to 17, wherein the isoprenoid compound-forming microorganism is a microorganism belonging to the family *Enterobacteriaceae.*
19. The method according to embodiment 18, wherein the isoprenoid compound-forming microorganism is a microorganism belonging to the genus *Pantoea, Enterobacter, or Escherichia.*
20. The method according to embodiment 19, wherein the isoprenoid compound-forming microorganism is *Pantoea ananatis, Enterobacter aerogenes or Escherichia coli.*
21. A method of producing an isoprene polymer comprising:
   (I) forming the isoprene monomer by the method according to any one of embodiments 6 d) to 20; and
   (II) polymerizing the isoprene monomer to form the isoprene polymer.
22. A method for producing a rubber composition, comprising:
   (A) preparing an isoprene polymer by the method of embodiment 21; and
   (B) mixing said isoprene polymer with one or more rubber composition components.
23. A method for producing a tire, comprising:
   (i) producing a rubber composition by the method of embodiment 22; and
   (ii) applying said rubber composition to manufacture a tire.

### EFFECT OF THE INVENTION

According to the present invention, which is defined in the claims, it is possible to produce an isoprenoid compound inexpensively by separating the growth phase of the microorganism and the formation phase of the isoprenoid compound without using an expensive inducer.

### BRIEF DESCRIPTION OF DRAWINGS

FIG. 1 indicates measurement with time of dissolved oxygen (DO) concentrations in cultivations of an arabinose-inducible isoprenoid compound-forming microorganism (GI08/Para) and microaerobically inducible isoprenoid compound-forming microorganism (GI08/Para);
FIG. 2 indicates growth (A) and amount of formed isoprene (B) (mg/batch) in cultivations of an arabinose-inducible isoprenoid compound-forming microorganism (GI08/Para) and a microaerobically inducible isoprenoid compound-forming microorganism (GI08/Para);
FIG. 3 indicates measurement with time of the dissolved oxygen (DO) concentrations in cultivation of microaerobically inducible isoprenoid compound-forming microorganisms (GI08/Para);
FIG. 4 indicates the growth (A) and amount of formed isoprene (B) (mg/batch) in cultivation of microaerobically inducible isoprenoid compound-forming microorganisms (GI08/Para) under various concentration conditions of dissolved oxygen;
FIG. 5 indicates a pAH162-Para-mvaES plasmid possessing an mvaES operon derived from *E. faecalis* under control of *E. coli* Para promoter and a repressor gene araC;
FIG. 6 indicates a map of pAH162-mvaES;
FIG. 7 indicates a plasmid for chromosome fixation of pAH162-MCS-mvaES.
FIG. 8 indicates a set of plasmids for chromosome fixation which possess an mvaES gene under transcription control of (A) P_{11dD}, (B) P_{phoC}, or (C) P_{pstS};
FIG. 9 indicates an outline for construction of a pAH162-λattL-KmR-λattR vector;
FIG. 10 indicates a pAH162-Ptac expression vector for chromosome fixation;
FIG. 11 indicates codon optimization in a KDyI operon obtained by chemical synthesis;
FIG. 12 indicates plasmids pAH162-Tc-Ptac-KDyI (A) and pAH162-Km-Ptac-KDyI (B) for chromosome fixation, which retain the KDyI operon with codon optimization;
FIG. 13 indicates a plasmid for chromosome fixation, which retains a mevalonate kinase gene derived from *M. paludicola;*
FIG. 14 indicates maps of genome modifications of (A) ΔampC::attBₚₕᵢ₈₀, (B) ΔampH::attBₚₕᵢ₈₀, and (C) Δcrt::attBₚₕᵢ₈₀;
FIG. 15 indicates maps of genome modifications of (A) Δcrt::pAH162-P_{tac}-mvk(X) and (B) Δcrt::P_{tac}-mvk(X) ;
FIG. 16 indicates maps of chromosome modifications of (A) ΔampH::pAH162-Km-P_{tac}-KDyI, (B) ΔampC::pAH162-Km-P_{tac}-KDyI and (C) ΔampC::P_{tac}-KDyI;
FIG. 17 indicates maps of chromosome modifications of (A) ΔampH::pAH162-Px-mvaES and (B) ΔampC::pAH162-Px-mvaES;
FIG. 18 indicates measurement with time of phosphorus concentrations in cultures of an arabinose inducible isoprenoid compound-forming microorganism (SWITCH-Para/ispSM) and a phosphorus deficient isoprenoid compound-forming microorganism (SWITCH-PphoC/ispSM, SWITCH-PpstS/ispSM) ;
FIG. 19 indicates growth (A) and amounts of produced isoprene (B) (mg/batch) in cultures of an arabinose inducible isoprenoid compound-forming microorganism (SWITCH-Para/ispSM) and a phosphorus deficient isoprenoid compound-forming microorganism (SWITCH-PphoC/ispSM, SWITCH-PpstS/ispSM);
FIG. 20 indicates isoprene concentrations (ppm) of fermentation gas in cultures of an arabinose inducible isoprenoid compound-forming microorganism (SWITCH-Para/ispSM) and a phosphorus deficient type isoprenoid compound-forming microorganism (SWITCH-PphoC/ispSM, SWITCH-PpstS/ispSM) ;
FIG. 21 indicates measurement with time of dissolved oxygen (DO) concentrations in cultures of the arabinose inducible isoprenoid compound-forming microorganism (SWITCH-Para/ispSM) and a microaerobically inducible isoprenoid compound-forming microorganism (SWITCH-lld/ispSM) ;
FIG. 22 indicates growth (A), amounts of formed isoprene (B) (mg/batch) and amounts of formed isoprene (C) (ppm) in cultures of an arabinose inducible isoprenoid compound-forming microorganism (SWITCH-Para/ispSM) and a microaerobically inducible isoprenoid compound-forming microorganism (SWITCH-lld/ispSM);
FIG. 23 indicates isoprene concentrations (ppm) of fermentation gas in cultures of an arabinose inducible isoprenoid compound-forming microorganism (SWITCH-Para/ispSM) and a microaerobically inducible isoprenoid compound-forming microorganism (SWITCH-lld/ispSM);
FIG. 24 indicates a burst limit of isoprene and a critical oxygen concentration in a gas phase (headspace) (extract from US8420360B2);
FIG. 25 indicates growth (A) and amounts of formed isoprene (B) (mg/batch) in cultures of a phosphorus deficient type isoprenoid compound-forming microorganism in which glucose dehydrogenase (gcd) gene is disrupted (SWITCH-PphoC Δgcd/ispSM); and
FIG. 26 indicates changes in accumulated gluconate in culture broth over time in a phosphorus deficient type isoprenoid compound-forming microorganism in which gcd gene is disrupted (SWITCH-PphoC Δgcd/ispSM).

### EMBODIMENTS FOR CARRYING OUT THE INVENTION

The present invention provides a method of producing an isoprenoid compound.

The isoprenoid compound includes one or more isoprene units which have the molecular formula (C₅H₈)ₙ. The precursor of the isoprene unit may be isopentenyl pyrophosphate or dimethylallyl pyrophosphate. More than 30,000 kinds of isoprenoid compounds have been identified and new compounds have been identified. Isoprenoids are also known as terpenoids. The difference between terpenes and terpenoids is that terpenes are hydrocarbons, whereas terpenoids may contain additional functional groups. Terpenes are classified by the number of isoprene units in the molecule: hemiterpenes (C5), monoterpenes (C10), sesquiterpenes (C15), diterpenes (C20), sesterterpenes (C25), triterpenes (C30), sesquarterpenes (C35), tetraterpenes (C40), polyterpenes, norisoprenoids, for example. Examples of monoterpenes include pinene, nerol, citral, camphor, menthol, limonene, and linalool. Examples of sesquiterpenes include nerolidol and farnesol. Examples of diterpenes include phytol and vitamin A1. Squalene is an example of a triterpene, and carotene (provitamin A1) is a tetraterpene (Nature Chemical Biology 2, 674 - 681 (2006), Nature Chemical Biology 5, 283 - 291 (2009) Nature Reviews Microbiology 3, 937-947 (2005), Adv Biochem Eng Biotechmol (DOI: 10.1007/10_2014_288). Preferably, the isoprenoid compound is an isoprene (monomer).

The method of the present invention comprises the following 1) to 3):
1) culturing an isoprenoid compound-forming microorganism in the presence of a growth promoting agent at a sufficient concentration to grow the isoprenoid compound-forming microorganism;
2) decreasing the concentration of the growth promoting agent to induce formation of the isoprenoid compound by the isoprene-forming microorganism; and
3) culturing the isoprenoid compound-forming microorganism to form the isoprenoid compound, wherein the isoprenoid compound-forming microorganism has an ability to form the isoprenoid compound depending on a promoter which is inversely dependent on the growth promoting agent, and wherein the isoprenoid compound-forming microorganism comprises a gene encoding an isoprenoid compound-synthetic enzyme wherein the gene is under control of the promoter.

IPP (isopentenyl diphosphate) or DMAPP (dimethylallyl diphosphate) that is a substrate of isoprene synthesis has been known to be a precursor of peptide glycan and an electron acceptor, such as menaquinone, and to be essential for growth of microorganisms (Fujisaki et al., J. Biochem., 1986; 99: 1137-1146). In the method of the present invention, in the light of efficient production of an isoprenoid compound, step 1) corresponding to a growth phase of a microorganism and step 3) corresponding to a formation phase of the isoprenoid compound are separated. The method also comprises step 2) corresponding to an induction phase of isoprenoid compound formation for transferring the growth phase of the microorganism to the formation phase of the isoprenoid compound.

In the present invention, the growth promoting agent refer to a factor essential for the growth of a microorganism or a factor having an activity of promoting the growth of the microorganism, which can be consumed by the microorganism, the consumption of which causes reduction of its amount in a culture medium, consequently lost or reduction of the growth of the microorganism. For example, when the growth promoting agent in a certain amount is used, a microorganism continues to grow until the growth promoting agent in that amount is consumed, but once the growth promoting agent is entirely consumed, the microorganism cannot grow or the growth rate can decrease. Therefore, the degree of the growth of the microorganism can be regulated by the growth promoting agent. Examples of such a growth promoting agent may include substances such as oxygen (gas); minerals such as ions of iron, magnesium, potassium and calcium; phosphorus compounds such as monophosphoric acid, diphosphoric acid and polyphosphoric acid, or salt thereof; nitrogen compounds such as ammonia, nitrate, nitrite, nitrogen (gas), and urea; sulfur compounds such as ammonium sulfate and thiosulfuric acid; and nutrients such as vitamins (e.g., vitamin A, vitamin D, vitamin E, vitamin K, vitamin B1, vitamin B2, vitamin B6, vitamin B12, niacin, pantothenic acid, biotin, ascorbic acid), and amino acids (e.g., alanine, arginine, asparagine, aspartic acid, cysteine, glutamine, glutamic acid, glycine, histidine, leucine, isoleucine, lysine, methionine, phenylalanine, proline, serine, threonine, tryptophan, tyrosine, valine, selenocysteine). One growth promoting agent may be used or two or more growth promoting agents may be used in combination in the method of the present invention.

In the present invention, the isoprenoid compound-forming microorganism refer to a microorganism having an ability to grow depending on the growth promoting agent and an ability to form an isoprenoid compound depending on a promoter which is inversely dependent on the growth promoting agent, to which an ability to synthesize the isoprenoid compound by an enzymatic reaction has been given. The isoprenoid compound-forming microorganism can grow in the presence of the growth promoting agent at concentration sufficient for the growth of the isoprenoid compound-forming microorganism. Here, the "sufficient concentration" can refer to that the growth promoting agent is used at concentration which is effective for the growth of the isoprenoid compound-forming microorganism. The expression "ability to form an (the) isoprenoid compound depending on a promoter which is inversely depending on the growth promoting agent" can mean that the isoprenoid compound cannot be formed or a forming efficiency of the isoprenoid compound is low in the presence of the growth promoting agent at relatively high concentration whereas the isoprenoid compound can be formed or the forming efficiency of the isoprenoid compound is high in the presence of the growth promoting agent at relatively low concentration or in the absence of the growth promoting agent. Therefore, the isoprenoid compound-forming microorganism used in the present invention can grow well but cannot form the isoprenoid compound or exhibits low forming efficiency of the isoprenoid compound in the presence of the growth promoting agent at sufficient concentration. The isoprenoid compound-forming microorganism cannot grow well but can form the isoprenoid compound and exhibits high forming efficiency of the isoprenoid compound in the presence of the growth promoting agent at insufficient concentration or in the absence of the growth promoting agent. Preferably, the isoprenoid compound-forming microorganism is an isoprene-forming microorganism.

In such an isoprenoid compound-forming microorganism, a gene encoding an isoprenoid compound-synthetic enzyme is present under the control of a promoter which is inversely dependent on the growth promoting agent. The expression "promoter which is inversely dependent on the growth promoting agent" can mean a promoter not having at all or having low transcription activity in the presence of the growth promoting agent at relatively high concentration but having some or high transcription activity in the presence of the growth promoting agent at relatively low concentration or in the absence of the growth promoting agent. Therefore, the promoter which is inversely dependent on the growth promoting agent can suppress the expression of the gene encoding an isoprenoid compound-synthetic enzyme in the presence of the growth promoting agent at a concentration sufficient for the growth of the isoprenoid compound-forming microorganism whereas it can promote the expression of the gene encoding an isoprenoid compound-synthetic enzyme in the presence of the growth promoting agent at the concentration insufficient for the growth of the isoprenoid compound-forming microorganism or in the absence of the growth promoting agent. Specifically, the isoprenoid compound-forming microorganism is a microorganism transformed with an expression vector comprising the gene encoding an isoprenoid compound-synthetic enzyme present under the control of the promoter which is inversely dependent on the growth promoting agent. The gene encoding an isoprenoid compound-synthetic enzyme refers to one or more genes encoding one or more enzymes involved in a synthesis of an isoprenoid compound. Examples of the gene encoding an isoprenoid compound-synthetic enzyme include an isoprene synthase gene, geranyl diphosphate synthase gene, a farnesyl diphosphate synthase gene, a linalool synthase gene, an amorpha-4,11-diene synthase gene, a beta-caryophyllene synthase gene, a germacrene A synthase gene, a 8-epicedrol synthase gene, a valencene synthase gene, a (+)-delta-cadinene synthase gene, a germacrene C synthase gene, a (E)-beta- farnesene synthase gene, a casbene synthase gene, a vetispiradiene synthase gene, a 5-epi-aristolochene synthase gene, an aristolochene synthase gene, an alpha-humulene synthase gene, an (E,E)-alpha-farnesene synthase gene, a (-)-beta-pinene synthase gene, a gamma-terpinene synthase gene, a limonene cyclase gene, a linalool synthase gene, a 1,8-cineole synthase gene, a (+)-sabinene synthase gene, an E-alpha-bisabolene synthase gene, a (+)-bornyl diphosphate synthase gene, a levopimaradiene synthase gene, an abietadiene synthase gene, an isopimaradiene synthase gene, a (E)-gamma-bisabolene synthase gene, a taxadiene synthase gene, a copalyl pyrophosphate synthase gene, a kaurene synthase gene, a longifolene synthase gene, a gamma-humulene synthase gene, a Delta-selinene synthase gene, a beta-phellandrene synthase gene, a limonene synthase gene, a myrcene synthase gene, a terpinolene synthase gene, a (-)-camphene synthase gene, a (+)-3-carene synthase gene, a syn-copalyl diphosphate synthase gene, an alpha-terpineol synthase gene, a syn-pimara-7,15-diene synthase gene, an ent-sandaracopimaradiene synthase gene, a stemer-13-ene synthase gene, a E-beta-ocimene gene, a S-linalool synthase gene, a geraniol synthase gene, an epi-cedrol synthase gene, an alpha-zingiberene synthase gene, a guaiadiene synthase gene, a cascarilladiene synthase gene, a cis-muuroladiene synthase gene, an aphidicolan-16b-ol synthase gene, an elizabethatriene synthase gene, a santalol synthase gene, a patchoulol synthase gene, a zinzanol synthase gene, a cedrol synthase gene, a sclareol synthase gene, a copalol synthase gene, a manool synthase gene, a limonene monooxygenase gene, a carveol dehydrogenase gene, and the isoprene synthase gene, geranyl diphosphate synthase gene, farnesyl diphosphate synthase gene, linalool synthase gene and limonene synthase gene are preferred.

For example, when the growth promoting agent is oxygen, a microaerobically inducible promoter can be utilized. The microaerobically inducible promoter can refer to a promoter that can promote the expression of a downstream gene under a microaerophilic condition. In general, the saturated concentration of dissolved oxygen is 7.22 ppm (under the air condition: 760 mmHg, 33°C, 20.9% oxygen and saturated water vapor). The microaerophilic condition can refer to a condition where a (dissolved) oxygen concentration is 0.35 ppm or less. The (dissolved) oxygen concentration under the microaerophilic condition may be 0.30 ppm or less, 0.25 ppm or less, 0.20 ppm or less, 0.15 ppm or less, 0.10 ppm or less, or 0.05 ppm or less. Examples of the microaerobically inducible promoter may include a promoter of the gene encoding a D- or L-lactate dehydrogenase (e.g., 11d, 1dhA), a promoter of the gene encoding an alcohol dehydrogenase (e.g., adhE), a promoter of the gene encoding a pyruvate formate lyase (e.g., pflB), and a promoter of the gene encoding an α-acetolactate decarboxylase (e.g., budA).

When the growth promoting agent is a phosphorus compound, a phosphorus deficiency-inducible promoter can be utilized. The expression "phosphorus deficiency-inducible promoter" can refer to a promoter that can promote the expression of a downstream gene at low concentration of phosphorus compound. The low concentration of phosphorus compound can refer to a condition where a (free) phosphorus concentration is 100 mg/L or less. The expression "phosphorus" is synonymous to the expression "phosphorus compound", and they can be used in exchangeable manner. The concentration of total phosphorus is able to quantify by decomposing total kinds of phosphorus compounds in liquid to phosphorus in the form of orthophosphoric acid by strong acid or oxidizing agent. The total phosphorus concentration under a phosphorus deficient condition may be 50 mg/L or less, 10 mg/L or less, 5 mg/L or less, 1 mg/L or less, 0.1 mg/L or less, or 0.01 mg/L or less. Examples of the phosphorus deficiency-inducible promoter may include a promoter of the gene encoding alkali phosphatase (e.g., phoA), a promoter of the gene encoding an acid phosphatase (e.g., phoC), a promoter of the gene encoding a sensor histidine kinase (phoR), a promoter of the gene encoding a response regulator (e.g., phoB), and a promoter of the gene encoding a phosphorus uptake carrier (e.g., pstS).

When the growth promoting agent is an amino acid, an amino acid deficiency-inducible promoter can be utilized. The amino acid deficiency-inducible promoter can refer to a promoter that can promote the expression of a downstream gene at low concentration of an amino acid. The low concentration of the amino acid can refer to a condition where a concentration of a (free) amino acid or a salt thereof is 100 mg/L or less. The concentration of the (free) amino acid or a salt thereof under the amino acid deficient condition may be 50 mg/L or less, 10 mg/L or less, 5 mg/L or less, 1 mg/L or less, 0.1 mg or less or 0.01 mg/L or less. Examples of the amino acid deficiency-inducible promoter may include a promoter of the gene encoding a tryptophan leader peptide (e.g., trpL) and a promoter of the gene encoding an N-acetylglutamate synthase (e.g., ArgA).

The isoprenoid compound-forming microorganism can be obtained by transforming a host microorganism with a vector for expressing the isoprenoid compound-synthetic enzyme such as the isoprene synthase. Examples of the isoprene synthase may include the isoprene synthase derived from kudzu (*Pueraria montana var. lobata*), poplar (*Populus alba* x *Populus tremula*), mucuna (*Mucuna bracteata*), willow (*Salix*), false acacia (*Robinia pseudoacacia*), Japanese wisteria (*Wisterria*), eucalyptus (*Eucalyptus globulus*), and tea plant (*Melaleuca alterniflora*) (see, e.g., Evolution 67 (4), 1026-1040 (2013)). The vector for expressing the isoprenoid compound-synthetic enzyme may be an integrative vector or a non-integrative vector. In the expression vector, the gene encoding the isoprenoid compound-synthetic enzyme may be placed under the control of the promoter which is inversely dependent on the growth promoting agent.

The phrase "derived from" as used herein for a nucleic acid sequence such as a gene, a promoter, or an amino acid sequence such as a protein, can mean a nucleic acid sequence or an amino acid sequence that are naturally or natively synthesized by a microorganism or can be isolated from the natural or wild-type microorganism.

The isoprenoid compound-forming microorganism may further express a mevalonate kinase in addition to the isoprenoid compound-synthetic enzyme. Therefore, the isoprenoid compound-forming microorganism may be transformed with a vector for expressing the mevalonate kinase. Examples of the mevalonate kinase gene may include genes from microorganisms belonging to the genus *Methanosarcina* such as *Methanosarcina mazei,* the genus *Methanocella* such as *Methanocella paludicola,* the genus Corynebacterium such as *Corynebacterium variabile,* the genus *Methanosaeta* such as *Methanosaeta concilii,* and the genus *Nitrosopumilus* such as *Nitrosopumilus maritimus.* The vector for expressing the mevalonate kinase may be an integrative vector or a non-integrative vector. In the expression vector, the gene encoding the mevalonate kinase may be placed under the control of a constitutive promoter or inducible promoter (e.g., the promoter which is inversely dependent on the growth promoting agent). Specifically, the gene encoding the mevalonate kinase may be placed under the control of the constitutive promoter. Examples of the constitutive promoter include the tac promoter, the lac promoter, the trp promoter, the trc promoter, the T7 promoter, the T5 promoter, the T3 promoter, and the SP6 promoter.

DMAPP, that is a precursor of the isoprenoid compound (e.g., a substrate of isoprene synthesis), is typically biosynthesized via either a methylerythritol phosphate pathway or a mevalonate pathway inherently or natively possessed by a microorganism. Therefore, in the light of DMAPP supply for efficiently producing the isoprenoid compound, the methylerythritol phosphate pathway and/or the mevalonate pathway may be enhanced in the isoprenoid compound-forming microorganism used in the present invention, as described later.

The isoprenoid compound-forming microorganism used in the present invention as a host can be a bacterium or a fungus. The bacterium may be a gram-positive bacterium or a gram-negative bacterium. The isoprenoid compound-forming microorganism can be a microorganism belonging to the family *Enterobacteriaceae,* and particularly preferably a microorganism belonging to the family *Enterobacteriaceae* among microorganisms described later.

Examples of the gram-positive bacterium may include bacteria belonging to the genera *Bacillus, Listeria, Staphylococcus, Streptococcus, Enterococcus, Clostridium, Corynebacterium,* and *Streptomyces.* Bacteria belonging to the genera *Bacillus* and *Corynebacterium* are preferable. Examples of the bacteria belonging to the genus *Bacillus* may include *Bacillus subtilis, Bacillus anthracis,* and *Bacillus cereus. Bacillus subtilis* is more preferable. Examples of the bacteria belonging to the genus *Corynebacterium* may include *Corynebacterium glutamicum, Corynebacterium efficiens,* and *Corynebacterium callunae. Corynebacterium glutamicum* is more preferable.

Examples of the gram-negative bacterium may include bacteria belonging to the genera *Escherichia, Pantoea, Salmonella, Vibrio, Serratia,* and *Enterobacter.* The bacteria belonging to the genera *Escherichia, Pantoea* and *Enterobacter* are preferable.

*Escherichia coli* is preferable as the bacterium belonging to the genus *Escherichia.*

Examples of the bacteria belonging to the genus Pantoea may include *Pantoea ananatis, Pantoea stewartii, Pantoea agglomerans,* and *Pantoea citrea. Pantoea ananatis* and *Pantoea citrea* are preferable. Strains exemplified in the European Patent Application Publication EP0952221 may be used as the bacteria belonging to the genus Pantoea. Examples of representative strains of the bacteria belonging to the genus *Pantoea* may include *Pantoea ananatis* AJ13355 strain (FERM BP-6614), *Pantoea ananatis* AJ13356 strain (FERM BP-6615) disclosed in the European Patent Application Publication EP0952221, Pantoea *ananatis* SC17 strain (FERMBP-11902), and *Pantoea ananatis* SC17(0) strain (VKPM B-9246; Katashikina JI et al., BMC Mol Biol 2009;10:34).

Examples of the bacteria belonging to the genus *Enterobacter* may include *Enterobacter agglomerans* and *Enterobacter aerogenes. Enterobacter aerogenes* is preferable as the bacterium belonging to the genus *Enterobacter.* The bacterial strains exemplified in the European Patent Application Publication EP0952221 may be used as the bacteria belonging to the genus *Enterobacter.* Examples of representative strains of the bacteria belonging to the genus *Enterobacter* may include *Enterobacter agglomerans* ATCC12287 strain, *Enterobacter* aerogenes ATCC13048 strain, *Enterobacter aerogenes* NBRC12010 strain (Biotechnol. Bioeng., 2007 Mar 27; 98(2) 340-348), and *Enterobacter aerogenes* AJ110637 (FERM BP-10955). The *Enterobacter aerogenes* AJ110637 strain was deposited to International Patent Organism Depositary (IPOD), National Institute of Advanced Industrial Science and Technology (AIST) (Chuo No. 6, Higashi 1-1-1, Tsukuba City, Ibaraki Pref., JP, Postal code 305-8566; currently, International Patent Organism Depositary, National Institute of Technology and Evaluation (IPOD NITE), #120, 2-5-8 Kazusakamatari, Kisarazu-shi, Chiba, 292-0818, Japan) as of August 22, 2007, and was transferred to the international deposition based on the Budapest Treaty on March 13, 2008, and the deposit number FERM BP-10955 was given thereto.

Examples of the fungus may include microorganisms belonging to the genera *Saccharomyces, Schizosaccharomyces, Yarrowia, Trichoderma, Aspergillus, Fusarium,* and *Mucor.* The microorganisms belonging to the genera *Saccharomyces, Schizosaccharomyces, Yarrowia,* or *Trichoderma* are preferable.

Examples of the microorganisms belonging to the genus *Saccharomyces* may include *Saccharomyces carlsbergensis, Saccharomyces cerevisiae, Saccharomyces diastaticus, Saccharomyces douglasii, Saccharomyces kluyveri, Saccharomyces norbensis,* and *Saccharomyces oviformis. Saccharomyces cerevisiae* is preferable as the fungus belonging to the genus *Saccharomyces.*

*Schizosaccharomyces pombe* is preferable as the microorganisms belonging to the genus *Schizosaccharomyces.*

*Yarrowia lypolytica* is preferable as the microorganisms belonging to the genus *Yarrowia.*

Examples of the microorganisms belonging to the genus *Trichoderma* may include *Trichoderma harzianum, Trichoderma koningii, Trichoderma longibrachiatum, Trichoderma reesei,* and *Trichoderma viride. Trichoderma reesei* is preferable.

The pathway to synthesize dimethylallyl diphosphate (DMAPP) that is a precursor of the isoprenoid compound (e.g., the substrate of the isoprene synthase) may further be enhanced in the isoprene-forming microorganism. For such an enhancement, an expression vector that expresses an isopentenyl-diphosphate delta isomerase having an ability to convert isopentenyl diphosphate (IPP) into dimethylallyl diphosphate (DMAPP) may be introduced into the isoprenoid compound-forming microorganism. An expression vector that expresses one or more enzymes involved in the mevalonate pathway and/or methylerythritol phosphate pathway associated with formation of IPP and/or DMAPP may also be introduced into the isoprenoid compound-forming microorganism. The expression vector for such an enzyme may be an integrative vector or a non-integrative vector. The expression vector for such an enzyme may express further a plurality of enzymes (e.g., one or more, two or more, three or more or four or more) involved in the mevalonate pathway and/or the methylerythritol phosphate pathway, and may be, for example, an expression vector for polycistronic mRNA. Origin of one or more enzymes involved in the mevalonate pathway and/or the methylerythritol phosphate pathway may be homologous or heterologous to the host. When the origin of the enzyme involved in the mevalonate pathway and/or the methylerythritol phosphate pathway is heterologous to the host, for example, the host may be a bacterium as described above (e.g., *Escherichia coli)* and the enzyme involved in the mevalonate pathway may be derived from a fungus (e.g., *Saccharomyces cerevisiae).* In addition, when the host inherently produces the enzyme involved in the methylerythritol phosphate pathway, an expression vector to be introduced into the host may express an enzyme involved in the mevalonate pathway.

Examples of the isopentenyl-diphosphate delta isomerase (EC: 5.3.3.2) may include Idilp (ACCESSION ID NP_015208), AT3G02780 (ACCESSION ID NP_186927), AT5G16440 (ACCESSION ID NP_197148) and Idi (ACCESSION ID NP_417365). In the expression vector, the gene encoding the isopentenyl-diphosphate delta isomerase may be placed under the control of the promoter which is inversely dependent on the growth promoting agent.

Examples of the enzymes involved in the mevalonate (MVA) pathway may include mevalonate kinase (EC: 2.7.1.36; example 1, Erg12p, ACCESSION ID NP_013935; example 2, AT5G27450, ACCESSION ID NP_001190411), phosphomevalonate kinase (EC: 2.7.4.2; example 1, Erg8p, ACCESSION ID NP_013947; example 2, AT1G31910, ACCESSION ID NP_001185124), diphosphomevalonate decarboxylase (EC: 4.1.1.33; example 1, Mvd1p, ACCESSION ID NP_014441; example 2, AT2G38700, ACCESSION ID NP_181404; example 3, AT3G54250, ACCESSION ID NP_566995), acetyl-CoA-C-acetyltransferase (EC: 2.3.1.9; example 1, Erg10p, ACCESSION ID NP_015297; example 2, AT5G47720, ACCESSION ID NP_001032028; example 3, AT5G48230, ACCESSION ID NP_568694), hydroxymethylglutaryl-CoA synthase (EC: 2.3.3.10; example 1, Erg13p, ACCESSION ID NP_013580; example 2, AT4G11820, ACCESSION ID NP_192919; example 3, MvaS, ACCESSION ID AAG02438), hydroxymethylglutaryl-CoA reductase (EC: 1.1.1.34; example 1, Hmg2p, ACCESSION ID NP_013555; example 2, Hmglp, ACCESSION ID NP_013636; example 3, AT1G76490, ACCESSION ID NP_177775; example 4, AT2G17370, ACCESSION ID NP_179329, EC: 1.1.1.88, example, MvaA, ACCESSION ID P13702), and acetyl-CoA-C-acetyltransferase/hydroxymethylglutaryl-CoA reductase (EC: 2.3.1.9/1.1.1.34, example, MvaE, ACCESSION ID AAG02439). In the expression vector, the gene(s) encoding one or more enzymes involved in the mevalonate (MVA) pathway (e.g., phosphomevalonate kinase, diphosphomevalonate decarboxylase, acetyl-CoA-C-acetyltransferase/hydroxymethylglutaryl-CoA reductase (preferably, mvaE), and hydroxymethylglutaryl-CoA synthase (preferably, mvaS)) may be placed under the control of the promoter which is inversely dependent on the growth promoting agent.

In a preferred embodiment, the acetyl-CoA-C-acetyltransferase/hydroxymethylglutaryl-CoA reductase is a protein that comprises an amino acid sequence having 70% or more amino acid sequence identity to an amino acid sequence of SEQ ID NO:32, and has an acetyl-CoA-C-acetyltransferase activity or hydroxymethylglutaryl-CoA reductase activity, and the hydroxymethylglutaryl-CoA synthase is a protein that comprises an amino acid sequence having 70% or more amino acid sequence identity to an amino acid sequence of SEQ ID NO:35, and has a hydroxymethylglutaryl-CoA synthase activity. The amino acid sequence percent identity may be, for example, 75% or more, 80% or more, 85% or more, 90% or more, 95% or more, 96% or more, 97% or more, 98% or more, or 99% or more. The acetyl-CoA-C-acetyltransferase activity or hydroxymethylglutaryl-CoA reductase activity refers to an activity of producing mevalonic acid and 2 NADP and HSCoA from 3-hydroxy-3-methylglutaryl-CoA and 2 NADPH. The hydroxymethylglutaryl-CoA synthase activity refers to an activity of producing 3-hydroxy-3-methylglutaryl-CoA and HSCoA from acetoacetyl-CoA and acetyl-CoA.

The percent identity of the amino acid sequences and the percent identity of the nucleotide sequences as described later can be determined using the BLAST algorithm (Pro. Natl. Acad. Sci. USA, 90, 5873 (1993)) by Karlin and Altschul, and the FASTA algorithm (Methods Enzymol., 183, 63 (1990)) by Pearson. The programs referred to as BLASTP and BLASTN were developed based on the BLAST algorithm (see http://www.ncbi.nlm.nih.gov). Thus, the percent identity of the nucleotide sequences and the amino acid sequences may be calculated using these programs with default settings. Also, for example, a numerical value obtained by calculating similarity as a percentage at a setting of "unit size to compare=2" using the full length of a polypeptide portion encoded in ORF with the software GENETYX Ver. 7.0.9 from Genetyx Corporation employing the Lipman-Pearson method may be used as the homology of the amino acid sequences. The lowest value among the values derived from these calculations may be employed as the percent identity of the nucleotide sequences and the amino acid sequences.

In another preferred embodiment, the acetyl-CoA-C-acetyltransferase/hydroxymethylglutaryl-CoA reductase is a protein that comprises an amino acid sequence having a mutation of one or several amino acids in the amino acid sequence of SEQ ID NO:32, and has the acetyl-CoA-C-acetyltransferase activity or hydroxymethylglutaryl-CoA reductase activity, and the hydroxymethylglutaryl-CoA synthase is a protein that comprises an amino acid sequence having a mutation of one or several amino acids in the amino acid sequence of SEQ ID NO:35, and has the hydroxymethylglutaryl-CoA synthase activity. Examples of the mutation of the amino acid residues may include deletion, substitution, addition and insertion of amino acid residues. The mutation of one or several amino acids may be introduced into one region or multiple different regions in the amino acid sequence. The term "one or several" indicates a range in which a three-dimensional structure and an activity of the protein are not impaired greatly. In the case of the protein, the number represented by "one or several" can be, for example, 1 to 100, preferably 1 to 80, more preferably 1 to 50, 1 to 30, 1 to 20, 1 to 10, or 1 to 5.

The acetyl-CoA-C-acetyltransferase/hydroxymethylglutaryl-CoA reductase preferably has an acetyl-CoA-C-acetyltransferase activity or hydroxymethylglutaryl-CoA reductase activity that is 50% or more, 60% or more, 70% or more, 80% or more, 90% or more, or 95% or more of the acetyl-CoA-C-acetyltransferase activity or hydroxymethylglutaryl-CoA reductase activity of the protein consisting of the amino acid sequence of SEQ ID NO:32 when measured under the same conditions. The hydroxymethylglutaryl-CoA synthase preferably has a hydroxymethylglutaryl-CoA synthase activity that is 50% or more, 60% or more, 70% or more, 80% or more, 90% or more, or 95% or more of the hydroxymethylglutaryl-CoA synthase activity of the protein consisting of the amino acid sequence of SEQ ID NO:35 when measured under the same conditions.

In the aforementioned enzymes, the mutation may be introduced into sites in a catalytic domain and sites other than the catalytic domain as long as an objective activity is retained. The positions of amino acid residues to be mutated which are capable of retaining the objective activity are understood by a person skilled in the art. Specifically, a person skilled in the art can recognize a correlation between structure and function, since a person skilled in the art can 1) compare the amino acid sequences of multiple proteins having the same type of activity, 2) clarify regions that are relatively conserved and regions that are not relatively conserved, and then 3) predict regions capable of playing a functionally important role and regions incapable of playing a functionally important role from the regions that are relatively conserved and the regions that are not relatively conserved, respectively. Therefore, a person skilled in the art can identify the positions of the amino acid residues to be mutated in the amino acid sequence of the aforementioned enzymes.

When the amino acid residue is mutated by substitution, the substitution of the amino acid residue may be conservative substitution. As used herein, the term "conservative substitution" refers to substitution of a certain amino acid residue with an amino acid residue having a similar side chain. Families of amino acid residues having similar side chains are well-known in the art. Examples of such families may include amino acids having a basic side chain (e.g., lysine, arginine, histidine), amino acids having an acidic side chain (e.g., aspartic acid, glutamic acid), amino acids having a non-charged polar side chain (e.g., asparagine, glutamine, serine, threonine, tyrosine, cysteine), amino acids having a non-polar side chain (e.g., glycine, alanine, valine, leucine, isoleucine, proline, phenylalanine, methionine, tryptophan), amino acids having a branched side chain at position β (e.g., threonine, valine, isoleucine), amino acids having an aromatic side chain (e.g., tyrosine, phenylalanine, tryptophan, histidine), amino acids having a hydroxyl group-containing (e.g., alcoholic, phenolic) side chain (e.g., serine, threonine, tyrosine), and amino acids having a sulfur-containing side chain (e.g., cysteine, methionine). Preferably, the conservative substitution of the amino acids may be the substitution between aspartic acid and glutamic acid, the substitution among arginine, lysine and histidine, the substitution between tryptophan and phenylalanine, the substitution between phenylalanine and valine, the substitution among leucine, isoleucine and alanine, and the substitution between glycine and alanine.

In another preferred embodiment, a gene encoding the acetyl-CoA-C-acetyltransferase/hydroxymethylglutaryl-CoA reductase may be a polynucleotide that comprises a nucleotide sequence having 70% or more nucleotide sequence identity to a nucleotide sequence of SEQ ID NO:33 or SEQ ID NO:34, and encodes a protein having the acetyl-CoA-C-acetyltransferase activity or hydroxymethylglutaryl-CoA reductase activity, and a gene encoding the hydroxymethylglutaryl-CoA synthase may be a polynucleotide that comprises a nucleotide sequence having 70% or more nucleotide sequence identity to a nucleotide sequence of SEQ ID NO:36 or SEQ ID NO:37, and encodes a protein having the hydroxymethylglutaryl-CoA synthase activity. The nucleotide sequence percent identity may be 70% or more, 75% or more, 80% or more, 85% or more, 90% or more, 95% or more, 96% or more, 97% or more, 98% or more, or 99% or more.

In another preferred embodiment, a gene encoding the acetyl-CoA-C-acetyltransferase/hydroxymethylglutaryl-CoA reductase may be a polynucleotide that hybridizes with a polynucleotide consisting of a nucleotide sequence complementary to a nucleotide sequence of SEQ ID NO:33 or SEQ ID NO:34 under a stringent condition, and encodes the protein having the acetyl-CoA-C-acetyltransferase activity or hydroxymethylglutaryl-CoA reductase activity, and a gene encoding the hydroxymethylglutaryl-CoA synthase may be a polynucleotide that hybridizes with a polynucleotide consisting of a nucleotide sequence complementary to a nucleotide sequence of SEQ ID NO:36 or SEQ ID NO:37 under a stringent condition, and encodes the protein having the hydroxymethylglutaryl-CoA synthase. The "stringent condition" refers to a condition where a so-called specific hybrid is formed whereas a non-specific hybrid is not formed. For example, such a condition is the condition where substantially the same polynucleotides having the high identity, for example, the polynucleotides having the percent identity described above hybridize to each other whereas polynucleotides having the lower identity than above do not hybridize to each other. Specifically, such a condition may include hybridization in 6×SCC (sodium chloride/sodium citrate) at about 45°C followed by one or two or more washings in 0.2×SCC and 0.1% SDS at 50 to 65°C.

Examples of the enzymes involved in the methylerythritol phosphate (MEP) pathway may include 1-deoxy-D-xylulose-5-phosphate synthase (EC: 2.2.1.7, example 1, Dxs, ACCESSION ID NP_414954; example 2, AT3G21500, ACCESSION ID NP_566686; example 3, AT4G15560, ACCESSION ID NP_193291; example 4, AT5G11380, ACCESSION ID NP_001078570), 1-deoxy-D-xylulose-5-phosphate reductoisomerase (EC: 1.1.1.267; example 1, Dxr, ACCESSION ID NP_414715; example 2, AT5G62790, ACCESSION ID NP_001190600), 4-diphosphocytidyl-2-C-methyl-D-erythritol synthase (EC: 2.7.7.60; example 1, IspD, ACCESSION ID NP_417227; example 2, AT2G02500, ACCESSION ID NP_565286), 4-diphosphocytidyl-2-C-methyl-D-erythritol kinase (EC: 2.7.1.148; example 1, IspE, ACCESSION ID NP_415726; example 2, AT2G26930, ACCESSION ID NP_180261), 2-C-methyl-D-erythritol-2,4-cyclodiphosphate synthase (EC: 4.6.1.12; example 1, IspF, ACCESSION ID NP_417226; example 2, AT1G63970, ACCESSION ID NP_564819), 1-hydroxy-2-methyl-2-(E)-butenyl-4-diphosphate synthase (EC: 1.17.7.1; example 1, IspG, ACCESSION ID NP_417010; example 2, AT5G60600, ACCESSION ID NP_001119467), and 4-hydroxy-3-methyl-2-butenyl diphosphate reductase (EC: 1.17.1.2; example 1, IspH, ACCESSION ID NP_414570; example 2, AT4G34350, ACCESSION ID NP_567965). In the expression vector, the gene(s) encoding one or more enzymes involved in the methylerythritol phosphate (MEP) pathway may be placed under the control of the promoter which is inversely dependent on the growth promoting agent.

Further, a gene encoding the enzyme involved in the mevalonate pathway or the methylerythritol phosphate pathway that synthesizes dimethylallyl diphosphate or isopentenyl diphosphate that is a precursor of the isoprenoid compound (e.g., the substrate of the isoprene synthase) may also be introduced into the isoprene-forming microorganism. Examples of such an enzyme may include 1-deoxy-D-xylose-5-phosphate synthase that converts a pyruvate and D-glycelaldehyde-3-phosphate into 1-deoxy-D-xylose-5-phosphate, and isopentenyl diphosphate isomerase that converts isopentenyl diphosphate into dimethylallyl diphosphate. In the expression vector, the gene encoding the enzyme involved in the mevalonate pathway or the methylerythritol phosphate pathway that synthesizes dimethylallyl diphosphate may be placed under the control of the constitutive promoter or inducible promoter (e.g., the promoter which is inversely dependent on the growth promoting agent).

The transformation of a host with an expression vector containing the gene(s) described above can be carried out using one or more known methods. Examples of such methods may include a competent cell method using a microbial cell treated with calcium, and an electroporation method. The gene may also be introduced by infecting the microbial cell with a phage vector other than the plasmid vector.

In step 1) of the method of the present invention, the isoprenoid compound-forming microorganism is grown in the presence of the growth promoting agent at sufficient concentration. More specifically, the isoprenoid compound-forming microorganism can be grown by culturing the isoprenoid compound-forming microorganism in a culture medium in the presence of the growth promoting agent at sufficient concentration.

For example, when oxygen is used as the growth promoting agent, the isoprenoid compound-forming microorganism can be an aerobic microorganism. The aerobic microorganism can grow well in the presence of oxygen in a sufficient concentration, and thus, oxygen can act as the growth promoting agent for the aerobic microorganism. When the growth promoting agent is oxygen, a concentration of dissolved oxygen in the culture medium, which is sufficient for the growth of the aerobic microorganism in step 1), is not particularly limited as long as oxygen at that concentration can promote the growth of the aerobic microorganism, and may be, for example, 0.50 ppm or more, 1.00 ppm or more, 1.50 ppm or more, or 2.00 ppm or more. The concentration of the dissolved oxygen for the growth of the aerobic microorganism may be, for example, 7.00 ppm or less, 5.00 ppm or less, or 3.00 ppm or less.

When a phosphorus compound or an amino acid is used as the growth promoting agent, the isoprenoid compound-forming microorganism can grow well in the presence of the phosphorus compound or the amino acid in a sufficient concentration, and thus, the phosphorus compound or the amino acid can act as the growth promoting agent. When the growth promoting agent is the phosphorus compound or the amino acid, a concentration of the phosphorus compound or the amino acid that is sufficient for the growth in step 1) is not particularly limited, and may be, for example, 200 mg/L or more, 300 mg/L or more, 500 mg/L or more, 1000 mg/L or more, or 2000 mg/L or more. The concentration of the phosphorus compound or the amino acid for the growth may be, for example, 20 g/L or less, 10 g/L or less, or 5 g/L or less.

In step 2) of the method of the present invention, the formation of the isoprenoid compound by the isoprenoid compound-forming microorganism is induced by decreasing the concentration of the growth promoting agent. More specifically, the concentration of the growth promoting agent can be decreased by decreasing an amount of the growth promoting agent supplied into a culture medium. Even if the amount of the growth promoting agent supplied into the culture medium is made constant throughout steps 1) and 2), the concentration of the growth promoting agent can be decreased by utilizing the growth of the microorganism. In early phase of the growth of the microorganism in step 1), the microorganism does not grow sufficiently and the number of the microorganism in the culture medium is small. Thus, a consumption of the growth promoting agent by the microorganism is relatively low. Therefore, the concentration of the growth promoting agent in the culture medium is relatively high in the early phase of the growth. On the other hand, in the late phase of the growth of the microorganism in step 1), the microorganism grows sufficiently and the number of the microorganism is large, and thus, the consumption of the growth promoting agent by the microorganism is relatively high. Therefore, the concentration of the growth promoting agent in the culture medium becomes relatively low in the late phase of the growth. As described above, when the constant amount of the growth promoting agent continues to be supplied into the culture medium throughout steps 1) and 2), the concentration of the growth promoting agent in the culture medium is decreased in inverse proportion to the growth of the microorganism. This decreased concentration can be used as a trigger to induce the formation of the isoprenoid compound (e.g., the isoprene monomer, linalool, limonene) by the isoprenoid compound-forming microorganism.

For example, when oxygen is used as the growth promoting agent, the concentration of dissolved oxygen in the culture medium, which can induce the formation of the isoprenoid compound by the isoprenoid compound-forming microorganism can be, for example, 0.35 ppm or less, 0.15 ppm or less, or 0.05 ppm or less. The concentration of dissolved oxygen in the culture medium may be a concentration under the microaerophilic condition as described above.

Also, when a phosphorus compound or an amino acid is used as the growth promoting agent, the concentration of the phosphorus compound or the amino acid in the culture medium, which can induce the formation of the isoprenoid compound by the isoprenoid compound-forming microorganism, can be, for example, 100 mg/L or less, 50 mg/L or less, or 10 mg/L or less.

In step 3) of the method of the present invention, the isoprenoid compound is formed by culturing the isoprenoid compound-forming microorganism under the conditions of claims 1 or 7. More specifically, the isoprenoid compound can be formed by culturing the isoprenoid compound-forming microorganism in the culture medium under the condition of step 2) where the concentration of the growth promoting agent is decreased. The concentration of the growth promoting agent in the culture medium can be maintained at the concentration described in step 2) above in order to make the formation of the isoprenoid compound by the isoprenoid compound-forming microorganism possible. In step 3), the concentration of the isoprenoid compound formed in the culture medium can be, for example, 600 ppm or more, 700 ppm or more, 800 ppm or more, or 900 ppm or more, for example, within 6 hours, or 5, 4, or 3 hours after culturing the isoprenoid compound-forming microorganism in the culture medium under the condition of step 2.

The method of the present invention also can be characterized by an isoprenoid compound-forming rate in an induction phase, when the isoprenoid compound is the isoprene. The isoprene-forming rate (ppm/vvm/h) in the induction phase is an index value of induction efficiency that can be determined by dividing a maximum concentration of the isoprene per vvm (volume per volume per minute) by an induction time, wherein the induction time is defined as the period of time from the start of isoprene formation (the concentration of the isoprene in a fermentation gas is defined as 50 ppm) to the time point when the maximum concentration of the isoprene is achieved. The value "vvm (volume per volume per minute)" indicates ventilation amount of gas per unit time per unit culture medium in a culture apparatus. Such an isoprene-forming rate can vary depending on the type of the growth promoting agent. For example, when the oxygen is used as the growth promoting agent, such an isoprene-forming rate can be 20 ppm/vvm/h or more, 40 ppm/vvm/h or more, 50 ppm/vvm/h or more, 60 ppm/vvm/h or more, or 70 ppm/vvm/h or more, and also can be 100 ppm/vvm/h or less, 90 ppm/vvm/h or less, or 80 ppm/vvm/h or less. When the phosphorus compound is used as the growth promoting agent, such a rate can be 50 ppm/vvm/h or more, 100 ppm/vvm/h or more, 150 ppm/vvm/h or more, 200 ppm/vvm/h or more, or 250 ppm/vvm/h or more, and also can be 1000 ppm/vvm/h or less, 900 ppm/vvm/h or less, 800ppm/vvm/h or less, 700 ppm/vvm/h or less, or 600 ppm/vvm/h or less.

In the method of the present invention, it is also possible that the period of time of culturing the isoprenoid compound-forming microorganism in step 3) is set longer than that period of step 1). In the conventional method which utilizes an inducer to obtain the isoprenoid compound in a higher amount, it is necessary to culture a microorganism for a long period of time using the inducer in the formation phase of the isoprenoid compound. However, when the cultivation is continued for a long period of time, the inducer is decomposed, and the microorganism fails to maintain the ability to produce the isoprenoid compound. Thus, it is necessary to continuously add the inducer into culture medium. As the inducer may be expensive, the cost for producing the isoprenoid compound may become inappropriate. Therefore, the culturing a microorganism for a long period of time using the inducer in the formation phase of the isoprenoid compound is problematic in that the cost for producing the isoprenoid compound can be elevated depending on the duration of the cultivation period. On the other hand, in the method of the present invention not using a particular substance such as the inducer in step 3), it is not necessary to consider the decomposition of the particular substance, and the conventional problem that the cultivation for a long period of time in the formation phase of the isoprenoid compound causes the elevation of the cost for producing the isoprenoid compound does not occur. Therefore, in the method of the present invention, the period of time of step 3) can easily be made longer, differently from the conventional method using the inducer. In the method of the present invention, the longer the period of time of step 3) is made, the more isoprenoid compound can be produced.

When the isoprenoid compound is a volatile substance, the method of the present invention can be performed in a system comprising a liquid phase and a gas phase. The volatile substance means a compound having a vapor pressure of 0.01 kPa or more at 20°C. For methods of determining a vapor pressure, static method, boiling-point method, isoteniscope, gas saturation method and DSC method are generally known (Japanese laid-open publication no. 2009-103584). One example of the volatile isoprenoid compound includes isoprene. A closed system, for example, a reactor such as a fermentation jar or a fermentation tank, can be used as such system so as to avoid disappearance by diffusion of formed isoprene. A culture medium containing the isoprene-forming microorganism can be used as the liquid phase. The gas phase is present in the space above the liquid phase in the system, also called a headspace, and contains fermentation gas. Isoprene has the boiling point of 34°C at standard atmosphere, it is poorly-soluble in water (solubility in water: 0.6 g/L) and it exhibits the vapor pressure of 60.8 kPa at 20°C (e.g., Brandes et al., Physikalisch Technische Bundesanstalt (PTB), 2008). Thus, when the isoprene-forming microorganism is cultured in the liquid phase under a temperature condition at 34°C or higher, isoprene formed in the liquid phase can be easily transferred into the gas phase. Therefore, when such a system is used, isoprene formed in the liquid phase can be collected from the gas phase. Also, as isoprene formed in the liquid phase can be easily transferred into the gas phase, an isoprene-formation reaction by the isoprene-forming microorganism (enzymatic reaction by an isoprene synthase) in the liquid phase can also be always biased in favor of the side of isoprene formation. Limonene is poorly-soluble in water (solubility in water: 13.8 mg/L) and it exhibits the vapor pressure of 0.19 kPa at 20°C ((R)-(+)-limonene safety data sheet; Junsei Chemical Co., Ltd.; 82205jis-1,2014/05/19). Thus, when the limonene-forming microorganism is cultured in the liquid phase under a temperature condition at 34°C or higher, isoprene formed in the liquid phase can be easily transferred into the gas phase. Linalool is poorly-soluble in water (solubility in water: 0.16 g/100 mL) and it exhibits the vapor pressure of 0.021 Pa at 25°C (linalool safety data sheet; Tokyo Chemical Industry Co., Ltd.; December 10, 2013). Thus, when the linalool-forming microorganism is cultured in the liquid phase under a temperature condition at 34°C or higher, isoprene formed in the liquid phase can be easily transferred into the gas phase. Therefore, when such a system is used, an isoprenoid compound formed in the liquid phase can be collected from the gas phase. Also, as a volatile isoprenoid compound formed in the liquid phase can be easily transferred into the gas phase, an isoprenoid compound-formation reaction by the isoprenoid-forming microorganism (enzymatic reaction by an isoprenoid synthetic enzyme) in the liquid phase can also be always biased in favor of the side of isoprenoid compound formation.

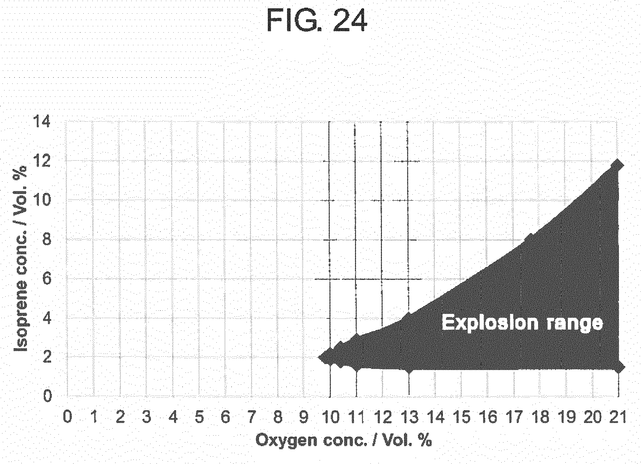

When the method of the present invention is performed in the system comprising the liquid phase and the gas phase, it is desirable to control the oxygen concentration in the gas phase. Volatile organic compounds include those having burst property. Isoprene has a burst limit of 1.0 to 9.7% (w/w) (e.g., Brandes et al., Physikalisch Technische Bundesanstalt (PTB), 2008) and a nature to easily burst, and a burst range of isoprene varies depending on a mixing ratio of isoprene with oxygen in the gas phase (see FIG. 24, US8420360B2). Thus, in the light of avoidance of the burst, it is necessary to control the oxygen concentration in the gas phase. Limonene has a burst range of 0.7 to 6.1% (w/w) (e.g., Brandes et al., Physikalisch Technische Bundesanstalt (PTB), 2008) and a nature to easily burst. Thus, in the light of avoidance of the burst, it is necessary to control the oxygen concentration in the gas phase.

The oxygen concentration in the gas phase can be controlled by supplying a gas in which the oxygen concentration has been regulated into the system. The gas supplied into the system may contain gas components such as nitrogen, carbon dioxide, and argon, other than oxygen. More specifically, the oxygen concentration in the gas phase can be controlled by adding an inert gas so that the oxygen concentration becomes equal to or less than a limit oxygen concentration in the gas having the burst range. The gas component other than oxygen is desirably the inert gas. Preferably, the gas in which the oxygen concentration has been regulated is supplied to the liquid phase, thereby indirectly controlling the oxygen concentration in the gas phase. Because, the oxygen concentration in the gas phase can be controlled by regulation of the dissolved oxygen concentration in the liquid phase as described below.

Oxygen in the gas supplied to the liquid phase is dissolved in the liquid phase, and before long reaches a saturation concentration. On the other hand, in the system in which a microorganism is present, dissolved oxygen in the liquid phase is consumed by metabolic activity of the microorganism which is cultured, and consequently the dissolved oxygen concentration decreases to a concentration less than the saturation concentration. In the liquid phase containing oxygen at concentration less than the saturation concentration, oxygen in the gas phase or oxygen in the gas freshly supplied can be transferred to the liquid phase by gas-liquid equilibrium. That is, the oxygen concentration in the gas phase decreases depending on an oxygen consumption rate by the microorganism. It is also possible to control the oxygen concentration in the gas phase by controlling the oxygen consumption rate by the microorganism which is cultured.

For example, by increasing a metabolic rate of a carbon source in the microorganism, the oxygen consumption rate can be elevated, and the oxygen concentration in the gas phase can be set to 9% (v/v) or less (e.g., 5% (v/v) or less, 0.8% (v/v) or less, 0.6% (v/v) or less, 0.5% (v/v) or less, 0.4% (v/v) or less, 0.3% (v/v) or less, 0.2% (v/v) or less, or 0.1% (v/v) or less) or substantially 0% (v/v). Alternatively, the oxygen concentration in the gas to be supplied can initially be set low. Therefore, the oxygen concentration in the gas phase can be set by considering the consumption rate of oxygen by the microorganism and the oxygen concentration in the supplied gas.

In step 1) that is the growth phase of a microorganism, when the dissolved oxygen is not present at constant concentration in the liquid phase, the microorganism cannot grow well depending on its type in the liquid phase in some cases. Also in step 1), isoprene is not formed, and thus, it is not always necessary to control the oxygen concentration in the gas phase in the light of avoidance of the burst.

For example, when oxygen is used as the growth promoting agent, the gas in which the oxygen concentration has been regulated can be supplied into the liquid phase so that the dissolved oxygen concentration that is suitable for the growth of the microorganism such as an aerobic microorganism can be maintained in the liquid phase. The gas supplied into the liquid phase can be dissolved in the liquid phase by stirring. The dissolved oxygen concentration in the liquid phase is not particularly limited as long as the isoprene-forming microorganism can grow sufficiently, and the concentration of the dissolved oxygen can vary depending on a type of the microorganism utilized such as an aerobic microorganism. For example, the concentration as described above can be employed as the concentration of dissolved oxygen in the culture medium, which is sufficient for the growth of the aerobic microorganism. Specifically, when oxygen is used as the growth promoting agent, the isoprene-forming microorganism can be grown in the presence of dissolved oxygen at the sufficient concentration as described above by supplying oxygen into a liquid phase containing the isoprene-forming microorganism in a system comprising a gas phase and the liquid phase.

In step 2) that is the induction phase of the isoprene formation, the oxygen concentration in the system is regulated in the light of avoidance of the burst by mixed gas of oxygen and isoprene which is formed in step 3) after the induction rather than in the light of growth of the microorganism in the liquid phase. For example, the oxygen concentration in the gas phase may be the same as in step 3) as described later in the light of avoidance of the burst by the mixed gas of oxygen and isoprene which is formed in the step 3).

For example, when oxygen is used as the growth promoting agent, the oxygen concentration in the system is regulated in the light of those mentioned above and in the light of inducing the formation of isoprene monomer by the isoprene-forming microorganism in the liquid phase. For example, the dissolved oxygen concentration in the liquid phase is not particularly limited as long as the aforementioned points of view are accomplished at that concentration, and varies depending on the type of the isoprene-forming microorganism utilized, and the promoter to be utilized, but may be, for example, 0.35 ppm or less, 0.25 ppm or less, 0.15 ppm or less, 0.10 ppm or less, or 0.05 ppm or less. The dissolved oxygen concentration in the liquid phase may also be the concentration under the microaerophilic condition as described above. The dissolved oxygen concentration in the liquid phase can be decreased by decreasing the amount of oxygen supplied into the liquid phase. Even if the amount of oxygen supplied into the liquid phase is made constant throughout steps 1) and 2), the dissolved oxygen concentration in the liquid phase can be decreased by utilizing the growth of the microorganism which is cultured. In the early phase of the growth of the microorganism in step 1), the microorganism does not grow sufficiently and the number of the microorganism in the culture medium is relatively small. Thus, the oxygen consumption by the microorganism is relatively low. Therefore, the dissolved oxygen concentration in the liquid phase and the oxygen concentration in the gas phase are relatively high in the early phase of the growth. On the other hand, in the late phase of the growth of the microorganism, the microorganism grows sufficiently and the number of the microorganism in the culture medium is relatively large. Thus, the oxygen consumption by the microorganism is relatively high. Therefore, the dissolved oxygen concentration in the liquid phase and the oxygen concentration in the gas phase become relatively low in the late phase of the growth. As described above, when the gas containing oxygen in the constant concentration continues to be supplied into the liquid phase throughout steps 1) and 2), the dissolved oxygen concentration in the liquid phase is decreased in inverse proportion to the growth of the microorganism. This decreased oxygen concentration in the liquid phase can be used as the trigger to induce the formation of the isoprene monomer by the isoprene-forming microorganism.

In step 3) that is the formation phase of isoprene, the oxygen concentration in the gas phase is not particularly limited as long as the burst by the mixed gas of isoprene and oxygen is avoided. When the oxygen concentration in such a mixed gas is about 9.5% (v/v) or less, the burst can be avoided regardless of isoprene concentration in the gas phase (see FIG. 24). Thus, the oxygen concentration in the gas phase can be about 9.5% (v/v) or less. In the light of acquiring a safety zone of the oxygen concentration for the burst, the oxygen concentration in the gas phase can be 9% (v/v) or less, 8% (v/v) or less, 7% (v/v) or less, or 6% (v/v) or less, 5% (v/v) or less, 4% (v/v) or less, 3% (v/v) or less, 2% (v/v) or less, or 1% (v/v) or less. In step 3), the gas in which the oxygen concentration has been adjusted can be supplied into the liquid phase so that such an oxygen concentration can be maintained in the gas phase.

For example, when oxygen is used as the growth promoting agent, an isoprene monomer can be formed by culturing the isoprene-forming microorganism under the condition of the oxygen concentration in the liquid phase as described in step 2). In this case, it is desirable that the oxygen concentration in the liquid phase as described in step 2) is balanced with the oxygen concentration in the gas phase as described in step 3). The oxygen concentration in the liquid phase as described in step 2) and the oxygen concentration in the gas phase as described in step (3) can be balanced by regulating the amount of supplied gas containing oxygen while considering the type of the microorganism in the liquid phase and a degree of its growth.

When isoprene is formed in the system comprising the liquid phase and the gas phase, isoprene formed in the liquid phase can be collected from the gas phase (fermentation gas) as described above. Isoprene can be collected from the gas phase by known methods. Examples of the method of collecting isoprene from the gas phase may include an absorption method, a cooling method, a pressure swing adsorption method (PSA method), and a membrane separation method. Before being subjected to these methods, the gas phase may be subjected to a pretreatment such as dehydration, pressure elevating, or pressure reducing, if necessary.

The method of the present invention may be combined with another method in terms of enhancing the amount of produced isoprenoid compound. Examples of such a method may include a method of utilizing an environmental factor such as light (Pia Lindberg, Sungsoon Park, Anastasios Melis, Metabolic Engineering 12 (2010): 70-79) or temperature (Norma A Valdez-Cruz, Luis Caspeta, Néstor O Pérez, Octavio T Ramirez, Mauricio A Trujillo-Roldán, Microbial Cell Factories 2010,9:1), change of pH (EP 1233068 A2), addition of surfactant (JP 11009296 A), and auto-inducible expression system (WO2013/151174).

The culture medium used in the method of the present invention may contain a carbon source for forming the isoprenoid compound. The carbon source may include carbohydrates such as monosaccharides, disaccharides, oligosaccharides and polysaccharides; invert sugars obtained by hydrolyzing sucrose; glycerol; compounds having one carbon atom (hereinafter referred to as a C1 compound) such as methanol, formaldehyde, formate, carbon monoxide and carbon dioxide; oils such as corn oil, palm oil and soybean oil; acetate; animal fats; animal oils; fatty acids such as saturated fatty acids and unsaturated fatty acids; lipids; phospholipids; glycerolipids; glycerine fatty acid esters such as monoglyceride, diglyceride and triglyceride; polypeptides such as microbial proteins and plant proteins; renewable carbon sources such as hydrolyzed biomass carbon sources; yeast extracts, or combinations thereof. For a nitrogen source, inorganic ammonium salts such as ammonium sulfate, ammonium chloride and ammonium phosphate, organic nitrogen such as hydrolyzed soybeans, ammonia gas, and ammonia water can be used. It is desirable that the culture medium contains required substances such as vitamin B1 and L-homoserine, or the yeast extract in an appropriate amount as an organic trace nutrient source. In addition thereto, potassium phosphate, magnesium sulfate, iron ion, manganese ion are added in a small amount if necessary. The culture medium used in the present invention may be a natural medium or a synthesized medium as long as it contains the carbon source, the nitrogen source, inorganic ions, and optionally the other organic trace ingredients.

Examples of the monosaccharide may include triose such as ketotriose (dihydroxyacetone) and aldotriose (glyceraldehyde); tetrose such as ketotetrose (erythrulose) and aldotetrose (erythrose, threose); pentose such as ketopentose (ribulose, xylulose), aldopentose (ribose, arabinose, xylose, lyxose) and deoxysaccharide (deoxyribose); hexose such as ketohexose (psichose, fructose, sorbose, tagatose), aldohexose (allose, altrose, glucose, mannose, gulose, idose, galactose, talose), and deoxysaccharide (fucose, fuculose, rhamnose); and heptose such as sedoheptulose. C6 sugars such as fructose, mannose, galactose and glucose; and C5 sugars such as xylose and arabinose are preferable.

Examples of the disaccharide may include sucrose, lactose, maltose, trehalose, turanose, and cellobiose. Sucrose and lactose are preferable.

Examples of the oligosaccharide may include trisaccharides such as raffinose, melezitose and maltotriose; tetrasaccharides such as acarbose and stachyose; and other oligosaccharides such as fructooligosaccharide (FOS), galactooligosaccharide (GOS) and mannan-oligosaccharide (MOS).

Examples of the polysaccharide may include glycogen, starch (amylose, amylopectin), cellulose, dextrin, and glucan (β-1,3-glucan), and starch and cellulose are preferable.

Examples of the microbial protein may include polypeptides derived from a yeast or bacterium.

Examples of the plant protein may include polypeptides derived from soybean, corn, canola, Jatropha, palm, peanut, sunflower, coconut, mustard, cotton seed, palm kernel oil, olive, safflower, sesame and linseed.

Examples of the lipid may include substances containing one or more saturated or unsaturated fatty acids of C4 or more.

The oil can be the lipid that contains one or more saturated or unsaturated fatty acids of C4 or more and is liquid at room temperature, and examples of the oil may include lipids derived from soybean, corn, canola, Jatropha, palm, peanut, sunflower, coconut, mustard, cotton seed, Palm kernel oil, olive, safflower, sesame, linseed, oily microbial cells, Chinese tallow tree, and a combination of two or more thereof.

Examplesof the fatty acid may include compounds represented by a formula RCOOH ("R" represents a hydrocarbon group having two or more carbon atoms).

The unsaturated fatty acid is a compound having at least one double bond between two carbon atoms in the group "R" as described above, and examples of the unsaturated fatty acid may include oleic acid, vaccenic acid, linoleic acid, palmitelaidic acid and arachidonic acid.

The saturated fatty acid is a compound where the "R" is a saturated aliphatic group, and examples of the saturated fatty acid may include docosanoic acid, eicosanoic acid, octadecanoic acid, hexadecanoic acid, tetradecanoic acid, and dodecanoic acid.

Among them, those containing one or more C2 to C22 fatty acids are preferable as the fatty acid, and those containing C12 fatty acid, C14 fatty acid, C16 fatty acid, C18 fatty acid, C20 fatty acid and C22 fatty acid are more preferable.

The carbon source may include salts and derivatives of these fatty acids and salts of these derivatives.

Examples of the salt may include lithium salts, potassium salts, sodium salts and so forth.

Examples of the carbon source may also include combinations of carbohydrates such as glucose with lipids, oils, fats, fatty acids and glycerol fatty acid esters.

Examples of the renewable carbon source may include hydrolyzed biomass carbon sources.

Examples of the biomass carbon source may include cellulose-based substrates such as waste materials of woods, papers and pulps, leafy plants, and fruit pulps; and partial plants such as stalks, grain particles, roots and tubers.

Examples of the plant to be used as the biomass carbon source may include corn, wheat, rye, sorghum, triticale, rice, millet, barley, cassava, legume such as pea, potato, sweet potato, banana, sugar cane and tapioca.

When the renewable carbon source such as biomass is added to the culture medium, the carbon source can be pretreated. Examples of the pretreatment may include an enzymatic pretreatment, a chemical pretreatment, and a combination of the enzymatic pretreatment and the chemical pretreatment.

It is preferred that the renewable carbon source is entirely or partially hydrolyzed before being added to the culture medium.

Examples of the carbon source may also include the yeast extract and a combination of the yeast extract with the other carbon source such as glucose. The combination of the yeast extract with the C1 compound such as carbon dioxide and methanol is preferable.

In the method of the present invention, it is preferable to culture the isoprenoid compound-forming microorganism in a standard culture medium containing saline and nutrients.

The culture medium is not particularly limited, and examples of the culture medium may include ready-made general media that is commercially available such as Luria Bertani (LB) broth, Sabouraud dextrose (SD) broth, and yeast medium (YM) broth. The medium suitable for the cultivation of the specific host can be selected appropriately for the use.

It is desirable to contain appropriate minerals, salts, supplemental elements, buffers, and ingredients known for those skilled in the art to be suitable for the cultivation and to facilitate the production of the isoprenoid compound in addition to the appropriate carbon source in the cell medium.

A standard cell culture condition except that the concentration of the growth promoting agent is regulated as described above can be used as a culture condition for the isoprenoid compound-forming microorganism.

A culture temperature can be 20 to 40°C, and a pH value can be about 4.5 to about 9.5.

The isoprenoid compound-forming microorganism can be cultured under an aerobic, oxygen-free, or anaerobic condition depending on a nature of the host for the isoprene-forming microorganism. A known fermentation method such as a batch cultivation method, a feeding cultivation method or a continuous cultivation method can appropriately be used as a cultivation method.

The present invention also provides a method of producing an isoprene polymer. The method of producing the isoprene polymer according to the present invention comprises the following (I) and (II):
(I) forming an isoprene monomer by the method of the present invention; and
(II) polymerizing the isoprene monomer to form an isoprene polymer.

The step (I) can be performed in the same manner as in the method of producing the isoprene monomer according to the present invention described above. The polymerization of the isoprene monomer in the step (II) can be performed by any method known in the art (e.g., synthesis methods such as addition polymerization in organic chemistry).

### Method for producing a rubber composition

The rubber composition of produced according to the methods the present invention comprises a polymer derived from isoprene produced by the method for producing isoprene according to the present invention. The polymer derived from isoprene may be a homopolymer (i.e., isoprene polymer) or a heteropolymer comprising an isoprene monomer unit and one or more monomer units other than the isoprene monomer unit (e.g., a copolymer such as a block copolymer). Preferably, the polymer derived from isoprene is a homopolymer (i.e., isoprene polymer) produced by the method for producing isoprene polymer according to the present invention. The rubber composition may further comprise one or more polymers other than the above polymer, one or more rubber components, and/or other components. The rubber composition can be manufactured using the polymer derived from isoprene. For example, the rubber composition can be prepared by mixing the polymer derived from isoprene with one or more polymers other than the above polymer, one or more rubber components, and/or other components such as a reinforcing filler, a crosslinking agent, a vulcanization accelerator and an antioxidant.

### Method for producing a tire

The tire produced according to the methods of the present invention is manufactured by using the rubber composition produced according to the methods of the present invention. The rubber composition may be applied to any portion of the tire without limitation, which may be selected as appropriate depending on the application thereof. For example, the rubber composition may be used in a tread, a base tread, a sidewall, a side reinforcing rubber and a bead filler of a tire. The tire can be manufactured by a conventional method. For example, a carcass layer, a belt layer, a tread layer, which are composed of unvulcanized rubber, and other members used for the production of usual tires are successively laminated on a tire molding drum, then the drum is withdrawn to obtain a green tire. Thereafter, the green tire is heated and vulcanized in accordance with an ordinary method, to thereby obtain a desired tire (e.g., a pneumatic tire).

### EXAMPLES

Hereinafter, the present invention will be described in detail with reference to Examples, but the present invention is not limited to the following Examples.

### Example 1: Construction of isoprenoid compound-forming microorganisms (arabinose-inducible isoprenoid compound-forming microorganism: Enterobacter aerogenes GI08-Para/ispSK strain, and microaerobically inducible isoprenoid compound-forming microorganism: Enterobacter aerogenes GI08-Pbud/ispSK strain)

### 1.1) Construction of GI05 (ES04Δlld::Ptac-KDyI strain)

*Enterobacter aerogenes* GI05 (ES04Δlld::Ptac-KDyI) strain was constructed by replacing a lld gene on a chromosome in ES04 strain (US2010-0297716A1) constructed from *Enterobacter aerogenes* AJ110637 (FERM BP-10955) strain with a Ptac-KDyI gene derived from E.coli MG1655 Ptac-KDyI strain (see Reference Example 1). A nucleotide sequence of the lld gene from *Enterobacter aerogenes* AJ110637 (FERM BP-10955) strain is described as SEQ ID NO:1.

### 1.1.1) Construction of gene fragment λattL-Tet^{R}-λattR-Ptac-KDyI

A Ptac-KDyI gene derived from MG1655 Ptac-KDyI strain encodes phosphomevalonate kinase (gene name: PMK) and diphosphomevalonate decarboxylase (gene name: MVD) and further isopentenyl diphosphate isomerase (gene name: yIDI) derived from *Saccharomyces cerevisiae* under the control of a tac promoter. PCR (TaKaRa Prime Star (registered trademark), 30 cycles of reactions at 94°C for 10 seconds, 54°C for 20 seconds and 72°C for 420 seconds) with genomic DNA from MG1655 Ptac-KDyI strain as a template was carried out using primers described as SEQ ID NO:2 and SEQ ID NO:3 designed based on the above nucleotide sequence to obtain a gene fragment λattL-Tet^{R}-λattR-Ptac-KDyI having a recombinant sequence of a gene encoding D-lactate dehydrogenase at both termini (gene name: lld).

### 1.1.2) Construction of ES04/RSFRedTER strain

ES04 strain (US20100297716A1) was cultured overnight in an LB liquid culture medium. Subsequently, 100 µL of the cultured medium was inoculated to 4 mL of a new LB liquid culture medium, and microbial cells were cultured with shaking at 34°C for 3 hours. After collecting the microbial cells, the microbial cells were washed three times with 10% glycerol to use as competent cells. RSFRedTER was introduced by an electroporation method (Katashkina JI et al., BMC Mol Biol. 2009; 10: 34). The electroporation was carried out using Gene Pulser II (supplied from BioRad) under the condition of an electric field intensity of 24 kV/cm, a condenser capacity of 25 µF, and a resistance value of 200 Ω. The cells were cultured in an SOC culture medium (20 g/L of bacto tryptone, 5 g/L of yeast extract, 0.5 g/L of NaCl, 10g/L of glucose) for 2 hours, and then was applied onto an LB culture medium containing 40 mg/L of chloramphenicol, and cultured for 16 hours. As a result, transformants exhibiting chloramphenicol resistance were obtained and designated as ES04/RSFRedTER strain.

### 1.1.3) Construction of ES04Δlld:: λattL-Tet^{R}-λattR-Ptac-KDyI strain

ES04/RSFRedTER strain was cultured in an LB liquid culture medium overnight. Subsequently, 1 mL of the cultured medium was inoculated to 100 mL of an LB liquid culture medium containing IPTG and chloramphenicol at final concentrations of 1 mM and 40 mg/L, respectively, and microbial cells were cultured at 34°C for 3 hours with shaking. After collecting the microbial cells, the microbial cells were washed three times with 10% glycerol to use as competent cells. An amplified λattL-Tet^{R}-λattR-Ptac-KDyI gene fragment purified using Wizard PCR Prep DNA Purification System (supplied from Promega) was introduced into the competent cells by the electroporation method. The cells were cultured in the SOC culture medium for 2 hours, then applied onto the LB culture medium containing 30mg/L of tetracycline, and cultured for 16 hours. Emerging colonies were refined in the same culture medium. Subsequently, colony PCR (TaKaRa Speed Star (registered trademark), 40 cycles of reactions at 92°C for 10 seconds, 56°C for 10 seconds and 72°C for 60 seconds) was carried out using primers described as SEQ ID NO:4 and SEQ ID NO:5 to identify that the lld gene on the chromosome was replaced with the λattL-Tet^{R}-λattR-Ptac-KDyI gene. The resulting colony was applied onto an LB agar medium containing 10% sucrose and 1 mM IPTG and delete the RSFRedTER plasmid to obtain ES04Δlld:: λattL-Tet^{R}-λattR-Ptac-KDyI strain.

### 1.1.4) Removal of tetracycline resistant gene from ES04Δlld:: λattL-Tet^{R}-λattR-Ptac-KDyI strain

In order to remove the tetracycline resistant gene from ES04Δlld:: λattL-Tet^{R}-λattR-Ptac-KDyI strain, an RSF-int-xis (US20100297716A1) plasmid was used. RSF-int-xis was introduced into λattL-Tet^{R}-λattR-Ptac-KDyI strain by the electroporation method, and the cells were applied onto the LB culture medium containing 40 mg/L of chloramphenicol and cultured at 30°C to obtain λattL-Tet^{R}-λattR-Ptac-KDyI/RSF-int-xis strain. The resulting plasmid-possessing strain was refined in the LB culture medium containing 40 mg/L of chloramphenicol and 1 mM IPTG to obtain a plurality of single colonies. Subsequently, the single colony was applied onto the culture medium containing 30 mg/L of tetracycline and cultured at 37°C overnight, and the colony was confirmed to be a strain in which the tetracycline resistant gene had been removed by confirming that the colony could not grow in this culture medium. Subsequently, the resulting strain was applied onto the LB culture medium containing 10% sucrose and 1 mM IPTG and cultured at 37°C overnight in order to delete the RSF-int-xis plasmid from the resulting strain. A colony that exhibited chloramphenicol sensitivity among emerging colonies was designated as GI05 (ES04Δlld::Ptac-KDyI) strain.

### 1.2) Construction of GI06 (GI05 ΔpoxB::Ptac-PMK) strain

*Enterobacter aerogenes* GI06 (ES04Δlld::Ptac-KDyIΔpoxB::Ptac-PMK) strain was constructed by replacing a pyruvate oxidase gene (gene name: poxB) on a chromosome of *Enterobacter aerogenes* GI05 strain with a phosphomevalonate kinase (PMK) gene derived from *E*. *coli* MG1655 Ptac-KDyI strain. A nucleotide sequence of the poxB gene from *Enterobacter aerogenes* AJ110637 (FERM BP-10955) strain is described as SEQ ID NO:6. A procedure will be described below.

### 1.2.1) Construction of gene fragment λattL-Km^{r}-λattR-Ptac-PMK

PCR (TaKaRa Prime Star (registered trademark), 30 cycles of reactions at 94°C for 10 seconds, 54°C for 20 seconds and 72°C for 120 seconds) with genomic DNA from *E*. *coli* MG1655 Ptac-KDyI strain as the template was carried out using primers described as SEQ ID NO:7 and SEQ ID NO:8 designed based on the above nucleotide sequence to obtain a DNA fragment containing an ORF region of PMK. Also, PCR (TaKaRa Prime Star (registered trademark), 30 cycles of reactions at 94°C for 10 seconds, 54°C for 20 seconds and 72°C for 90 seconds) with a DNA fragment containing λattL-Km^{r}-λattR-Ptac (WO2008090770A1) as the template was carried out using primers described as SEQ ID NO:9 and SEQ ID NO:10 to obtain a DNA fragment containing λattL-Km^{r}-λattR-Ptac. Subsequently, overlapping PCR (TaKaRa Prime Star (registered trademark), 35 cycles of reactions at 94°C for 10 seconds, 54°C for 20 seconds and 72°C for 180 seconds) with the DNA fragment containing the ORF region of PMK and the DNA fragment containing λattL-Km^{r}-λattR-Ptac as the template was carried out using the primers described as SEQ ID NO:7 and SEQ ID NO:9 to obtain a gene fragment λattL-Km^{r}-λattR-Ptac-PMK having a recombinant sequence of the gene (gene name: poxB) encoding pyruvate oxidase at both termini.

### 1.2.2) Acquisition of GI06 strain by λ-Red method

GI05ΔpoxB:: λattL-Km^{r}-λattR-Ptac-PMK exhibiting kanamycin resistance was obtained by introducing RSFRedTER into GI05 strain, introducing the λattL-Km^{r}-λattR-Ptac-PMK gene fragment into poxB by λ-Red method, and selecting in the LB culture medium containing 100 mg/L of kanamycin in the same manner as in the procedure for constructing the aforementioned GI05 strain. After refining the resulting colonies in the LB culture medium, colony PCR (TaKaRa Speed Star (registered trademark), 40 cycles of reactions at 92°C for 10 seconds, 56°C for 10 seconds and 72°C for 60 seconds) was carried out using primers described as SEQ ID NO:11 and SEQ ID NO:12 to confirm that the poxB gene on the chromosome was replaced with the λattL-Km^{R}-λattR-Ptac-PMK gene. Subsequently, in order to remove the kanamycin resistant gene from GI05ApoxB:: λattL-Km^{R}-λattR-Ptac-PMK strain from which RSFRedTER was deleted, pRSF-int-xis was introduced and the drug resistant gene was removed in the same manner as in the procedure for constructing the GI05 strain. A strain exhibiting kanamycin sensitivity was designated as GI06 (GI05ΔpoxB::Ptac-PMK).

### 1.3) Construction of GI07 ΔpflB::Ptac-MVD strain(GI06 ΔpflB::Ptac-MVD)

*Enterobacter aerogenes* GI07 (GI06 ΔpflB::Ptac-MVD) strain was constructed by replacing a pyruvate formate lyase B gene (gene name: pflB) on a chromosome from *Enterobacter aerogenes* GI06 strain with a diphosphomevalonate decarboxylase (MVD) gene derived from *E*. *coli* MG1655 Ptac-KDyI strain. A nucleotide sequence of the pflB gene from *Enterobacter aerogenes* AJ110637 (FERM BP-10955) is described as SEQ ID NO:13. The procedure will be described below.

### 1.3.1) Construction of gene fragment λattL-Km^{r}-λattR-Ptac-MVD

PCR (TaKaRa Prime Star (registered trademark), 30 cycles of reactions at 94°C for 10 seconds, 54°C for 20 seconds and 72°C for 120 seconds) with genomic DNA derived from *E*. *coli* MG1655 Ptac-KDyI strain as the template was carried out using primers described as SEQ ID NO:14 and SEQ ID NO:15 designed based on the above nucleotide sequence to obtain a DNA fragment containing an ORF region of MVD. Also, PCR (TaKaRa Prime Star (registered trademark), 30 cycles of reactions at 94°C for 10 seconds, 54°C for 20 seconds and 72°C for 90 seconds) with a DNA fragment containing λattL-Km^{r}-λattR-Ptac (WO2008090770A1) as the template was carried out using primers described as SEQ ID NO:16 and SEQ ID NO:17 to obtain a DNA fragment containing λattL-Km^{r}-λattR-Ptac. Subsequently, overlapping PCR (TaKaRa Prime Star (registered trademark), 35 cycles of reactions at 94°C for 10 seconds, 54°C for 20 seconds and 72°C for 240 seconds) with the DNA fragment containing the ORF region of MVD and the DNA fragment containing λattL-Km^{r}-λattR-Ptac as the template was carried out using the primers described as SEQ ID NO:15 and SEQ ID NO:16 to obtain a gene fragment λattL-Km^{r}-λattR-Ptac-MVD having a recombinant sequence of the gene (gene name: pflB) encoding pyruvate formate lyase B at both termini.

### 1.3.2) Acquisition of GI07 strain by λ-Red method

GI06ΔpflB:: λattL-Km^{r}-λattR-Ptac-MVD exhibiting kanamycin resistance was obtained by introducing RSFRedTER into GI06 strain, introducing the λattL-Km^{r}-λattR-Ptac-MVD gene fragment into pflB by the λ-Red method, and selecting in the LB culture medium containing 100 mg/L of kanamycin in the same manner as in the procedure for constructing the aforementioned GI05 strain. After refining the resulting colonies, colony PCR (TaKaRa Speed Star (registered trademark), 40 cycles of reactions at 92°C for 10 seconds, 56°C for 10 seconds and 72°C for 60 seconds) was carried out using primers described as SEQ ID NO:18 and SEQ ID NO:19 to confirm that the pflB gene on the chromosome was replaced with the λattL-Km^{R}-λattR-Ptac-MVD gene. Subsequently, in order to remove the kanamycin resistant gene from GI06ΔpflB:: λattL-Km^{R}-λattR-Ptac-MVD strain from which RSFRedTER was deleted, pRSF-int-xis was introduced and the drug resistant gene was removed in the same manner as in the procedure for constructing the GI05 strain. A strain exhibiting kanamycin sensitivity was designated as GI07 (GI06ΔpflB::Ptac-MVD).

### 1.4) Construction of GI08 (GI07ΔpflA::Ptac-yIDI) strain

*Enterobacter aerogenes* GI08 (GI07 ΔpflA::Ptac-yIDI) strain was constructed by replacing a pyruvate formate lyase A gene (gene name: pflA) on a chromosome from *Enterobacter aerogenes* GI07 strain with a isopentenyl diphosphate isomerase (yIDI) gene derived from *E. coli* MG1655 Ptac-KDyI strain. A nucleotide sequence of the pflA gene from *Enterobacter aerogenes* AJ110637 (FERM BP-10955) is described as SEQ ID NO:20. The procedure will be described below.

### 1.4.1) Construction of gene fragment λattL-Km^{r}-λattR-Ptac-yIDI

PCR (TaKaRa Prime Star (registered trademark), 30 cycles of reactions at 94°C for 10 seconds, 54°C for 20 seconds and 72°C for 120 seconds) with genomic DNA derived from *E*. *coli* MG1655 Ptac-KDyI strain as the template was carried out using primers described as SEQ ID NO:21 and SEQ ID NO:22 designed based on the above nucleotide sequence to obtain a DNA fragment containing an ORF region of yIDI. Also, PCR (TaKaRa Prime Star (registered trademark), 30 cycles of reactions at 94°C for 10 seconds, 54°C for 20 seconds and 72°C for 90 seconds) with a DNA fragment containing λattL-Km^{r}-λattR-Ptac (WO2008090770A1) as the template was carried out using primers described as SEQ ID NO:23 and SEQ ID NO:24 to obtain a DNA fragment containing λattL-Km^{r}-λattR-Ptac. Subsequently, PCR (TaKaRa Prime Star (registered trademark), 35 cycles of reactions at 94°C for 10 seconds, 54°C for 20 seconds and 72°C for 240 seconds) with the DNA fragment containing the ORF region of yIDI and the DNA fragment containing λattL-Km^{r}-λattR-Ptac as the template was carried out using the primers described as SEQ ID NO:23 and SEQ ID NO:24 to obtain a gene fragment λattL-Km^{r}-λattR-Ptac-yIDI having a recombinant sequence of the gene (gene name: pflA) encoding pyruvate formate lyase A at both termini.

### 1.4.2) Acquisition of GI08 strain by λ-Red method

GI07ApflA:: λattL-Km^{r}-λattR-Ptac-yIDI strain exhibiting kanamycin resistance was obtained by introducing RSFRedTER into GI07 strain, introducing the λattL-Km^{r}-λattR-Ptac-yIDI gene fragment into pflA by the λ-Red method, and selecting in the LB culture medium containing 100 mg/L of kanamycin in the same manner as in the procedure for constructing the aforementioned GI05 strain. After refining the resulting colonies, colony PCR (TaKaRa Speed Star (registered trademark), 40 cycles of reactions at 92°C for 10 seconds, 56°C for 10 seconds and 72°C for 60 seconds) was carried out using primers described as SEQ ID NO:25 and SEQ ID NO:26 to confirm that the pflA gene on the chromosome was replaced with the λattL-Km^{R}-λattR-Ptac-yIDI gene. Subsequently, in order to remove the kanamycin resistant gene from GI07ΔpflA:: λattL-Km^{r}-λattR-Ptac-yIDI strain from which RSFRedTER was deleted, pRSF-int-xis was introduced and the drug resistant gene was removed in the same manner as in the procedure for constructing the GI05 strain. A strain exhibiting kanamycin sensitivity was designated as GI08 (GI07ApflA::Ptac-ylDI).

### 1.5) Construction of arabinose-inducible isoprenoid compound-forming microorganism GI08-Para/ispSK strain (GI08/pMW-Para-mvaES-Ttrp/pSTV28-Ptac-ispSK)

In order to impart an ability to produce an isoprenoid compound to GI08 strain, pMW-Para-mvaES-Ttrp (see Reference Example 2) and pSTV28-Ptac-ispSK (see WO2013/179722) were introduced by the electroporation method. After preparing competent cells of GI08 strain according to the above method, pMW-Para-mvaES-Ttrp and pSTV28-Ptac-ispSK were introduced by the electroporation method, and cells were selected in the LB culture medium containing 100 mg/L of kanamycin and 60 mg/L of chloramphenicol. GI08 strain/pMW-Para-mvaES-Ttrp/pSTV28-Ptac-ispSK retaining both the plasmids was designated as GI08-Para/ispSK strain.

### 1.6) Construction of pMW-Pbud-mvaES

It has been already known that *Enterobacter aerogenes* forms 2,3-butandiol under a microaerophilic condition (Converti, A et al., Biotechnol. Bioeng., 82, 370-377, 2003). An enzyme group involved in a formation pathway and a catalytic reaction of 2,3-butandiol has been already elucidated, and their gene information and amino acid sequences have been demonstrated from the genome sequence (NC_015663) of *Enterobacter aerogenes* KCT2190. The formation pathway of 2,3-butandiol is composed of α-acetolactate decarboxylase (gene name: budA), acetolactate synthase (gene name: budB), and further acetoin reductase (gene name budC). These genes form an operon on the genome sequence, and its expression amount is controlled by BudR (gene name: budR) that is a transcription factor. A promoter region for BudR and the bud operon was cloned into pMW-Para-mvaES-Ttrp by the following procedure. The promoter region for BudR and the bud operon is shown as SEQ ID NO:29.

PCR (TaKaRa Prime Star (registered trademark), 30 cycles of reactions at 94°C for 10 seconds, 54°C for 20 seconds and 72°C for 120 seconds) with genomic DNA derived from *Enterobacter aerogenes* AJ11063 strain as the template was carried out using primers described as SEQ ID NO:27 and SEQ ID NO:28 to obtain a DNA fragment containing an ORF region of BudR and the promoter region for the bud operon.

Subsequently, PCR (TaKaRa Prime Star (registered trademark), 30 cycles of reactions at 94°C for 10 seconds, 54°C for 20 seconds and 72°C for 240 seconds) with pMW-Para-mvaES-Ttrp as the template was carried out using primers described as SEQ ID NO:30 and SEQ ID NO:31 to obtain a DNA fragment of pMW-Para-mvaES-Ttrp in which an arabinose promoter had been deleted. The DNA fragment containing the ORF region of BudR and the promoter region of the bud operon was ligated to the DNA fragment of pMW-Para-mvaES-Ttrp in which the arabinose promoter had been deleted using In-Fusion HD Cloning Kit (supplied from Clontech). The resulting plasmid in which the arabinose promoter had been replaced with the ORF region of BudR and the promoter region for the bud operon was designated as pMW-Pbud-mvaES-Ttrp.

### 1.7) Construction of microaerobically inducible isoprenoid compound-forming microorganism GI08-Pbud/ispSK strain (GI08/pMW-Pbud-mvaES/pSTV28-Ptac-ispSK)

In order to impart the ability to produce an isoprenoid compound to GI08 strain, pMW-Pbud-mvaES-Ttrp and pSTV28-Ptac-ispSK (see WO2013/179722) were introduced by the electroporation method. After preparing competent cells of GI08 strain according to the above method, pMW-Pbud-mvaES-Ttrp and pSTV28-Ptac-ispSK were introduced by the electroporation method, and cells were selected in the LB culture medium containing 100 mg/L of kanamycin and 60 mg/L of chloramphenicol. GI08 strain/pMW-Pbud-mvaES-Ttrp/pSTV28-Ptac-ispSK retaining both the plasmids was designated as GI08-Pbud/ispSK strain.

### Example 2: Condition for Jar culture of isoprenoid compound-forming microorganisms, GI08-Para/ispSK strain and GI08-Pbud/ispSK strain

A jar culture was carried out for growing microbial cells of the isoprenoid compound-forming microorganisms, GI08-Para/ispSK strain and GI08-Pbud/ispSK strain. A fermentation jar (system comprising a liquid phase and a gas phase) having a 1 L volume was used for the jar culture. A glucose medium was prepared in a composition shown in Table 1. Microbial cells of the isoprenoid compound-forming microorganisms, GI08-Para/ispSK strain and GI08-Pbud/ispSK strain were applied onto an LB plate containing chloramphenicol (60 mg/L) and kanamycin (50 mg/L), and cultured at 37°C for 16 hours. After adding 0.3 L of the glucose culture medium into the fermentation jar having the 1 L volume, and microbial cells sufficiently grown on one plate were inoculated thereto, and the culture was started. The culture was carried out under a condition at pH 7.0 (controlled by ammonia gas) at 30°C and air (oxygen concentration: 20% (v/v)) was supplied at 150 mL/minute into the culture medium. Dissolved oxygen (DO) in the culture medium was measured using a galvanic mode DO sensor SDOU model (supplied from ABLE & Biott Co., Ltd), and controlled by stirring so that DO was a given concentration. During the cultivation, a solution of glucose adjusted to 500 g/L was continuously added so that a glucose concentration in the culture medium was 10 g/L or more. An OD value (indicator for growth of microorganism) was measured at 600 nm using a spectrophotometer (HITACHI U-2900).

The detection limit of the galvanic mode DO sensor SDOU model used for the measurement of the DO concentration is 0.003 ppm. Hereinafter, when the measured DO concentration is below the detection limit, it is represented by " 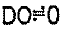 ppm".

**Table 1. Composition of glucose medium**

| Group A | Final concentration |
|---|---|
| Glucose | 80 g/L |
| MgSO₄.7aq | 2.0 g/L |

| Group B | Final concentration |
|---|---|
| (NH₄)₂SO₄ | 2.0 g/L |
| KH₂PO₄ | 2.0 g/L |
| FeSO₄.7aq | 20 mg/L |
| MnSO₄.5aq | 20 mg/L |
| Yeast Extract | 4.0 g/L |

Each 0.15 L of Group A and Group B was prepared, and then sterilized with heat at 115°C for 10 minutes. After cooling, Group A and Group B were mixed, and chloramphenicol (60 mg/L) and kanamycin (50mg/L) were added, and used as the medium.

### Example 3: Production of isoprenoid compound by isoprenoid compound-forming microorganism

### 3.1) Induction to formation phase of isoprene

In an arabinose-inducible isoprenoid compound-forming microorganism (GI08-Para/ispSK), genes upstream of the mevalonate pathway are expressed by an arabinose inducible promoter, and thus an amount of isoprene produced in the presence of L-arabinose (Wako Pure Chemical Industries, Ltd.) is notably enhanced. To induce to a formation phase of isoprene, L-arabinose was added at a final concentration of 20 mM when the OD value by analysis of the culture medium with time was 16.

In a microaerobically inducible isoprenoid compound-forming microorganism (GI08-Pbud/ispSK), genes upstream of the mevalonate pathway are expressed and controlled by the promoter for the bud operon, which is a microaerobically inducible promoter, and thus an amount of isoprene produced under a microaerophilic condition is notably enhanced. In this Example, the formation phase of isoprene was induced by culturing under a constant condition for a ventilated amount and a stirring frequency and making the dissolved oxygen concentration in the culture medium to be the detection limit( 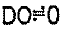 ppm) or below with the increase of the microbial cells.

After inducing the isoprene formation by the isoprenoid compound-forming microorganism as described above, the cultivation of the isoprenoid compound-forming microorganism was continued for the isoprene formation.

### 3.2) Measurement of isoprene concentration in fermentation gas

Isoprene is poorly soluble in water and is easily volatilized. Thus, isoprene formed in the liquid phase (culture medium) is rapidly transferred as a fermentation gas into the gas phase. Therefore, the formed isoprene was measured by quantifying an isoprene concentration in the fermentation gas. Specifically, the fermentation gas was collected in a gas bag on a timely basis after inducing the isoprene formation, and the isoprene concentration was quantified using gas chromatography (GC-2010 Plus AF supplied from Shimadzu Corporation). A standard curve for isoprene was made using the following isoprene standard samples. An analysis condition for the gas chromatography will be described below.

### Preparation of isoprene standard samples

A reagent isoprene (supplied from Tokyo Chemical Industry, specific gravity: 0.681) was diluted with cooled methanol to 10, 100, 1,000, 10,000 and 100,000 times to prepare standard solutions for addition. Subsequently, each 1 µL of each standard solution for the addition was added to a headspace vial in which 1 mL of water had been already added, and used as a standard sample.

Headspace sampler (Turbo Matrix 40 supplied from Perkin Elmer)
Heat retention temperature for vial: 40°C
Heat retention time for vial: 30 minutes
Pressurization time: 3.0 minutes
Injection time: 0.02 minutes
Needle temperature: 70°C
Transfer temperature: 80°C
Carrier gas pressure (high purity helium): 124 kPa
Gas chromatography (GC-2010 Plus AF, supplied from Shimadzu Corporation)
Column: Rxi (registered trade name) -1 ms: length 30 m, inner diameter 0.53 mm, liquid phase membrane thickness 1.5 µm, cat #13370)
Column temperature: 37°C
Pressure: 24.8 kPa
Column flow rate: 5 mL/minute
Inflow method; Split 1:0 (actual measurement 1:18)
Transfer flow amount: 90 mL
GC injection amount: 1.8 mL (transfer flow amount × injection time)
Sample amount injected into column: 0.1 mL
Inlet temperature 250°C
Detector: FID (hydrogen 40 mL/minute, Air 400 mL/minute, makeup gas helium 30 mL/minute)
Detector temperature: 250°C

### 3.3) Isoprene formation by culturing arabinose-inducible isoprenoid compound-forming microorganism and microaerobically inducible isoprenoid compound-forming microorganism

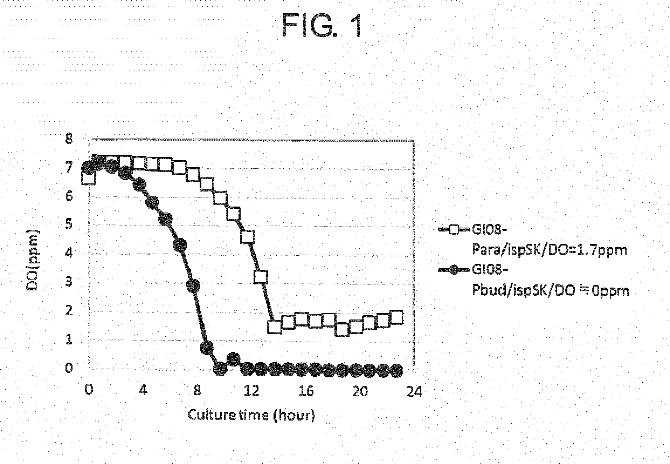

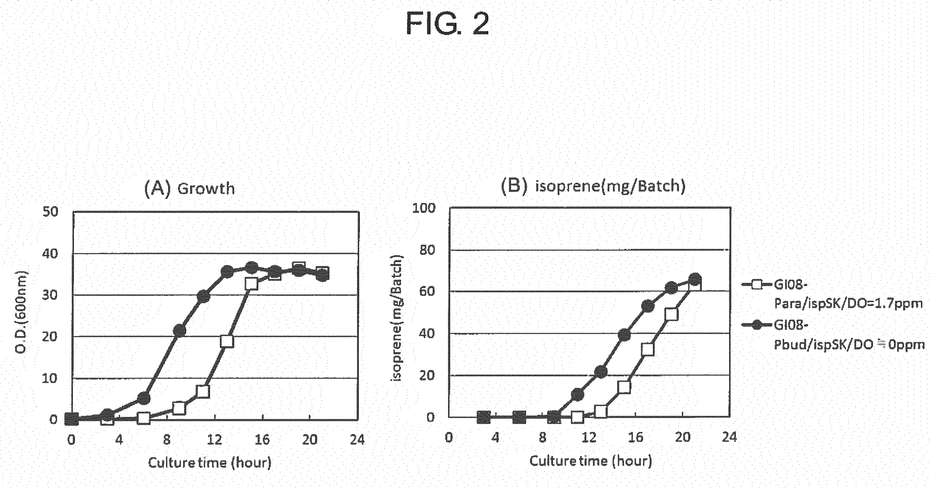

The arabinose-inducible isoprenoid compound-forming microorganism (GI08-Para/ispSK) and microaerobically inducible isoprenoid compound-forming microorganism (GI08-Pbud/ispSK) were cultured under the jar culture condition described in above Example 2, and amounts of formed isoprene were measured. As shown in FIG. 1, by controlling the stirring frequency during the cultivation, the dissolved oxygen concentration in the culture medium in which GI08-Para/ispSK was cultured was kept at 1.7 ppm from 14 hours after the start of the cultivation, and the dissolved oxygen concentration in the culture medium in which GI08-Pbud/ispSK was cultured became the detection limit or below ( 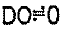 ppm) from 9 hours after the start of the cultivation. As shown in FIG. 2(A), GI08-Para/ispSK and GI08-Pbud/ispSK grew well under the jar culture condition using the fermentation jar (system comprising a liquid phase and a gas phase). As shown in FIG. 2(B), the production of isoprene was detected at 11 hours and 8 hours after the start of the cultivation in GI08-Pbud/ispSK and GI08-Para/ispSK, respectively, indicating that the production of isoprene was induced. The amounts of formed isoprene until 21 hours after starting the cultivation were 63 mg and 66 mg in GI08-Para/ispSK and GI08-Pbud/ispSK, respectively. This result indicates that the arabinose-inducible isoprenoid compound-forming microorganism and the microaerobically inducible isoprenoid compound-forming microorganism have an equivalent ability to produce isoprene.

### Example 4: Amount of isoprene formed by microaerobically inducible isoprenoid compound-forming microorganism under various dissolved oxygen condition

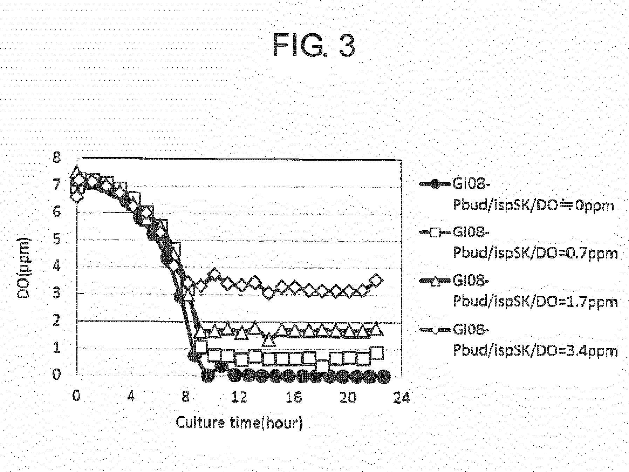

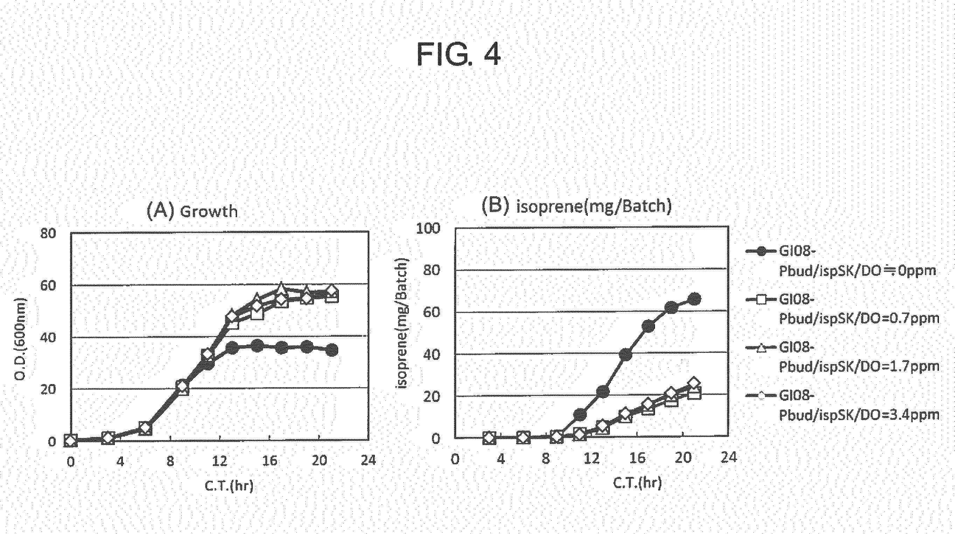

The microaerobically inducible isoprenoid compound-forming microorganism was cultured under the condition of the dissolved oxygen at 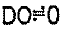 ppm, 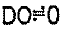 ppm, DO=1.7 ppm and DO=3.4 ppm by supplying air containing 20% oxygen and controlling the stirring frequency during the cultivation. Changes with time of the dissolved oxygen concentration in the culture medium are shown in FIG. 3. The OD values (indicator for the growth of the microorganism) in the culture medium were 35, 55, 57 and 57 under the condition of 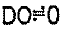 ppm, DO=0.7 ppm, DO=1.7 ppm and DO=3.4 ppm, respectively. The OD value was higher under the aerobic culture condition than that under the condition of 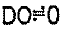 ppm (FIG. 4). Under the condition of 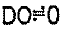 ppm, the production of isoprene was induced after 9 hours after starting the cultivation at which DO became 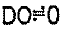 ppm, and high productivity of isoprene was observed after 13 hours after starting the cultivation at which the OD value became plateau. The amounts of isoprene formed until 21 hours after starting the cultivation were 66 mg, 21 mg, 24 mg and 25 mg under the condition of 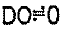 ppm, DO=0.7 ppm, DO=1.7 ppm and DO=3.4 ppm, respectively (FIG. 4). This result indicated that the dissolved oxygen concentration became the detection limit or below, thereby transferring to the formation phase of isoprene, and subsequently the production of isoprene was also continued in the microaerobically inducible isoprenoid compound-forming microorganism.

### Example 5: Construction of microaerobically inducible isoprenoid compound-forming microorganism (SWITCH-Plld/IspSM), phosphate deficiency-inducible isoprenoid compound-forming microorganism (SWITCH-PphoC/IspSM, SWITCH-PpstS/IspSM) and arabinose-inducible isoprenoid compound-forming microorganism (SWITCH-Para/IspSM)

### 5-1) Construction of pMW-Para-mvaES-Ttrp

### 5-1-1) Chemical synthesis of mvaES gene derived from Enterococcus faecalis

A nucleotide sequence and an amino acid sequence of mvaE encoding acetyl-CoA acetyltransferase and hydroxymethylglutaryl-CoA reductase and derived from *Enterococcus faecalis* have been already known (Accession number of nucleotide sequence: AF290092.1,(1479..3890), Accession number of amino acid sequence: AAG02439) (J. Bacteriol. 182 (15), 4319-4327 (2000)). The amino acid sequence of the mvaE protein derived from *Enterococcus faecalis* and the nucleotide sequence of its gene are shown as SEQ ID NO:32 and SEQ ID NO:33, respectively. In order to efficiently express the mvaE gene in *E. coli*, an mvaE gene in which codon usage in *E*. *coli* had been optimized was designed, and this was designated as EFmvaE. This nucleotide sequence is shown as
SEQ ID NO:34. The mvaE gene was chemically synthesized, then was cloned into pUC57 (supplied from GenScript), and the resulting plasmid was designated as pUC57-EFmvaE.

### 5-1-2) Chemical synthesis of mvaS gene derived from Enterococcus faecalis

A nucleotide sequence encoding hydroxymethylglutaryl-CoA synthase and derived from *Enterococcus faecalis,* and its amino acid sequence have been already known (Accession number of nucleotide sequence: AF290092.1, complement (142..1293), Accession number of amino acid sequence: AAG02438) (J. Bacteriol. 182(15), 4319-4327 (2000)). The amino acid sequence of the mvaS protein derived from *Enterococcus faecalis* and the nucleotide sequence of the mvaS gene are shown as SEQ ID NO:35 and SEQ ID NO:36, respectively. In order to efficiently express the mvaS gene in *E*. *coli,* an mvaS gene in which the codon usage in *E*. *coli* had been optimized was designed, and this was designated as EFmvaS. This nucleotide sequence is shown as SEQ ID NO:37. The mvaS gene was chemically synthesized, then was cloned into pUC57 (supplied from GenScript), and the resulting plasmid was designated as pUC57-EFmvaS.

### 5-1-3) Construction of expression vector for arabinose-inducible mvaES

An expression vector for arabinose-inducible gene upstream of the mevalonate pathway was constructed by the following procedure. PCR with plasmid pKD46 as the template was carried out using synthesized oligonucleotides shown as SEQ ID NO:38 and SEQ ID NO:39 as primers to obtain a PCR fragment containing Para composed of araC and an araBAD promoter derived from *E. coli.* PCR with plasmid pUC57-EFmvaE as the template was carried out using synthesized oligonucleotides shown as SEQ ID NO:40 and SEQ ID NO:41 as primers to obtain a PCR fragment containing the EFmvaE gene. PCR with plasmid pUC57-EFmvaS as the template was carried out using synthesized oligonucleotides shown as SEQ ID NO:42 and SEQ ID NO:43 as primers to obtain a PCR fragment containing the EFmvaS gene. PCR with plasmid pSTV-Ptac-Ttrp (WO2013069634A1) as the template was carried out using synthesized oligonucleotides shown as SEQ ID NO:44 and SEQ ID NO:45 as primers to obtain a PCR fragment containing a Ttrp sequence. Prime Star polymerase (supplied from Takara Bio Inc.) was used for PCR for obtaining these four PCR fragments. A reaction solution was prepared according to a composition attached to a kit, and DNA was amplified through 30 cycles of reactions at 98°C for 10 seconds, 55°C for 5 seconds and 72°C for one minute per kb. PCR with the purified PCR product containing Para and PCR product containing the EFmvaE gene as the template was carried out using synthesized oligonucleotides shown as SEQ ID NO:38 and SEQ ID NO:41 as primers, and PCR with the purified PCR product containing the EFmvaS gene and PCR product containing Ttrp as the template was carried out using synthesized oligonucleotides shown in SEQ ID NO:42 and SEQ ID NO:45 as primers. As a result, a PCR product containing Para and the EFmvaE gene and a PCR product containing the EFmvaS gene and Ttrp were obtained. A plasmid pMW219 (supplied from Nippon Gene Co., Ltd.) was digested with SmaI according to a standard method. pMW219 after being digested with SmaI was ligated to the purified PCR product containing Para and the EFmvaE gene and the purified PCR product containing the EFmvaS gene and Ttrp using In-Fusion HD Cloning Kit (supplied from Clontech). The resulting plasmid was designated as pMW-Para-mvaES-Ttrp.

### 5-2) Construction of the integrative conditionally replicated plasmids carrying genes of upper and lower mevalonate pathways

To construct the integrative plasmids carrying genes of upper and lower mevalonate pathways the pAH162-λattL-TcR-λattR vector (Minaeva NI et al., BMC Biotechnol., 2008; 8: 63) was used.

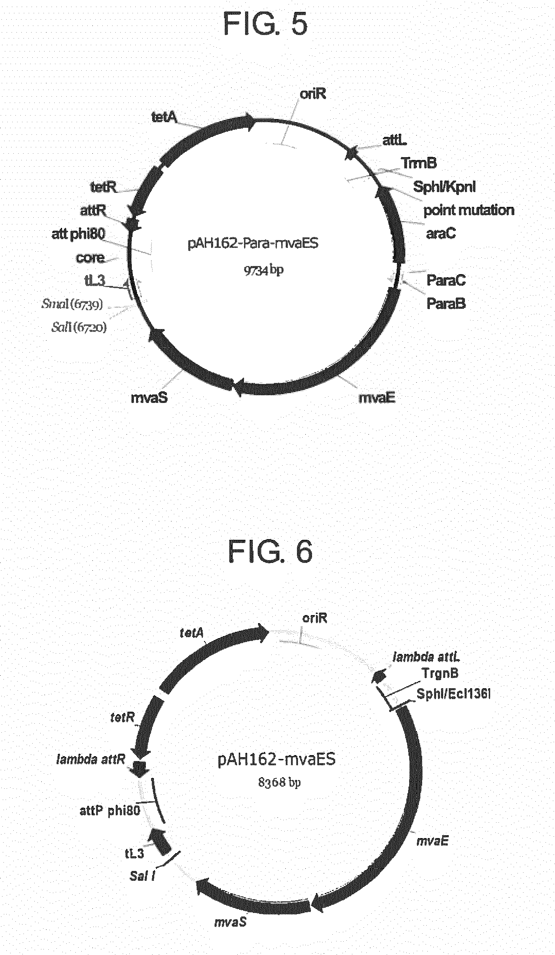

KpnI-SalI fragment of pMW-Para-mvaES-Ttrp was cloned into SphI-SalI recognition sites of pAH162-λattL-TcR-λattR. As a result, the pAH162-Para-mvaES plasmid carrying mvaES operon from *E. faecalis* under control of the *E. coli* Para promoter and repressor gene araC have been constructed (FIG. 5) .

In order to obtain a variant of promoter-deficient operon, an Ecl136II-SalI fragment of pMW219-Para-mvaES-Ttrp was subcloned into the same integrative vector. A map of the resulting plasmid is shown in FIG. 6.

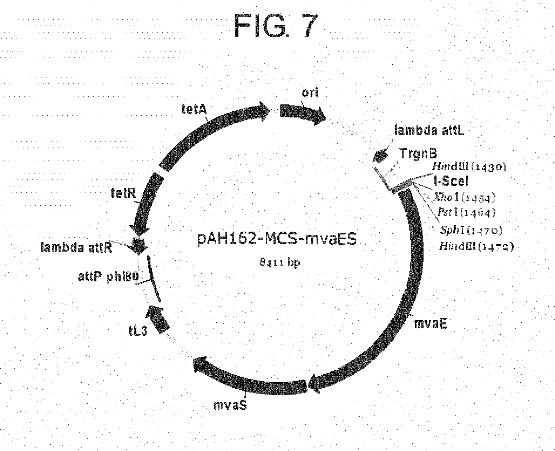

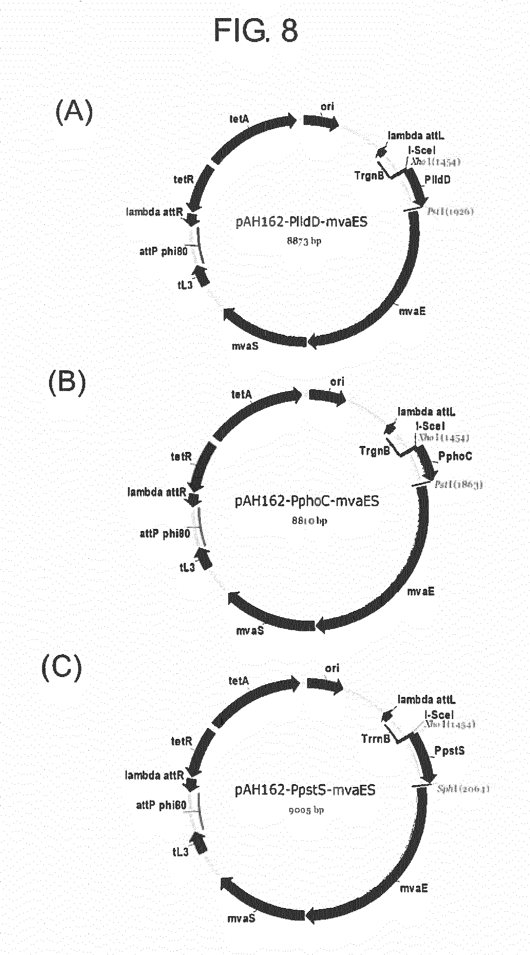

A set of plasmids for chromosome fixation, which retained the mvaES gene under the control of a different promoter was constructed. For this purpose, a polylinker containing I-SceI, XhoI, PstI and SphI recognition sites was inserted into unique HindIII recognition site located upstream of the mvaES gene. In order to accomplish this purpose, annealing was carried out using the primers 1 and 2 (Table 2). After that the resulting double-stranded DNA fragment was 5' phosphorylated with polynucleotide kinase and the resulting phosphorylated fragment was inserted into a pAH162-mvaES plasmid cleaved with HindIII by a ligation reaction. The resulting pAH162-MCS-mvaES plasmid (FIG. 7) is convenient for cloning of a promoter while a desired orientation is kept before the mvaES gene. DNA fragments retaining a regulatory region of a lldD, phoC and pstS genes were formed by PCR with genomic DNA from *P.ananatis* SC17(0) strain (Katashkina JI et al., BMC Mol Biol., 2009; 10: 34) as the template using primers 3 and 4, primers 5 and 6, and primers 7 and 8 (Table 2), respectively, and cloned into an appropriate restriction enzyme recognition site of pAH162-MCS-mvaES. The resulting plasmids are shown in FIG. 8. The cloned promoter fragments were sequenced and confirmed to exactly correspond to predicted nucleotide sequences.

### 5-2-2) Construction of pAH162-Km-Ptac-KDyI plasmid for chromosome fixation

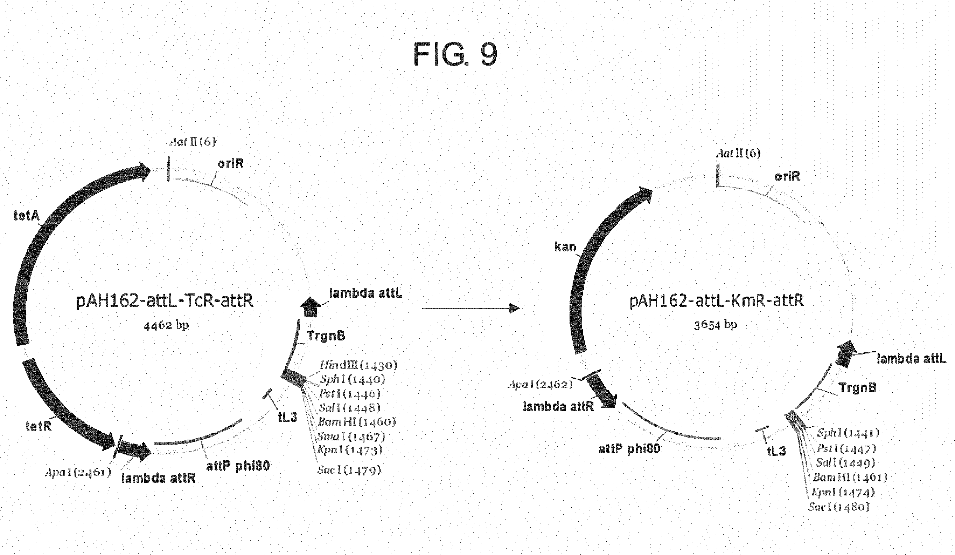

An AatII-ApaI fragment of pAH162-λattL-Tc^{R}-λattR containing a tetAR gene (Minaeva NI et al., BMC Biotechnol., 2008; 8: 63) was replaced with a DNA fragment obtained by PCR with a pUC4K plasmid (Taylor LA and Rose RE., Nucleic Acids Res., 16, 358, 1988) as the template using the primers 9 and 10 (Table 2). As a result, pAH162-λattL-Km^{R}-λattR was obtained (FIG. 9).

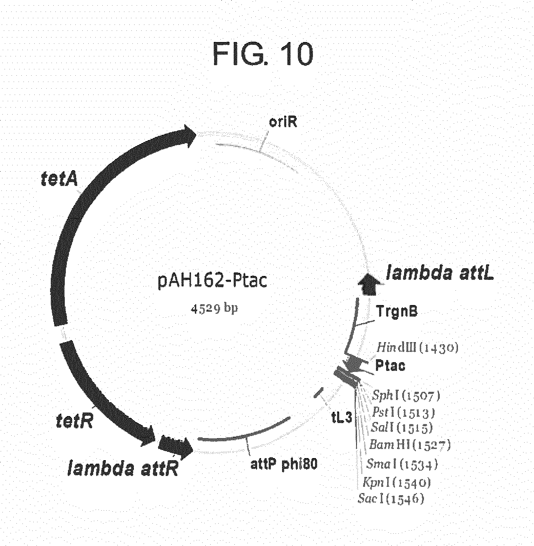

A P_{tac} promoter was inserted into a HindIII-SphI recognition site of the pAH162-λattL-Tc^{R}-λattR vector (Minaeva NI et al., BMC Biotechnol., 2008; 8: 63). As a result, an expression vector pAH162-P_{tac} for the chromosome fixation was constructed. The cloned promoter fragment was sequenced and confirmed to be the sequence as designed. A map of pAH162-P_{tac} is shown in FIG. 10.

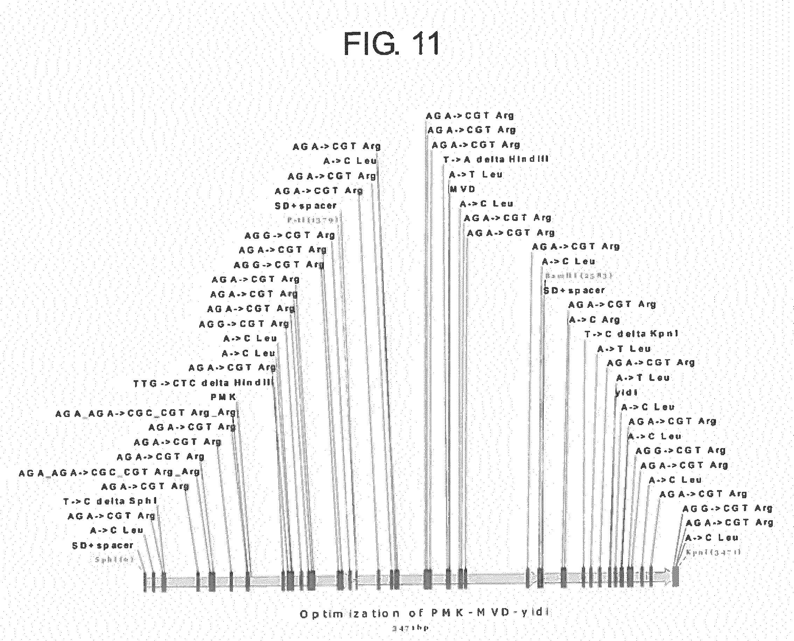

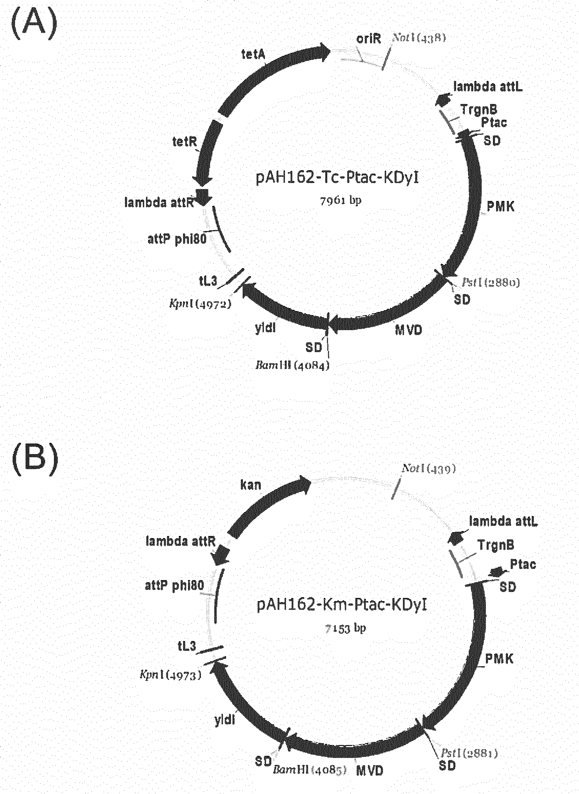

A DNA fragment that retained a PMK, MVD and yIdI genes derived from *S. cerevisiae,* in which rare codons had been replaced with synonymous codons, and had been synthesized by ATG Service Gene (Russia) (FIG. 11) was subcloned into a SphI-KpnI restriction enzyme recognition site of the vector pAH162-Ptac for the chromosome fixation. The DNA sequence including synthesized KDyI operon is shown in SEQ ID NO:70. The resulting plasmid pAH162-Tc-Ptac-KDyI retaining a Ptac-KDyI expression cassette is shown in FIG. 12(A). Subsequently, for the purpose of replacing a drug resistant marker gene, a NotI-KpnI fragment of pAH162-Tc-P_{tac}-KDyI retaining the tetAR gene was replaced with a corresponding fragment of pAH162-λattL-Km^{R}-λattR. As a result, a plasmid pAH162-Km-Ptac-KDyI having a kanamycin resistant gene, kan as a marker was obtained (FIG. 12(B)).

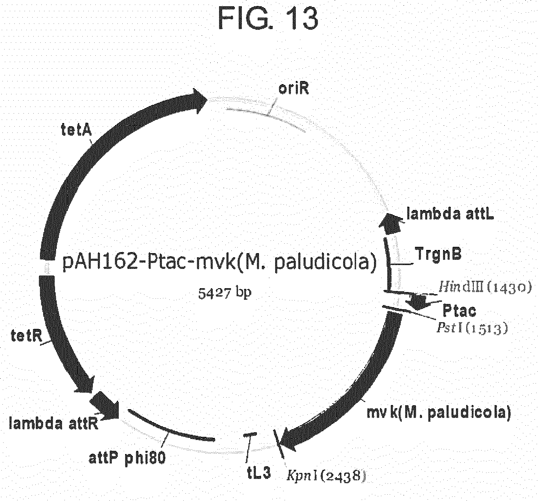

A chemically synthesized DNA fragment containing a coding region of a putative mvk gene derived from SANAE (for full-length genomic sequence, see GenBank Accession Number AP011532) that was strain of *Methanocella paludicola,* which had been ligated to a classical SD sequence, was cloned into a PstI-KpnI recognition site of the above integrative expression vector pAH162-P_{tac}. A map of the plasmid for the chromosome fixation retaining the mvk gene is shown in FIG. 13.

### 5-3) Construction of recipient strain SC17(0) ΔampC: : attBₚₕᵢ₈₀ ΔampH: : attBₚₕᵢ₈₀ Δcrt: : P_{tac}-mvk (M. paludicola)

Using two-stage technique including λ-Red dependent integration of a PCR amplified DNA fragment containing the kan gene flanked by attLₚₕᵢ₈₀ and attRₚₕᵢ₈₀ and 40 bp sequences homologous to a target chromosome site (Katashkina JI et al., BMC Mol Biol., 2009; 10: 34), and subsequent phi80 Int/Xis dependent removal of the kanamycin resistant marker (Andreeva IG et al., FEMS Microbiol Lett., 2011; 318(1): 55-60), chromosomal modifications of ΔampH: : attBₚₕᵢ₈₀ and ΔampC: : attBₚₕᵢ₈₀ was introduced into P. *ananatis* SC17(0) strain in a stepwise fashion. SC17(0) is a λ-Red resistant derivative of *P. ananatis* AJ13355 (Katashkina JI et al., BMC Mol Biol., 2009; 10: 34); an annotated full-length genomic sequence of *P. ananatis* AJ13355 is available as PRJDA162073 or GenBank Accession Numbers AP012032.1 and AP012033.1. Using pMWattphi plasmid [Minaeva NI et al., BMC Biotechnol.,2008;8:63] as the template and using primers 11 and 12, and primers 13 and 14 (Table 2) as the primers, DNA fragments used for integration into an ampH and ampC gene regions, respectively, were formed. The primers 15 and 16, and the primers 17 and 18 (Table 2) were used to verify the resulting chromosome modifications by PCR.

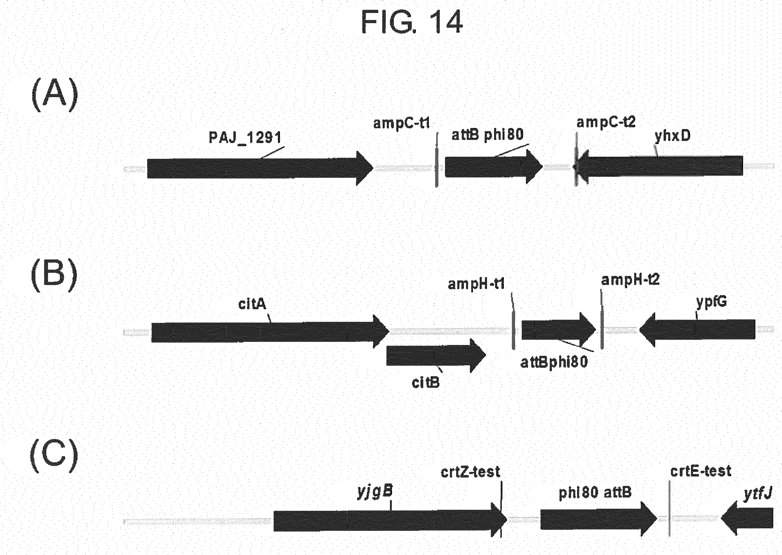

In parallel, a derivative of *P. ananatis* SC17(0) retaining an attB site of phi80 phage in place of a crt operon located on pEA320 320 kb megaplasmid that was a part of *P. ananatis* AJ13355 genome was constructed. In order to obtain this strain, λ-Red dependent integration of PCR-amplified DNA fragment retaining attLₚₕᵢ₈₀-kan-attRₚₕᵢ₈₀ flanked by a 40 bp region homologous to a target site in genome was carried out according to the previously described technique (Katashkina JI et al., BMC Mol Biol., 2009; 10: 34). Therefore, a DNA fragment to be used in the replacement of the crt operon with attLₚₕᵢ₈₀-kan-attRₚₕᵢ₈₀ was amplified in the reaction using the primers 19 and 20 (Table 2). A pMWattphi plasmid (Minaeva NI et al., BMC Biotechnol., 2008; 8: 63) was used as template in this reaction. The resulting integrated product was designated as SC17 (0) Δcrt: :attLₚₕᵢ₈₀-kan-attRₚₕᵢ₈₀. The primers 21 and 22 (Table 2) were used to verify the chromosome structure of SC17 (0) Δcrt: : attLₚₕᵢ₈₀-kan-attRₚₕᵢ₈₀ by PCR. The kanamycin resistance marker was removed from the constructed strain according to the reported technique using a pAH129-cat helper plasmid (Andreeva IG et al., FEMS Microbiol Lett., 2011; 318(1): 55-60). The Oligonucleotides 21 and 22 were used to verify the resulting SC17 (0) Δcrt: :attBₚₕᵢ₈₀ strain by PCR. Maps of genome-modified products, ΔampC: :attBₚₕᵢ₈₀, ΔampH: :attBₚₕᵢ₈₀ and Δcrt: :attBₚₕᵢ₈₀ are shown in FIG. 14 (A), (B) and (C), respectively.

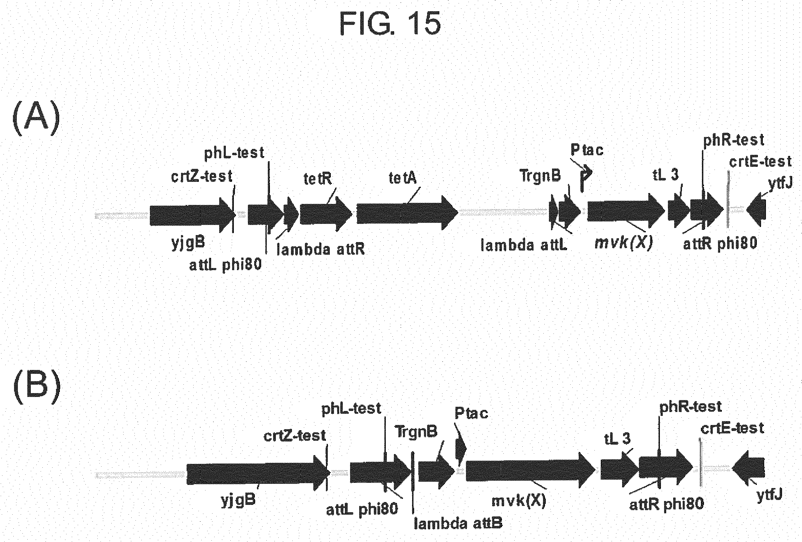

The aforementioned pAH162-Ptac-mvk (*M. paludicola*) plasmid was integrated into an attBₚₕᵢ₈₀ site of SC17(0) Δcrt::attBₚₕᵢ₈₀ according to the reported protocol (Andreeva IG et al., FEMS Microbiol Lett., 2011; 318(1): 55-60). The integration of the plasmid was confirmed by the polymerase chain reaction using the primers 21 and 23 and the primers 22 and 24 (Table 2). As a result, SC17(0) Δcrt::pAH162-P_{tac}-mvk (*M. paludicola*) strain was obtained. A map of the modified genome of Δcrt::pAH162-P_{tac}-mvk (*M. paludicola*) is shown in FIG. 15(A).

Subsequently, a genetic trait of SC17(0) Δcrt::pAH162-P_{tac}-mvk *(M. paludicola)* was transferred to SC17(0) ΔampC: : attBₚₕᵢ₈₀ ΔampH: : attBₚₕᵢ₈₀ via a genome DNA electroporation method (Katashkina JI et al., BMC Mol Biol., 2009; 10: 34). The resulting strain utilizes a tetracycline resistant gene, tetRA as the marker. Vector part of the pAH162-Ptac-mvk *(M. paludicola)* integrative plasmid including tetRA marker genes was eliminated using the reported pMW-intxis-cat helper plasmid (Katashkina JI et al., BMC Mol Biol., 2009; 10: 34). As a result, SC17(0) ΔampH: : attB_{ϕ80} ΔampC: :attB_{ϕ80} Δcrt: : P_{tac}-mvk (*M*. *paludicola*) with deletion of the marker gene was obtained. A map of the modified genome of Δcrt: :P_{tac}-mvk (*M. paludicola*) is shown in FIG. 15(B).

### 5-4) Construction of set of SWITCH strains

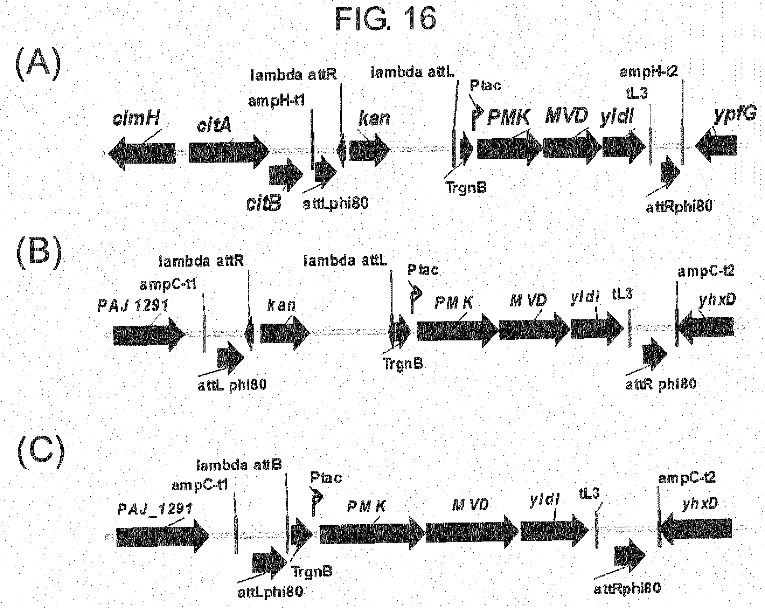

The pAH162-Km-Ptac-KDyI plasmid was integrated into a chromosome of SC17(0) ΔampH: :attB_{ϕ80} ΔampC: :attB_{ϕ80} Δcrt::P_{tac}-mvk (*M*. *paludicola*)/pAH123-cat strain according to the reported protocol (Andreeva IG et al., FEMS Microbiol Lett. 2011; 318(1): 55-60). The cells were seeded on an LB agar containing 50 mg/L of kanamycin. A grown Km^{R} clone was examined by PCR using the primers 11 and 15 and the primers 11 and 17 (Table 2). Strains retaining the pAH162-Km-Ptac-KDyI plasmid integrated into ΔampH::attB_{ϕ80} or ΔampC: :attB_{ϕ80}m were chosen. Maps of the modified chromosomes of ΔampH::pAH162-Km-Ptac-KDyI and ΔampC::pAH162-Km-Ptac-KDyI are shown in FIG. 16(A) and (B).

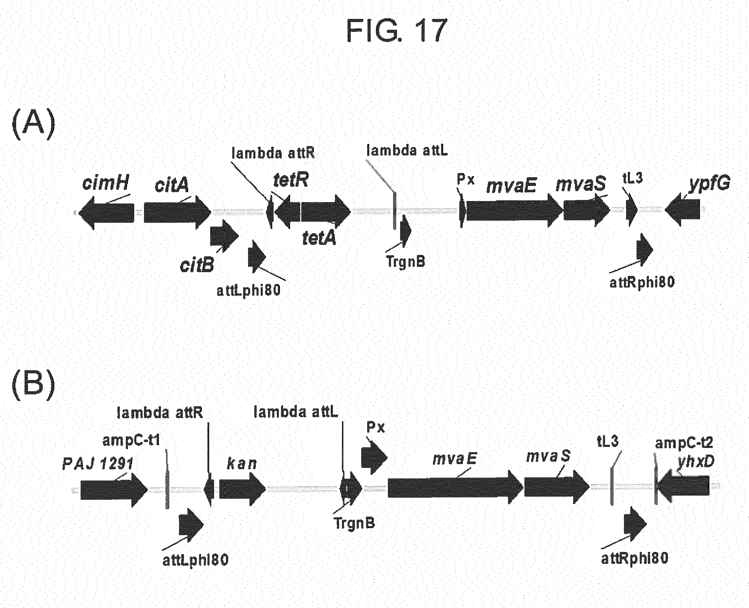

pAH162-Px-mvaES (here, Px is one of the following regulatory regions: araC-Pₐᵣₐ (*E. coli*), P_{11dD}, P_{phoC}, P_{pstS}) was inserted into the attBₚₕᵢ₈₀ site of SC17(0) ΔampC::pAH162-Km-P_{tac}-KDyI ΔampH: :attBₚₕᵢ₈₀ Δcrt: :P_{tac}-mvk(M. *paludicola*) and SC17(0) ΔampC::attBₚₕᵢ₈₀ AampH: :pAH162-Km-P_{tac}-KDyI Δcrt: : P_{tac}-mvk *(M. paludicola)* recipient strains using a pAH123-cat helper plasmid according to the reported protocol (Andreeva IG et al., FEMS Microbiol Lett., 2011; 318(1): 55-60). As a result, two sets of strains designated as SWITCH-Px-1 and SWITCH-Px-2 were obtained. Maps of the modified chromosomes of ΔampH: :pAH162-Px-mvaES and ΔampC::pAH162-Px-mvaES are shown in FIG. 17.

**Table 2. Primer sequences utilized in Example 5**

| N° | Name | Sequence 5'-> 3' |
|---|---|---|
| 1 | Linker-F | AGCTTTAGGGATAACAGGGTAATCTCGAGCTGCAGGCATGCA (SEQ ID NO:46) |
| 2 | Linker-R | AGCTTGCATGCCTGCAGCTCGAGATTACCCTGTTATCCCTAA (SEQ ID NO:47) |
| 3 | lldD5'CAS | TTTTTAAGCTTTAGGGATAACAGGGTAATCTCGAGATTTAAAGCGGCTGCTTTAC (SEQ ID NO:48) |
| 4 | lldD3'CAS | TTTTTAAGCTTGCATGCCTGCAGTATTTAATAGAATCAGGTAG (SEQ ID NO:49) |
| 5 | phoC5'CAS | TTTTTAAGCTTTAGGGATAACAGGGTAATCTCGAGTGGATAACCTCATGTAAAC (SEQ ID NO:50) |
| 6 | phoC3' CAS | TTTTTAAGCTTGCATGCCTGCAGTTGATGTCTGATTATCTCTGA (SEQ ID NO:51) |
| 7 | pstS5'CAS | TTTTTAAGCTTTAGGGATAACAGGGTAATCTCGAGAGCCTCTACGCGTGAATC (SEQ ID NO:52) |
| 8 | pstS3'CAS | TTTTTAAGCTTGCATGCCTGCAGAGGGGAGAAAAGTCAGGCTAA (SEQ ID NO:53) |
| 9 | n67 | TGCGAAGACGTCCTCGTGAAGAAGGTGTTGCTG (SEQ ID NO:54) |
| 10 | n68 | TGCGAAGGGCCCCGTTGTGTCTCAAAATCTCTGATG (SEQ ID NO:55) |
| 11 | ampH-attL-phi80 | |
| 12 | ampH-attR-phi80 | |
| 13 | DampC-phL | |
| 14 | DampC-phR | |
| 15 | ampH-t1 | GCGAAGCCCTCTCCGTTG (SEQ ID NO:60) |
| 16 | ampH-t2 | AGCCAGTCAGCCTCATCAGCG (SEQ ID NO:61) |
| 17 | ampC-t1 | GATTCCCACTTCACCGAGCCG (SEQ ID NO:62) |
| 18 | ampC-t2 | GGCAGGTATGGTGCTCTGACG (SEQ ID NO:63) |
| 19 | crtE-attRphi80 | |
| 20 | crtZ-attLphi80 | |
| 21 | crtZ-test | CCGTGTGGTTCTGAAAGCCGA (SEQ ID NO:66) |
| 22 | crtE-test | CGTTGCCGTAAATGTATCCGT (SEQ ID NO:67) |
| 23 | phL-test | GGATGTAAACCATAACACTCTGCGAAC (SEQ ID NO:68) |
| 24 | phR-test | GATTGGTGGTTGAATTGTCCGTAAC (SEQ ID NO:69) |

### 5-5) Introduction of isoprene synthase expression plasmid

Competent cells of SWITCH strains were prepared according to a standard method, and pSTV28-Ptac-IspSM (WO2013/179722) that was an expression vector for isoprene synthase derived from mucuna was introduced thereto by the electroporation. The resulting isoprenoid compound-forming microorganisms were designated as SWITCH-Para/IspSM, SWITCH-Plld/IspSM, SWITCH-PpstS/IspSM, and SWITCH-PphoC/IspSM.

### Example 6: Cultivation of phosphate deficiency-inducible isoprenoid compound-forming microorganisms SWITCH-PphoC/IspSM and SWITCH-PpstS/IspSM and arabinose-inducible isoprenoid compound-forming microorganism SWITCH-Para/IspSM

### 6-1) Cultivation of isoprenoid compound-forming microorganisms (SWITCH-Para/ispSM, SWITCH-PphoC/ispSM, SWITCH-PpstS/ispSM)

A fermentation jar having a 1L volume was used for the cultivation of the isoprenoid compound-forming microorganisms (SWITCH-Para/ispSM, SWITCH-PphoC/ispSM, SWITCH-PpstS/ispSM). The glucose medium was prepared in the composition shown in Table 3. Each of the isoprenoid compound-forming microorganism was applied onto an LB plate containing chloramphenicol (60 mg/L), and cultured at 34°C for 16 hours. After adding 0.3 L of the glucose medium into the fermentation jar having the 1 L volume, The microbial cells sufficiently grown on one plate were inoculated thereto, and the culture was started. The culture was carried out under a condition at pH 7.0 (controlled by ammonia gas) at 30°C, and air was supplied at 150 mL/minute. When an aerobic cultivation was carried out, the dissolved oxygen (DO) in the culture medium was measured using the galvanic mode DO sensor SDOU model (supplied from ABLE & Biott Co., Ltd), and controlled by stirring so that DO was a given concentration. During the cultivation, a solution of glucose adjusted to 500 g/L was continuously added so that a glucose concentration in the culture medium was 10 g/L or more. An OD value was measured at 600 nm using the spectrophotometer (HITACHI U-2900).

**[Table 3]**

| Group A | Final concentration |
|---|---|
| Glucose | 80 g/L |
| MgSO₄.7aq | 2.0 g/L |

| Group B | Final concentration |
|---|---|
| (NH₄)₂SO₄ | 2.0 g/L |
| KH₂PO₄ | 2.0 g/L |
| FeSO₄.7aq | 20 mg/L |
| MnSO₄.5aq | 20 mg/L |
| Yeast Extract | 4.0 g/L |

Each 0.15 L of Group A and Group B was prepared, and then sterilized with heat at 115°C for 10 minutes. After cooling, Group A and Group B were mixed, and chloramphenicol (60 mg/L) was added to use as the medium.

### 6-2) Method of inducing isoprene production phase

In an arabinose-inducible isoprenoid compound-forming microorganism, genes upstream of the mevalonate pathway are expressed by an arabinose inducible promoter, and thus an amount of isoprene produced in the presence of L-arabinose (Wako Pure Chemical Industries, Ltd.) is notably enhanced. To induce to an isoprene production phase, a broth in the fermentation jar was analyzed with time, and L-arabinose was added so that its final concentration was 20 mM at a time point when the OD value was 16.

In a phosphorus e deficiency-inducible isoprenoid compound-forming microorganism, genes upstream of the mevalonate pathway are expressed by a phosphorus deficiency-inducible promoter, and thus an amount of isoprene produced is notably enhanced when a concentration of phosphorus in the culture medium becomes a certain concentration or below.

### 6-3) Method of measuring concentration of isoprene in fermentation gas and method of measuring concentration of total phosphorus in culture medium

The isoprene concentration in the fermentation gas was a multi-gas analyzer (F10, supplied from GASERA). The concentration of total phosphorus in the culture medium was measured using a phosphate C-Test Wako (Wako Pure Chemical Industries Ltd.).

### 6-4) Amounts of isoprene formed in jar culture of arabinose-inducible isoprenoid compound-forming microorganism and phosphorus deficiency-inducible isoprenoid compound-forming microorganism

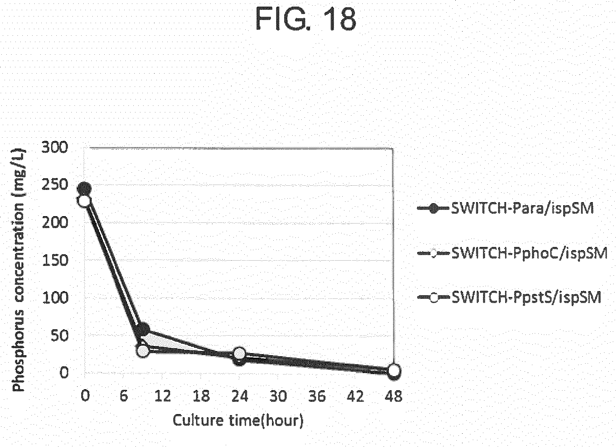

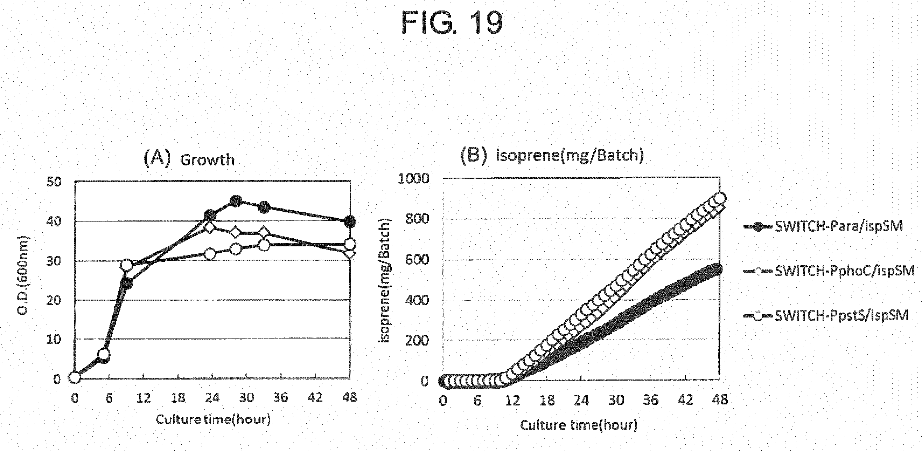

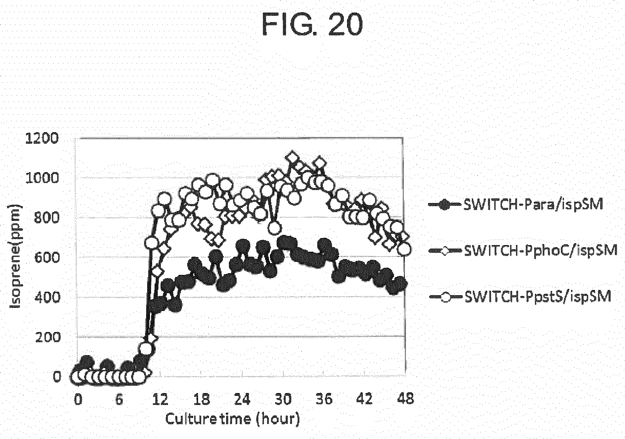

The arabinose-inducible isoprenoid compound-forming microorganism (SWITCH-Para-ispSM) and the phosphorus deficiency-inducible isoprenoid compound-forming microorganisms (SWITCH-PphoC/ispSM, SWITCH-PpstS/ispSM) were cultured under the above jar culture condition, and amounts of formed isoprene (mg/batch) and the isoprene concentration (ppm) in the fermentation gas were measured (FIG.19 and 20). During the cultivation, as shown in FIG. 18, the concentration of total phosphorus became 50 mg/L or less at 9 hours after starting the cultivation, and the production of isoprene was detected at the same timing in SWITCH-PphoC/ispSM and SWITCH-PpstS/ispSM. A period of time required from the start of the isoprene formation to a time at which a maximum rate of the isoprene formation was observed was shorter, and the formation rate increased more rapidly in SWITCH-PphoC/ispSM and SWITCH-PpstS/ispSM than in SWITCH-Para/ispSM (FIG. 19). The amounts of isoprene formed for 48 hours of the cultivation were 563 mg, 869 mg, and 898 mg in SWITCH-Para/ispSM, SWITCH-PphoC/ispSM and SWITCH-PpstS/ispSM, respectively (FIG. 19). This results indicated that the phosphorus deficiency-inducible isoprenoid compound-forming microorganism induced the isoprene formation under the condition where the concentration of phosphorus was 50 mg/L or less and had a more excellent ability to produce isoprene than the arabinose-inducible isoprenoid compound-forming microorganism.

### Example 7: Cultivation of microaerophilically inducible isoprenoid compound-forming microorganism (SWITCH-Plld/IspSM) and Cultivation of arabinose-inducible isoprenoid compound-forming microorganism (SWITCH-Para/IspSM)

### 7-1) Cutivation of isoprenoid compound-forming microorganisms (SWITCH-Para/ispSM, SWITCH-Plld/ispSM)

The isoprenoid compound-forming microorganisms (SWITCH-Para/ispSM, SWITCH-Plld/ispSM) were cultured in the same condition as in 6-1) above.

### 7-2) Method of inducing isoprene production phase

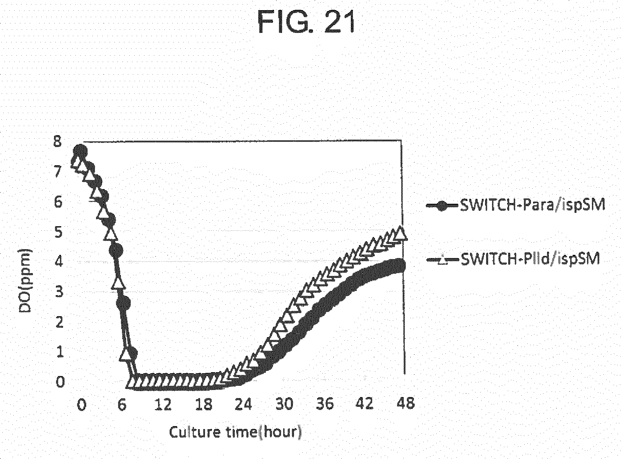

In the microaerobically inducible isoprenoid compound-forming microorganism (SWITCH-lld/ispSM), the isoprene-production phase was induced by supplying an air containing 20% (v/v) oxygen into the culture medium and regulating the stirring frequency during the cultivation to make the dissolved oxygen in the culture medium to be 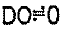 ppm. Changes with time of the dissolved oxygen concentration in the culture medium are shown in FIG. 21.

### 7-3) Method of measuring isoprene concentration in fermentation gas and method of measuring dissolved oxygen in culture medium

The isoprene concentration in the fermentation gas was the multi-gas analyzer (F10, supplied from GASERA). The dissolved oxygen concentration in the culture medium was measured using the galvanic mode DO sensor SDOU model (supplied from ABLE & Biott Co., Ltd).

### 7-4) Amounts of isoprene formed in jar culture of arabinose-inducible isoprenoid compound-forming microorganism and microaerobically inducible isoprenoid compound-forming microorganism.

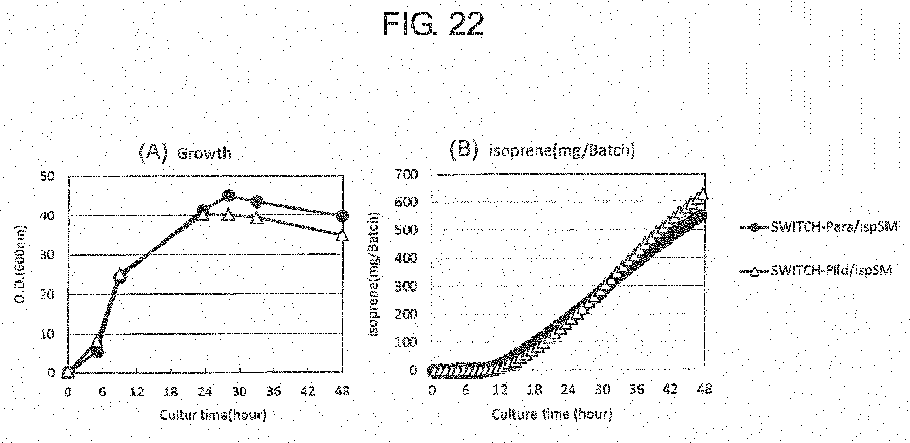

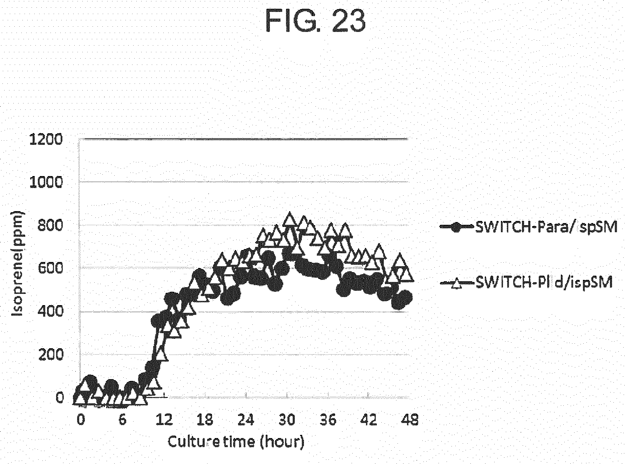

The arabinose-inducible isoprenoid compound-forming microorganism (SWITCH-Para/ispSM) and the microaerobically inducible isoprenoid compound-forming microorganism (SWITCH-lld-ispSM) were cultured under the above jar culture condition, and the amounts of formed isoprene (mg/batch) and the isoprene concentration (ppm) in the fermentation gas were measured (FIGS. 22 and 23). As shown in FIG. 21, the dissolved oxygen concentration in the culture medium reached 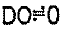 ppm at 8 hours after starting the cultivation, and shortly after, the production of isoprene was detected. The amounts of isoprene formed for 48 hours of the cultivation were 563 mg and 642 mg in SWITCH-Para/ispSM and SWITCH-Plld/ispSM, respectively (FIG. 22). This result indicated that the microaerobically inducible isoprenoid compound-forming microorganism induced the formation of isoprene under the condition of 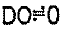 ppm or less and had the ability to produce isoprene, which was equivalent to that of the arabinose-inducible isoprenoid compound-forming microorganism.

### Reference Example 1) Construction of E. coli MG1655 Ptac-KDyI strain

*E. coli* MG1655 Ptac-KDyI strain was made by deleting an ERG12 gene in MG1655 Ptac-KKDyI strain (see Example 7-5 in WO2013/179722). A specific procedure is as follows.

A plasmid pKD46 having a temperature sensitive replication capacity was introduced into MG1655 Ptac-KKDyI strain by the electroporation method. The plasmid pKD46 (Proc. Natl. Acad. Sci. USA, 2000, vol.97, No.12, p6640-6645) contains a DNA fragment of total 2154 bases of λ phage (GenBank/EMBL Accession Number: J02459, 31088^{th} to 33241^{st}) containing genes (λ, β, exo genes) of a λ-Red system controlled by an arabinose-inducible ParaB promoter. Competent cells of MG1655 Ptac-KKDyI strain were prepared, and then pKD46 was introduced thereto by the electroporation method. The cells were evenly applied onto an LB plate containing ampicillin (100 mg/L), and cultured at 37°C for 18 hours. Subsequently, transformants exhibiting ampicillin resistance were obtained from the resulting plate. A strain in which pKD46 had been introduced into *E.coli* MG1655 Ptac-KDDyI strain was designated as MG1655 Ptac-KDDyI/pKD46. PCR was carried out with attL-tetR-attR-Ptac gene fragment (SEQ ID NO:38 in WO2013/179722) as the template using synthesized oligonucleotides consisting of SEQ ID NO:39 and SEQ ID NO:40 in WO2013/179722 and using Prime Star polymerase (supplied from Takara Bio Inc.). A reaction solution was prepared according to the composition attached to the kit, and DNA was amplified through 30 cycles of reactions at 98°C for 10 seconds, 55°C for 5 seconds and 72°C for one minute per kb. As a result, an MVK gene deficient fragment containing attL-tetR-attR-Ptac was obtained. Competent cells of MG1655 Ptac-KDDyI/pKD46 were prepared, and then the purified MVK gene deficient fragment containing attL-tetR-attR-Ptac was introduced thereto by the electroporation method. After the electroporation, a colony that had acquired tetracycline resistance was obtained. PCR reaction was carried out using synthesized oligonucleotides consisting of SEQ ID NO:41 and SEQ ID NO:42 in WO2013/179722 as the primers to confirm that the ERG12 gene on the chromosome was deficient. The obtained mutant was designated as *E. coli* MG1655 Ptac-KDyI.

### Reference Example 2) Construction of arabinose-inducible mvaES expression vector (pMW-Para-mvaES-Ttrp)

An arabinose-inducible expression vector for mevalonate pathway upstream genes was constructed by the following procedure. A PCR fragment containing Para consisting of araC and araBAD promoter sequences derived from *E. coli* was obtained by PCR with the plasmid pKD46 as the template using synthesized oligonucleotides represented by SEQ ID NO:49 and SEQ ID NO:50 in WO2013/179722 as the primers. A PCR fragment containing the EFmvaE gene was obtained by PCR with the plasmid pUC57-EFmvaE as the template using the synthesized oligonucleotides represented by SEQ ID NO:51 and SEQ ID NO:52 in WO2013/179722 as the primers. A PCR fragment containing the EFmvaS gene was obtained by PCR with the plasmid pUC57-EFmvaS as the template using the synthesized oligonucleotides represented by SEQ ID NO:53 and SEQ ID NO:54 in WO2013/179722 as the primers. A PCR fragment containing a Ttrp sequence was obtained by PCR with the plasmid pSTV-Ptac-Ttrp as the template (source of the plasmid) using the synthesized oligonucleotides represented by SEQ ID NO:55 and SEQ ID NO:56 in WO2013/179722 as the primers. Prime Star polymerase (TAKARA BIO Inc.) was used for PCR for obtaining these four PCR fragments. Reaction solutions were prepared according to the composition attached to the kit, and DNA was amplified through 30 cycles of the reactions at 98°C for 10 seconds, 55°C for 5 seconds and 72°C for one minute per kb. PCR with the purified PCR product containing Para and the PCR product containing the EFmvaE gene as the template was carried out using the synthesized oligonucleotides represented by SEQ ID NO:49 and SEQ ID NO:52 in WO2013/179722 as the primers. PCR with the purified PCR product containing the EFmvaS gene and the PCR product containing Ttrp as the template was also carried out using the synthesized oligonucleotides represented by SEQ ID NO:53 and SEQ ID NO:56 in WO2013/179722 as the primers. As a result, a PCR product containing Para and the EFmvaE gene and a PCR product containing the EFmvaS gene and Ttrp were obtained. A plasmid pMW219 (supplied from Nippon Gene Co., Ltd.) was digested with SmaI according to a standard method. Then, pMW219 after being digested with SmaI was ligated to the PCR product containing Para and the EFmvaE gene and the PCR product containing the EFmvaS gene and Ttrp using In-Fusion HD Cloning Kit (supplied from Clontech). The obtained plasmid was designated as pMW-Para-mvaES-Ttrp.

### Phosphate starvation induction

### (Background)

In this study, the transfer from a cell growth phase to a substance-production phase is realized by a metabolic switch to respond to phosphate starvation. Generally, an optimal metabolic condition is different between the growth phase and the substance-production phase. Conventionally, methods of optimizing the metabolic condition in each phase have been known, but even if a culture condition optimal for the growth phase is switched to a culture condition optimal for the substance production phase, this switch often does not work well due to reasons such as reduced cellular activity.

It has been known that the decrease of a phosphate concentration in a cell reduces an acquired amount of ATP (Schuhmacher, T., Löffler, M., Hurler, T., Takors, R., 2014. Phosphate limited fed-batch processes: Impact on carbon usage and energy metabolism in Escherichia coli, J. Biotechnol., doi: 10.1016/j.jbiotec.2014.04.025). Thus, the decrease of the phosphate concentration is predicted to reduce a production rate of a metabolite in the production of the metabolite that requires a high ATP amount.

Multiple stages of phosphate reactions using ATP as a substrate are present in the mevalonate pathway that is a formation pathway of isoprene (Michelle C Y Chang & Jay D Keasling, Production of isoprenoid pharmaceuticals by engineered microbes, Nature Chemical Biology 2, 674-681 (2006)). Thus, isoprene fermentation is thought to be the production of the metabolite that requires the high APT amount. Therefore, it was easily feared that the cultivation of an isoprene-producing strain having the mevalonate pathway at low phosphate concentration caused a decreased amount of produced isoprene.

It has been known in literatures that the response to the phosphate starvation rapidly acts upon expression control of a gene (Baek JH et al., J Microbiol Biotechnol. 2007 Feb; 17 (2) : 244-52; WO2003054140 A2). However, it cannot be easily expected that the metabolic condition is rapidly switched by the response to the phosphate starvation and a quick response is observed at level of substance production under a condition where inhibition at enzymatic level are known in combination of the response to the phosphate starvation with control of constitutively expressed genes.

### (Example of effect observed by study)

The higher concentration of isoprene was identified in the phosphate starvation-inducible isoprene-producing strain constructed by us than the arabinose-inducible isoprene-producing strain that was a control.

Also as an unexpected effect, rapid responsiveness (time required to reach a maximum isoprene concentration) was observed (Example 6, FIG. 19). IPTG has been mainly used as the inducer in previous cases of the isoprene fermentation (US 8,288,148 B2; US 8, 361, 762 B2; US 8,470,581 B2; US 8, 569, 026 B2; US 8,507,235 B2; US 8,455,236 B2; US 2010-0184178 A1). In these cases, it takes about 8 to 22 hours for an ability per microbial cell to produce isoprene to reach the maximum or for an accumulated isoprene to reach the maximum after adding the inducer. On the contrary, when the technique for the phosphate starvation shown in Example 6, the isoprene gas concentration in the reactor reached the maximum within 3 to 6 hours.

**Table 4. Comparison of responsiveness by difference of technique for induction**

| | | | | | |
|---|---|---|---|---|---|
| | | Induction method | | | |
| | | Arabinose | Microaerophilic | Phosphorus deficiency | |
| | | SWITCH-Para/ IspSM | SWITCH-Plld /IspSM | SWITCH-Ppho C/IspSM | **SWITCH-Ppst** S/IspSM |

| | | Example 7 | Example 7 | Example 6 | Example 6 |
|---|---|---|---|---|---|
| Induction time | hour | 21 | 21 | 6 | 3 |
| Induction index | ppm/vvm/h | 64.2 | 79 | 283 | 595 |

| | | | | | |
|---|---|---|---|---|---|
| Induction time: Time from start of isoprene formation (defined as concentration of 50 ppm) to maximum. Induction index: Value obtained by dividing maximum isoprene concentration (ppm/vvm) by induction time vvm: Volume per volume per minute (in the case of ventilation stirring, ventilation amount of gas per unit volume) | | | | | |

### Microaerophilic induction

### (Background)

In this study, the transfer from the cell growth phase to the substance-production phase is realized by change of metabolism caused by deficiency of the dissolved oxygen. Generally, it has been known that the metabolic condition in the cell is largely different between the culture condition where oxygen is available for a microorganism and the culture condition where oxygen is not available for the microorganism (Martinez I., Bennett G. N., San K. Y.. 2010. Metabolic impact of the level of aeration during cell growth on anaerobic succinate production by an engineered Escherichia coli strain. Metab. Eng. 12:499-509). Conventionally, methods of optimizing the metabolic condition under an aerobic condition and an anaerobic condition have been known. In order to perform the fermentation under an essentially anaerobic condition such as succinate and alcohol fermentation, it is often studied that applying this, the culture condition optimal for the growth phase is switched to the culture condition optimal for the substance production phase by changing the oxygen concentration in the cultivation (Blombach B, Riester T, Wieschalka S, Ziert C, Youn JW, Wendisch VF, Eikmanns BJ. Corynebacterium glutamicum tailored for efficient isobutanol production. Appl Environ Microbiol. 2011 May; 77(10): 3300-10). On the contrary, in the fermentation that requires that excess reducing capacity such as isoprene and glutamic acid is reoxidized by oxygen respiration, the condition where the dissolved oxygen is deficient is not regarded as the condition that leads to the metabolic condition suitable for the substance production phase. Thus, this method is not the method of transferring from the cell growth phase to the substance production phase, which is actively employed by sector peer companies.

The method in more detail is as follows. Under the aerobic condition, typically oxygen works as a terminal electron acceptor, thereby NADH is reoxidized (respiration). Under a low oxygen concentration environment such as a microaerophilic condition, an amount of supplied oxygen is a limiting factor, and an NADH concentration in a cell is increased. In *E. coli,* synthesis pathways for lactic acid and ethanol are present as reoxidation reaction of this excess NADH, and NADH is reoxidized in processes of producing these substances. Likewise in *P. ananatis,* 2,3-butandiol, lactic acid, and ethanol are synthesized under the low oxygen concentration environment to keep a balance between oxidation and reduction. That is, under the low oxygen concentration environment, metabolic flux to these substance is increased, and thus it is presumed that the amount of produced isoprene is decreased. When isoprene is produced via the mevalonate pathway, it is evident from calculation of a theoretic yield that excessive NADH is produced (Yadav GV et al., The future of metabolic engineering and synthetic biology: Towards a systematic practice, Metabolic Engineering, 14, 233-241, 2012). Typically, oxygen is needed for this reoxidation of NADH, and thus a person skilled in the art does not allow himself/herself to select the culture under the low oxygen concentration environment. In fact, in the study on isoprene production by *Saccharomyces cerevisiae,* it has been known that the growth of microbial cells and the ability to produce isoprene are enhanced under the aerobic condition where the dissolved oxygen is sufficiently supplied than in the microaerophilic condition where the dissolved oxygen is deficient, as a result of the comparative study of the culture conditions (Lv X et al., Journal of Biotechnology, 186, 128-136, 2014).

### (Example of effect observed by study)

When *E. aerogenes* was used as a parent strain of the isoprene-producing strain, it was demonstrated that GI08-Pbud/IspSK could successfully switch the growth phase to the isoprene-production phase when the dissolved oxygen (DO) concentration was almost zero (Example 4, FIG. 4).

When *P. ananatis* was used as a parent strain of the isoprene-producing strain, the growth phase was switched to the isoprene production phase by exposing to the condition where the dissolved oxygen (DO) concentration was almost zero, and this phase was transferred to the metabolic condition suitable for the isoprene production by increasing the dissolved oxygen concentration again in the isoprene production phase (Example 7, FIGS. 22 and 23). As shown in FIG. 23, the higher isoprene concentration than that in the arabinose-inducible isoprene-producing strain that was the control was confirmed.

### Example 8: Production of polyisoprene

Isoprene is collected with a trap cooled with liquid nitrogen by passing the fermentation exhaust. Collected of isoprene is mixed with 35g of hexane (Sigma-Aldrich, catalog No.) and 10g of silica gel (Sigma-Aldrich, catalog No. 236772) and 10g of alumina (Sigma-Aldrich, catalog No. 267740) under a nitrogen atmosphere in 100 mL glass vessel that is sufficiently dried. Resulting mixture is left at room temperature for 5 hours. Then supernatant liquid is skimmed and is added into 50 ml glass vessel that is sufficiently dried.

Meanwhile, in a glove box under a nitrogen atmosphere, 40.0 pmol of Tris[bis(trimethylsilyl)amido]gadrinium, 150.0 pmol of tributylaluminium, 40.0 µmol of Bis(2-(diphenylphosphino)phenyl]amine, 40.0 µmol of triphenylcarbonium tetrakis(pentafluorophenyl)borate (Ph3CBC6F5)4) are provided in a glass container, which was dissolved into 5 mL of toluene (Sigma-Aldrich, catalog No. 245511), to thereby obtain a catalyst solution. After that, the catalyst solution is taken out from the glove box and added to the monomer solution, which is then subjected to polymerization at 50°C for 120 minutes.

After the polymerization, 1 mL of an isopropanol solution containing, by 5 mass %, 2,2'-methylene-bis(4-ethyl-6-t-butylphenol) (NS-5), is added to stop the reaction. Then, a large amount of methanol is further added to isolate the copolymer, and the copolymer is vacuum dried at 70°C to obtain a polymer.

### Example 9: Production of rubber compound

The rubber compositions formulated as shown in Table 5 are prepared, which are vulcanized at 145°C for 35 minutes.

**Table 5**

| | Parts by Mass |
|---|---|
| Polyisoprene | 100 |
| Stearic Acid | 2 |
| Carbon Black (HAF class) | 50 |
| Anti Oxidant (*1) | 1 |
| Zinc Oxide | 3 |
| Cure Accelerator (*2) | 0.5 |
| Sulfur | 1.5 |

| | |
|---|---|
| *1 N-(1,3-dimethylbutyl)-N'-p-phenylenediamine *2 N-cyclohexyl-2-benzothiazolesulfenamide | |

### Example 10: Construction of SC17 (0) Δgcd and SWITCH-PphoC Δgcd strains, and introduction of isoprene synthase

The gcd gene in *P*. *ananatis* codes for glucose dehydrogenase, and it has been known that *P. ananatis* accumulates gluconate during aerobic growth (Andreeva IG et al., FEMS Microbiol Lett. 2011 May; 318(1):55-60*)*

The SC17 (0) Δgcd strain in which gcd gene is disrupted is constructed using λRed-dependent integration of DNA fragments obtained in PCRs with the primers gcd-attL and gcd-attR (Table 6) and pMW118-attL-kan-attR plasmid [Minaeva NI et al., BMC Biotechnol. 2008; 8:63] as a template. To verify the integrant, the primers gcd-t1 and gcd-t2 (Table 6) are used.

Genomic DNA of the SC17 (0) Δgcd strain is isolated using the Wizard Genomic DNA Purification Kit (Promega) and electro-transformed into the marker-less derivative of the SWITCH-PphoC strain according to the previously described method [Katashkina JI et al., BMC Mol Biol. 2009; 10:34]*.* As a result, the SWITCH-PphoC Δgcd (KmR) strain is obtained. The primers gcd-t1 and gcd-t2 (Table 6) are used for PCR analysis of the obtained integrant. The kanamycin resistant marker gene is obtained according to the standard λInt/Xis-mediated procedure [Katashkina JI et al., BMC Mol Biol. 2009; 10:34]. The obtained strain is designated as SWITCH-PphoC Δgcd strain.

Competent cells of SWITCH-PphoC Δgcd strain was prepared according to a standard method, and pSTV28-Ptac-IspSM (WO2013/179722) that was an expression vector for isoprene synthase derived from mucuna was introduced thereto by the electroporation. The resulting isoprenoid compound-forming microorganisms were designated as SWITCH-PphoC Δgcd/IspSM.

**Table 6. Primer List**

| Primer | Nucleotide sequence (SEQ ID NO:) |
|---|---|
| gcd-attL | |
| gcd-attR | |
| gcd-t1 | TGACAACAATCTATCTGATT (SEQ ID NO:73) |
| gcd-t2 | TGCGCCTGGTTAAGCTGGCG (SEQ ID NO:74) |

### Example 11: Evaluation of cultivation of SWITCH-PphoC Δgcd/IspSM

### 11-1) Condition for jar cultivation of isoprene-producing microorganism

A one liter volume fermenter was used for cultivation of isoprene-producing microorganisms (SWITCH-PphoC/IspSM and SWITCH-PphoCAgcd/IspSM). Glucose medium was conditioned in a composition shown in Table 7. Each of these isoprene-producing microorganisms was applied onto an LB plate containing chloramphenicol (60 mg/L), and cultured at 34°C for 16 hours. 0.3 L of the glucose medium was added to the one liter volume fermenter, and microbial cells that had sufficiently grown on the one LB plate were inoculated thereto to start the cultivation. As a culture condition, pH was 7.0 (controlled with ammonia gas), temperature was 30°C, and ventilation at 150 mL/minute was carried out. When aerobic cultivation was carried out, a concentration of oxygen in culture medium (dissolved oxygen (DO)) was measured using a galvanic type DO sensor SDOU model (ABLE Cooperation), and was controlled with stirring so that a DO value was 5%. During the cultivation, a glucose solution adjusted at 50 g/L was continuously added so that a concentration of glucose in the culture medium was 10 g/L or higher. The OD value was measured at 600 nm using a spectrophotometer (HITACHI U-2900). In the cultivation for 48 hours, SWITCH-PphoC/IspSM and SWITCH-PphoCΔgcd/IspSM consumed 63.9 g and 77.8 g of glucose, respectively.

**Table 7. Composition of glucose medium**

| Group A | (Final concentration) |
|---|---|
| Glucose | 80 g/L |
| MgSO₄·7aq | 2.0 g/L |
| | |

| Group B | |
|---|---|
| (NH₄)₂SO₄ | 2.0 g/L |
| KH₂PO₄ | 2.0 g/L |
| FeSO₄·7aq | 20 mg/L |
| MnSO₄·5aq | 20 mg/L |
| Yeast Extract | 4.0 g/L |

Each 0.15 L was prepared for Group A and Group B and sterilized with heating at 115°C for 10 minutes. After cooling, Group A and Group B were mixed, and chloramphenicol (60 mg/L) was added thereto to use as the medium.

### 11-2) Method of inducing isoprene production phase

A phosphorus-deficient isoprene-producing microorganism expresses genes upstream of a mevalonate pathway with a phosphorus deficiency-inducible promoter. Thus, when the concentration of phosphorus in the medium is below a certain concentration, the amount of produced isoprene is remarkably increased.

### 11-3) Method of measuring isoprene concentration in fermentation gas

A concentration of isoprene in fermentation gas was measured using a multi-gas analyzer (supplied from GASERA, F10) .

### 11-4) Method of measuring gluconic acid concentration in medium

Culture supernatant was diluted with pure water to 10 times, and filtrated through a 0.45 µm filter followed by being analyzed according to the following method using high performance liquid chromatography ELITE LaChrom (Hitachi High Technologies).

### <Separation condition>

Columns: Shim-pack SCR-102H (8 mm I.D.x300 mm L), tandemly connected two columns
Guard column: SCR-102H (6 mm I.D.x50 mm L)
Mobile phase: 5 mM p-toluenesulfonic acid
Flow: 0.8 mL/minute
Temperature: 50°C
Injection volume: 10 µL

### <Detection condition>

Buffer: 20 mM Bis-Tris aqueous solution containing 5 mM p-toluenesulfonic acid and 100 µM EDTA
Flow: 0.8 mL/minute
Detector: CDD-10 AD polarity + response SLOW, temperature: 53°C (column temperature: +3°C); scale 1×2⁴ µS/cm

### 11-5) Amount of produced isoprene in jar cultivation of isoprene-producing microorganisms (SWITCH-PphoC/IspSM and SWITCH-PphoCΔgcd/IspSM)

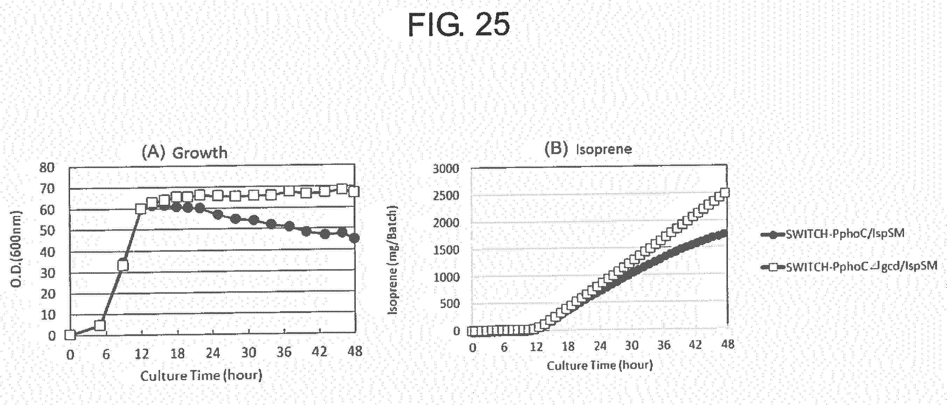

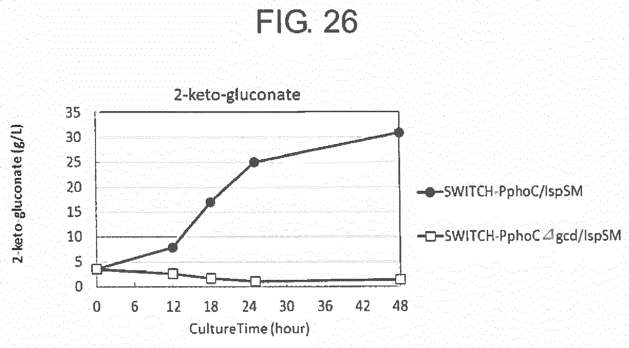

The isoprene-producing microorganisms (SWITCH-PphoC/IspSM and SWITCH-PphoCAgcd/IspSM) were cultured according to the jar cultivation condition as described above, and amounts of produced isoprene were measured. SWITCH-PphoC/IspSM exhibited a decreased O.D. value when production of isoprene was started (FIG.25(A)), and accumulated 30.9 g/L of 2-ketogluconic acid in the cultivation for 48 hours (FIG.26). SWITCH-PphoCAgcd/IspSM exhibited a constant O.D. value even after starting the production of isoprene (FIG.25(A)), and an accumulated amount of 2-ketogluconic acid in the cultivation for 48 hours was 1.4 g/L, which was an extremely low amount (FIG.26). The amounts of isoprene produced in the cultivation for 48 hours were 1771 mg and 2553 mg in SWITCH-PphoC/IspSM and SWITCH-PphoCΔgcd/IspSM, respectively (FIG.25(B)). From this result, it was shown that the production of 2-ketogluconic acid was suppressed while the amount of produced isoprene was increased in SWITCH-PphoCΔgcd/IspSM.

### Example 12: Construction of expression plasmid for linalool synthase

### 12-1) Acquisition of linalool synthase gene derived from Actinidia arguta (hardy kiwifruit)

A nucleotide sequence (GenBank accession number: GQ338153) and an amino acid sequence (GenPept accession number: ADD81294) of a linalool synthase (AaLINS) gene derived from *Actinidia arguta* have been already known. The amino acid sequence of a linalool synthase protein and the nucleotide sequence of its gene derived from *Actinidia arguta* are shown in SEQ ID NO:75 and SEQ ID NO:76, respectively. In order to efficiently express the AaLINS gene, codons were optimized to resolve a secondary structure, an AaLINS gene in which a chloroplast localization signal had been cleaved was designed, and this was designated as opt_AaLINS. A nucleotide sequence of opt_AaLINS is shown in SEQ ID NO:77. DNA in which a tac promoter region (deBoer, et al., (1983) Proc. Natl. Acad. Sci. USA., 80, 21-25) had been added to the opt_AaLINS gene was chemically synthesized, cloned into pUC57 (supplied from GenScript) and the resulting plasmid was designated as pUC57-Ptac-opt_AaLINS.

### 12-2) Acquisition of linalool synthase gene derived from Coriandrum sativum (coriander)

A nucleotide sequence (GenBank accession number: KF700700) and an amino acid sequence (GenPept accession number: AHC54051) of a linalool synthase (CsLINS) gene derived from *Coriandrum sativum* have been already known. The amino acid sequence of a linalool synthase protein and the nucleotide sequence of its gene derived from *Coriandrum sativum* are shown in SEQ ID NO:78 and SEQ ID NO:79, respectively. In order to efficiently express the CsLINS gene, codons were optimized to resolve a secondary structure, a CsLINS gene in which the chloroplast localization signal had been cleaved was designed, and this was designated as opt_CsLINS. A nucleotide sequence of opt_CsLINS is shown in SEQ ID NO:80. DNA in which the tac promoter region (deBoer, et al., (1983) Proc. Natl. Acad. Sci. USA., 80, 21-25) had been added to the opt_CsLINS gene was chemically synthesized, cloned into pUC57 (supplied from GenScript), and the resulting plasmid was designated as pUC57-Ptac-opt_CsLINS.

### 12-3) Acquisition of mutated farnesyl diphosphate synthase gene derived from Escherichia coli

Farnesyl diphosphate synthase derived from *Escherichia coli* is encoded by an ispA gene (SEQ ID NO:81) (Fujisaki S., et al. (1990) J. Biochem. (Tokyo) 108:995-1000*).* A mutation which increases a concentration of geranyl diphosphate in microbial cells has been demonstrated in farnesyl diphosphate synthase derived from *Bacillus stearothermophilus* (Narita K., et al. (1999) J Biochem 126(3):566-571*).* Based on this finding, the similar mutant has been also produced in farnesyl diphosphate synthase derived from *Escherichia coli* (Reiling KK et al. (2004) Biotechnol Bioeng. 87(2) 200-212*).* In order to efficiently express an ispA mutant (S80F) gene having a high activity for producing geranyl diphosphate, a sequence in which the codons were optimized to resolve the secondary structure was designed and designated as ispA^{*}. A nucleotide sequence of ispA^{*} is shown in SEQ ID NO:82. The ispA^{*} gene was chemically synthesized, subsequently cloned into pUC57 (supplied from GenScript) and the resulting plasmid was designated as pUC57-ispA*.

### 12-4) Construction of co-expression plasmid for opt_AaLINS and ispA* genes

PCR with pUC57-Ptac-opt_AaLINS as a template was carried out using primers shown in SEQ ID NO:83 and SEQ ID NO:85 to obtain a Ptac-opt_AaLINS fragment. Further, PCR with pUC57-ispA* as a template was carried out using primers shown in SEQ ID NO:86 and SEQ ID NO:87 to obtain an ispA* fragment. The purified Ptac-opt_AaLINS fragment and ispA* fragment were ligated to pACYC177 (supplied from Nippon Gene) digested with restriction enzymes PstI and ScaI using In-Fusion HD cloning kit (supplied from Clontech) to construct pACYC177-Ptac-opt_AaLINS-ispA*.

### 12-5) Construction of co-expression plasmid for opt_CsLINS and ispA* genes

PCR with pUC57-Ptac-opt_CsLINS as a template was carried out using primers shown in SEQ ID NO:83 and SEQ ID NO:88 to obtain a Ptac-opt_CsLINS fragment. Further, PCR with pUC57-ispA* as a template was carried out using primers shown in SEQ ID NO:89 and SEQ ID NO:87 to obtain an ispA* fragment. The purified Ptac-opt_CsLINS fragment and ispA* fragment were ligated to pACYC177 (supplied from Nippon Gene) digested with restriction enzymes PstI and ScaI using In-Fusion HD cloning kit (supplied from Clontech) to construct pACYC177-Ptac-opt_CsLINS-ispA*.

### 12-6) Preparation of linalool production strains

Competent cells of SWITCH-PphoCAgcd were prepared, and pACYC177, pACYC177-Ptac-opt_AaLINS-ispA* or pACYC177-Ptac-opt_CsLINS-ispA* was introduced thereto by electroporation. Resulting strains were designated as SWITCH-PphoCΔgcd/pACYC177, SWITCH-PphoCΔgcd/AaLINS-ispA* and SWITCH-PphoCΔgcd/CsLINS-ispA*.

Example 13: Evaluation of ability to produce linalool by linalool synthase-expressing strains derived from SWITCH-PphoCΔgcd strain

Microbial cells of SWITCH-PphoCΔgcd/AaLINS-ispA*, SWITCH-PphoCAgcd/CsLINS-ispA* and SWITCH-PphoCΔgcd/pACYC177 strains obtained in Example 12 in glycerol stock were thawed. Subsequently 50 µL of a microbial cell suspension from each strain was evenly applied onto an LB plate containing 50 mg/L of kanamycin, and cultured at 34□C for 16 hours. The resulting microbial cells on the plate were picked up in an amount corresponding to about 1/4 of a loop part of a 10 µL inoculating loop (supplied from Thermo Fisher Scientific Inc.). The picked up microbial cells were inoculated to 5 mL of fermentation medium described below containing 50 mg/L of kanamycin in a test tube supplied from AGC Techno Glass Co., Ltd. (diameter x length x thickness=25x200x1.2 mm), and cultured at 30°C on a reciprocal shaking culture apparatus at 120 rpm for 24 hours.

**Table 8. Fermentation medium for SWITCH-PphoCAgcd, host strain for production of linalool**

| Group A | |
|---|---|
| D-Glucose | 40 g/L |
| MgSO₄·7H₂O | 1 g/L |
| Not adjusted pH, AC 115°C, 10 minutes | |

| Group B | |
|---|---|
| (NH₄)₂SO₄ | 20 g/L |
| KH₂PO₄ | 0.5 g/L |
| Yeast extract | 2 g/L |
| FeSO₄·7H₂O | 0.01 g/L |
| MnSO₄·5H₂O | 0.01 g/L |
| After adjusting pH to 7.0 with KOH, AC 115°C, 10 minutes | |

| Group C | |
|---|---|
| CaCO₃ | 20 g/L |
| Dry-heat sterilization | 180°C, 2 hours |

After the sterilization, the above Groups A, B and C were mixed. Then, 1 mL of isopropyl myristate (Tokyo Chemical Industry Co., Ltd.) was added to 5 mL of the fermentation medium dispensed in the test tube.

Twenty-four hours after starting the cultivation, the concentrations of isopropyl myristate and linalool in the culture supernatant were measured under the following condition using gas chromatography GC-2025AF (supplied from Shimadzu Corporation). DB-5 (supplied from Agilent, length 30 m, internal diameter 0.25 mm, thickness 0.25 µm) was used as a column, and a linalool standard solution was prepared using a reagent Linalool (supplied from Wako Pure Chemical Industries Ltd.).

| | |
|---|---|
| Temperature in vaporization room | 360.0°C |
| Injection amount | 1.0 µL |
| Injection mode | Split 1:10 |
| Carrier gas | He |
| Control mode | Line velocity |
| Pressure | 125.5 kPa |
| Total flow | 20.5 mL/minute |
| Column flow | 1.59 mL/minute |
| Line velocity | 36.3 cm/sec |
| Purge flow | 3.0 mL/minute |

Column open temperature program Total time 21.5 minutes

| Rate (°C/minute) | Temperature (°C) | Hold time (min) |
|---|---|---|
| | 65.0 | 5.0 |
| 5.0 | 105.0 | 0.5 |
| 35.0 | 297.5 | 2.5 |

| | |
|---|---|
| Detector temperature | 375.0°C |
| Detector | FID |
| Make-up gas | He (30.0 mL/min) |
| Hydrogen flow | 40.0 mL/min |
| Air | 400.0 mL/min |

The concentration of linalool is shown as a value in terms of medium amount. A mean value of results obtained from two test tubes is shown in Table 9. No linalool production was observed in the control strain having the introduced control vector pACYC177, whereas the linalool production was confirmed in SWITCH-PphoCΔgcd/AaLINS-ispA* and SWITCH-PphoCAgcd/CsLINS-ispA* strains.

**Table 9. Accumulation of linalool when linalool synthase derived from Actinidia arguta and linalool synthase derived from Coriandrum sativum were introduced**

| Strain | O.D. (620nm) | Linalool (mg/L) |
|---|---|---|
| SWITCH-PphoC Δgcd/pACYC177 | 15.9 | 0.0 |
| SWITCH-PphoC Δgcd/CsLINS-ispA* | 20.2 | 2.6 |
| SWITCH-PphoC Δgcd/AaLINS-ispA* | 12.0 | 1328.2 |

### Example 14

### 14-1) Acquisition of limonene synthase gene derived from Picea sitchensis (Sitka spruce)

A nucleotide sequence (GenBank accession number: DQ195275.1) and an amino acid sequence (GenPept accession number: ABA86248.1.) of a limonene synthase (PsLMS) gene derived from *Picea sitchensis* have been already known. The amino acid sequence of a limonene synthase protein and the nucleotide sequence of its gene derived from *P. sitchensis* are shown in SEQ ID NO:90 and SEQ ID NO:91, respectively. In order to efficiently express the PsLMS gene, its secondary structure was resolved and codons were optimized so that the codon usage was same as it in *P. ananatis.* Moreover, its chloroplast localization signal had been cleaved. An obtained gene was designated as opt_PsLMS. A nucleotide sequence of opt_PsLMS is shown in SEQ ID NO:92. After opt_PsLMS gene was chemically synthesized, cloned into pUC57 (supplied from GenScript) and the resulting plasmid was designated as pUC57-opt_PsLMS.

### 14-2) Acquisition of limonene synthase gene derived from Abies grandis (Grand fir)

A nucleotide sequence (GenBank accession number: AF006193.1) and an amino acid sequence (GenPept accession number: AAB70907.1.) of a limonene synthase (AgLMS) gene derived from *Abies grandis* have been already known. The amino acid sequence of a limonene synthase protein and the nucleotide sequence of its gene derived from *A. grandis* are shown in SEQ ID NO:93 and SEQ ID NO:94, respectively. In order to efficiently express the AgLMS gene, its secondary structure was resolved and codons were optimized so that the codon usage was same as it in *P. ananatis.* Moreover, its chloroplast localization signal had been cleaved. An obtained gene was designated as opt_AgLMS. A nucleotide sequence of opt_AgLMS is shown in SEQ ID NO:95. After opt_AgLMS gene was chemically synthesized, cloned into pUC57 (supplied from GenScript) and the resulting plasmid was designated as pUC57-opt_AgLMS.

### 14-3) Acquisition of limonene synthase gene derived from Mentha spicata (spearmint)

A nucleotide sequence (GenBank accession number: L13459.1) and an amino acid sequence (GenPept accession number: AAC37366.1.) of a limonene synthase (MsLMS) gene derived from *Mentha spicata* have been already known. The amino acid sequence of a limonene synthase protein and the nucleotide sequence of its gene derived from *M. spicata* are shown in SEQ ID NO:96 and SEQ ID NO:97, respectively. In order to efficiently express the MsLMS gene, its secondary structure was resolved and codons were optimized so that the codon usage was same as it in *P. ananatis.* Moreover, its chloroplast localization signal had been cleaved. An obtained gene was designated as opt_MsLMS. A nucleotide sequence of opt_MsLMS is shown in SEQ ID NO:98. After opt_MsLMS gene was chemically synthesized, cloned into pUC57 (supplied from GenScript) and the resulting plasmid was designated as pUC57-opt_MsLMS.

### 14-4) Acquisition of limonene synthase gene derived from Citrus unshiu (Unshu mikan)

A nucleotide sequence (GenBank accession number: AB110637.1) and an amino acid sequence (GenPept accession number: BAD27257.1) of a limonene synthase (CuLMS) gene derived from *Citrus unshiu* have been already known. The amino acid sequence of a limonene synthase protein and the nucleotide sequence of its gene derived from *C. unshiu* are shown in SEQ ID NO:99 and SEQ ID NO:100, respectively. In order to efficiently express the CuLMS gene, its secondary structure was resolved and codons were optimized so that the codon usage was same as it in *P. ananatis.* Moreover, its chloroplast localization signal had been cleaved. An obtained gene was designated as opt_CuLMS. A nucleotide sequence of opt_CuLMS is shown in SEQ ID NO:101. After opt_CuLMS gene was chemically synthesized, cloned into pUC57 (supplied from GenScript) and the resulting plasmid was designated as pUC57-opt_CuLMS.

### 14-5) Acquisition of limonene synthase gene derived from Citrus limon (lemon)

A nucleotide sequence (GenBank accession number: AF514287.1) and an amino acid sequence (GenPept accession number: AAM53944.1) of a limonene synthase (ClLMS) gene derived from *Citrus limon* have been already known. The amino acid sequence of a limonene synthase protein and the nucleotide sequence of its gene derived from *C. limon* are shown in SEQ ID NO:102 and SEQ ID NO:103, respectively. In order to efficiently express the ClLMS gene, its secondary structure was resolved and codons were optimized so that the codon usage was same as it in *P. ananatis.* Moreover, its chloroplast localization signal had been cleaved. An obtained gene was designated as opt_ClLMS. A nucleotide sequence of opt_ClLMS is shown in SEQ ID NO:104. After opt_ClLMS gene was chemically synthesized, cloned into pUC57 (supplied from GenScript) and the resulting plasmid was designated as pUC57-opt_ClLMS.

### 14-6) Construction of co-expression plasmid for opt_PsLMS and ispA* genes

PCR with pUC57-opt_PsLMS as a template was carried out using primers shown in SEQ ID NO:105 and SEQ ID NO:106. Then, PCR with obtained PCR fragment as a template was carried out using primers shown in SEQ ID NO:107 and SEQ ID NO:106 to obtain a Ptac-opt_PsLMS fragment. Further, PCR with pUC57-ispA* as a template was carried out using primers shown in SEQ ID NO:108 and SEQ ID NO:109 to obtain an ispA* fragment. The purified Ptac-opt_PsLMS fragment and ispA* fragment were ligated to pACYC177 (supplied from Nippon Gene) digested with restriction enzymes PstI and ScaI using In-Fusion HD cloning kit (supplied from Clontech) to construct pACYC177-Ptac-opt_PsLMS-ispA*.

### 14-7) Construction of co-expression plasmid for opt_AgLMS and ispA* genes

PCR with pUC57-opt_AgLMS as a template was carried out using primers shown in SEQ ID NO:110 and SEQ ID NO:111. Then, PCR with obtained PCR fragment as a template was carried out using primers shown in SEQ ID NO:107 and SEQ ID NO:111 to obtain a Ptac-opt_AgLMS fragment. Further, PCR with pUC57-ispA* as a template was carried out using primers shown in SEQ ID NO:108 and SEQ ID NO:109 to obtain an ispA* fragment. The purified Ptac-opt_AgLMS fragment and ispA* fragment were ligated to pACYC177 (supplied from Nippon Gene) digested with restriction enzymes PstI and ScaI using In-Fusion HD cloning kit (supplied from Clontech) to construct pACYC177-Ptac-opt_AgLMS-ispA*.

### 14-8) Construction of co-expression plasmid for opt_MsLMS and ispA* genes

PCR with pUC57-opt_MsLMS as a template was carried out using primers shown in SEQ ID NO:112 and SEQ ID NO:113. Then, PCR with obtained PCR fragment as a template was carried out using primers shown in SEQ ID NO:107 and SEQ ID NO:113 to obtain a Ptac-opt_MsLMS fragment. Further, PCR with pUC57-ispA* as a template was carried out using primers shown in SEQ ID NO:108 and SEQ ID NO:109 to obtain an ispA* fragment. The purified Ptac-opt_MsLMS fragment and ispA* fragment were ligated to pACYC177 (supplied from Nippon Gene) digested with restriction enzymes PstI and ScaI using In-Fusion HD cloning kit (supplied from Clontech) to construct pACYC177-Ptac-opt_MsLMS-ispA*.

### 14-9) Construction of co-expression plasmid for opt_CuLMS and ispA* genes

PCR with pUC57-opt_CuLMS as a template was carried out using primers shown in SEQ ID NO:114 and SEQ ID NO:115. Then, PCR with obtained PCR fragment as a template was carried out using primers shown in SEQ ID NO:107 and SEQ ID NO:115 to obtain a Ptac-opt_CuLMS fragment. Further, PCR with pUC57-ispA* as a template was carried out using primers shown in SEQ ID NO:108 and SEQ ID NO:109 to obtain an ispA* fragment. The purified Ptac-opt_CuLMS fragment and ispA* fragment were ligated to pACYC177 (supplied from Nippon Gene) digested with restriction enzymes PstI and ScaI using In-Fusion HD cloning kit (supplied from Clontech) to construct pACYC177-Ptac-opt_CuLMS-ispA*.

### 14-10) Construction of co-expression plasmid for opt_ClLMS and ispA* genes

PCR with pUC57-opt_ClLMS as a template was carried out using primers shown in SEQ ID NO:116 and SEQ ID NO:117. Then, PCR with obtained PCR fragment as a template was carried out using primers shown in SEQ ID NO:107 and SEQ ID NO:117 to obtain a Ptac-opt_ClLMS fragment. Further, PCR with pUC57-ispA* as a template was carried out using primers shown in SEQ ID NO:108 and SEQ ID NO:109 to obtain an ispA* fragment. The purified Ptac-opt_ClLMS fragment and ispA* fragment were ligated to pACYC177 (supplied from Nippon Gene) digested with restriction enzymes PstI and ScaI using In-Fusion HD cloning kit (supplied from Clontech) to construct pACYC177-Ptac-opt_ClLMS-ispA*.

### 14-11) Preparation of limonene production strains

Competent cells of SWITCH-PphoCAgcd were prepared, and pACYC177, pACYC177-Ptac-opt_PsLMS-ispA/*, pACYC177-Ptac-opt_AgLMS-ispA*, pACYC177-Ptac-opt_MsLMS-ispA*, pACYC177-Ptac-opt_CuLMS-ispA* or pACYC177-Ptac-opt_ClLMS-ispA* was introduced thereto by electroporation. Resulting strains were designated as SWITCH-PphoCΔgcd/pACYC177, SWITCH-PphoCΔgcd/PsLMS-ispA*, SWITCH-PphoCΔgcd/AgLMS-ispA*, SWITCH-PphoCΔgcd/MsLMS-ispA*, SWITCH-PphoCΔgcd/CuLMS-ispA* and SWITCH-PphoCΔgcd/ClLMS-ispA*.

Example 15: Evaluation of ability to produce limonene by limonene synthase-expressing strains derived from SWITCH-PphoCΔgcd strain

Microbial cells of SWITCH-PphoCΔgcd/PsLMS-ispA*, SWITCH-PphoCΔgcd/AgLMS-ispA*, SWITCH-PphoCΔgcd/MsLMS-ispA*, SWITCH-PphoCΔgcd/CuLMS-ispA*, SWITCH-PphoCAgcd/ClLMS-ispA* and SWITCH-PphoCΔgcd/pACYC177 strains obtained in Example 14 in glycerol stock were thawed. Subsequently 10 µL of a microbial cell suspension from each strain was evenly applied onto an LB plate containing 50 mg/L of kanamycin, and cultured at 34°C for 16 hours. The resulting microbial cells on the plate were picked up in an amount corresponding to about 1 of a loop part of a Nunc™ disposable 1 µL inoculating loop (supplied from Thermo Fisher Scientific Inc.). The picked up microbial cells were inoculated to 1 mL of limonene fermentation medium described below Table 10 containing 50 mg/L of kanamycin in a head space vial (manufactured by Perkin Elmer, 22ml CLEAR CRIMP TOP VIAL cat #B0104236), and the vial was tightly sealed with a cap with a butyl rubber septum for the headspace vial (CRIMPS (Cat #B0104240) manufactured by Perkin Elmer). SWITCH-PphoCΔgcd/pACYC177, SWITCH-PphoCΔgcd/PsLMS-ispA*, SWITCH-PphoCΔgcd/AgLMS-ispA* and SWITCH-PphoCΔgcd/MsLMS-ispA* strains were cultured at 30°C on a reciprocal shaking culture apparatus at 120 rpm for 48 hours. SWITCH-PphoCΔgcd/pACYC177, SWITCH-PphoCAgcd/CuLMS-ispA* and SWITCH-PphoCΔgcd/ClLMS-ispA* strains were fermented with same manner, cultivation time of these strains were 72 hours.

**Table 10. Fermentation medium for limonene production (final concentration)**

| Group A | |
|---|---|
| D-Glucose | 4 g/L |
| MgSO₄·7H₂O | 1 g/L |
| Not adjusted pH, AC 115°C 10 minutes | |
| | |

| Group B | |
|---|---|
| (NH₄)₂SO₄ | 10 g/L |
| Yeast extract | 50 mg/L |
| FeSO₄·7H₂O | 5 mg/L |
| MnSO₄ ·5H₂O | 5 mg/L |
| Not adjusted pH, AC 115°C, , 10 minutes | |
| | |

| Group C | |
|---|---|
| MES | 20 mM |

After adjusting pH to 7.0 with NaOH, sterilized by filtration
After the sterilization, the above Groups A, B and C were mixed.

After completion of cultivation, limonene concentration in the headspace vial was measured by the gas chromatograph mass spectrometer (GCMS-QP2010 manufactured by Shimadzu Corporation) with head space sampler (TurboMatrix 40 manufactured by Perkin Elmer). Detailed analytical condition was shown in below. For GC column, HP-5ms Ultra Inert (Agilent) was used and limonene standard liquid was prepared with limonene agent (Tokyo Kasei Kogyo).

### Headspace sampler

| | |
|---|---|
| Injection time: | 0.02 minute |
| Oven temperature: | 80°C |
| Needle temperature: | 80°C |
| Transfer temperature: | 80°C |
| GC cycle time: | 5 minutes |
| Pressurizationtime: | 3.0 minutes |
| Pull-up time: | 0.2minutes |
| Heat retentiontime: | 5 minutes |
| Carrier gas pressure (high purity helium): | 124 kPa |

### Gas chromatography part

Carrier gas: He

| | |
|---|---|
| Temperature in vaporization room | 200.0°C |
| Temperature condition | 175°C (constant temperature) |

### Mass spectrometry part

| | |
|---|---|
| Temperature in ion source | 250°C |
| Temperature in interface | 250°C |
| Electric voltage for detector | 0.1 kV |
| Detection ion molecular weight | 68 |
| Auxiliary ion molecular weight | 93 |
| Filament lighting time | 2.0 - 3.5 min |

The concentration of limonene is shown as a value in terms of medium amount. A mean value of results obtained from two vials is shown in Tables 11 and 12. No limonene production was observed in the control strain having the introduced control vector pACYC177, whereas the limonene production was confirmed in SWITCH-PphoCΔgcd/PsLMS-ispA*, SWITCH-PphoCΔgcd/AgLMS-ispA*, SWITCH-PphoCΔgcd/MsLMS-ispA*, SWITCH-PphoCAgcd/CuLMS-ispA*, and SWITCH-PphoCAgcd/ClLMS-ispA* strains.

**Table 11. Accumulation of limonene when limonene synthase derived from Picea sitchensis, Abies grandis and Mentha spicata were introduced**

| Strain | O.D. (600nm) | Limonene (mg/L) |
|---|---|---|
| SWITCH-PphoC Δgcd/pACYC177 | 2.6 | 0.0 |
| SWITCH-PphoC Δgcd/PsLMS-ispA* | 1.6 | 0.3 |
| SWITCH-PphoC Δgcd/AgLMS-ispA* | 1.6 | 121 |
| SWITCH-PphoC Δgcd/MsLMS-ispA* | 1.6 | 117 |

**Table 12. Accumulation of limonene when limonene synthase derived from Citrus unshiu and Citrus limon were introduced**

| Strain | O.D. (600nm) | Limonene (mg/L) |
|---|---|---|
| SWITCH-PphoC Δgcd/pACYC177 | 2.2 | 0.0 |
| SWITCH-PphoC Δgcd/CuLMS-ispA* | 1.5 | 33 |
| SWITCH-PphoC Δgcd/ClLMS-ispA* | 1.1 | 129 |

While the invention has been described in detail with reference to preferred embodiments thereof, it will be apparent to the person skilled in the art that various changes can be made, without departing from the scope of the invention as described in the claims

### INDUSTRIAL APPLICABILITY

The method of the present invention is useful for the production of an isoprene monomer and an isoprene polymer.

## Claims

1. A method of producing an isoprenoid compound comprising:
1) culturing an isoprenoid compound-forming microorganism in the presence of a growth promoting agent at a sufficient concentration to grow the isoprenoid compound-forming microorganism;
2) decreasing the sufficient concentration of the growth promoting agent to induce formation of the isoprenoid compound by the isoprenoid compound-forming microorganism; and
3) culturing the isoprenoid compound-forming microorganism to form the isoprenoid compound, wherein the isoprenoid compound-forming microorganism has an ability to form the isoprenoid compound depending on a promoter which is inversely dependent on the growth promoting agent, and wherein the isoprenoid compound-forming microorganism contains a gene encoding an isoprenoid compound-synthetic enzyme, wherein the gene is under the control of the promoter.

2. The method according to claim 1, wherein the growth promoting agent is oxygen.

3. The method according to claim 2, wherein the isoprenoid compound-forming microorganism is an aerobic microorganism.

4. The method according to any one of claims 2 or 3, wherein the promoter is a microaerobically inducible promoter.

5. The method according to claim 4, wherein the microaerobically inducible promoter is a promoter of the gene encoding a lactate dehydrogenase, or a promoter of the gene encoding an α-acetolactate decarboxylase.

6. The method according to any one of claims 1-5, wherein
a) the isoprenoid compound is a monoterpene or
b) the isoprenoid compound is a limonene or
c) the isoprenoid compound is a linanool or
d) the isoprenoid compound is an isoprene monomer.

7. A method of producing an isoprenoid compound, comprising:
1') supplying oxygen into a liquid phase containing the isoprenoid compound-forming microorganism in a system comprising a gas phase and the liquid phase to grow the isoprenoid compound-forming microorganism in the presence of dissolved oxygen at a sufficient concentration;
2') decreasing the dissolved oxygen concentration in the liquid phase to induce formation of the isoprenoid compound by the isoprenoid compound-forming microorganism in the liquid phase;
3') culturing the isoprenoid compound-forming microorganism in the liquid phase to form the isoprenoid compound; and
4') collecting the isoprenoid compound, wherein the isoprenoid compound-forming microorganism has an ability to form the isoprenoid compound depending on a promoter which is inversely dependent on the dissolved oxygen concentration, and wherein the isoprenoid compound-forming microorganism contains a gene encoding an isoprenoid compound-synthetic enzyme, wherein the gene is under the control of the promoter.

8. The method according to claim 7, wherein an oxygen concentration in the gas phase is controlled depending on the dissolved oxygen concentration in the liquid phase, and wherein the dissolved oxygen concentration in the liquid phase is decreased to avoid isoprene burst in the gas phase.

9. The method according to claim 7 or 8, wherein the dissolved oxygen concentration in the liquid phase is 0.34 ppm or less in 2') and 3').

10. The method according to claim 1, wherein the growth promoting agent is a phosphorus compound.

11. The method according to claim 10, wherein the promoter is a phosphorus deficiency-inducible promoter.

12. The method according to claim 11, wherein the phosphorus deficiency-inducible promoter is a promoter of the gene encoding an acid phosphatase, or a promoter of the gene encoding a phosphorus uptake carrier.

13. The method according to claim 11 or 12, wherein formation of the isoprenoid by the isoprenoid-forming microorganism is induced in the presence of a total phosphorus at concentration of 50 mg/L or less.

14. The method according to any one of claims 7 to 13, wherein the isoprenoid-forming rate in the induction phase is 600 ppm/vvm/h or less, preferably 80 ppm/vvm/h or less.

15. The method according to any one of claim 7 to 14, wherein the isoprenoid compound is an isoprene.

16. The method according to any one of claims 1 to 15, wherein the isoprenoid compound-forming microorganism has an ability to synthesize dimethylallyl diphosphate via a methylerythritol phosphate pathway.

17. The method according to any one of claims 1 to 16, wherein the isoprenoid compound-forming microorganism has the ability to synthesize dimethylallyl diphosphate via a mevalonate pathway.

18. The method according to any one of claims 1 to 17, wherein the isoprenoid compound-forming microorganism is a microorganism belonging to the family *Enterobacteriaceae.*

19. The method according to claim 18, wherein the isoprenoid compound-forming microorganism is a microorganism belonging to the genus *Pantoea, Enterobacter, or Escherichia.*

20. The method according to claim 19, wherein the isoprenoid compound-forming microorganism is *Pantoea ananatis, Enterobacter aerogenes or Escherichia coli.*

21. A method of producing an isoprene polymer comprising:
(I) forming the isoprene monomer by the method according to any one of claims 6 d) or 15; and
(II) polymerizing the isoprene monomer to form the isoprene polymer.

22. A method for producing a rubber composition, comprising:
(A) preparing an isoprene polymer by the method of claim 21; and
(B) mixing said isoprene polymer with one or more rubber composition components.

23. A method for producing a tire, comprising:
(i) producing a rubber composition by the method of claim 22; and
(ii) applying said rubber composition to manufacture a tire.

## Patentansprüche

1. Verfahren zum Herstellen einer Isoprenoidverbindung, umfassend:
1) Kultivieren eines eine Isoprenoidverbindung bildenden Mikroorganismus in Anwesenheit eines wachstumsfördernden Mittels in einer ausreichenden Konzentration, um den die Isoprenoidverbindung bildenden Mikroorganismus wachsen zu lassen,
2) Verringern der ausreichenden Konzentration des wachstumsfördernden Mittels, um die Bildung der Isoprenoidverbindung durch den eine Isoprenoidverbindung bildenden Mikroorganismus zu induzieren, und
3) Kultivieren des eine Isoprenoidverbindung bildenden Mikroorganismus zum Bilden der Isoprenoidverbindung, wobei der eine Isoprenoidverbindung bildende Mikroorganismus die Fähigkeit hat, die Isoprenoidverbindung in Abhängigkeit von einem Promotor zu bilden, welcher umgekehrt abhängig von dem wachstumsfördernden Mittel ist, und wobei der eine Isoprenoidverbindung bildende Mikroorganismus ein Gen enthält, das für ein Isoprenoidverbindungs-Syntheseenzym codiert, wobei das Gen unter der Kontrolle des Promotors steht.

2. Verfahren gemäß Anspruch 1, wobei das wachstumsfördernde Mittel Sauerstoff ist.

3. Verfahren gemäß Anspruch 2, wobei der eine Isoprenoidverbindung bildende Mikroorganismus ein aerober Mikroorganismus ist.

4. Verfahren gemäß einem der Ansprüche 2 oder 3, wobei der Promotor ein mikroaerob induzierbarer Promotor ist.

5. Verfahren gemäß Anspruch 4, wobei der mikroaerob induzierbare Promotor ein Promotor des Gens, das für eine Laktatdehydrogenase codiert, oder ein Promotor des Gens, das für eine α-Acetolaktatdecarboxylase codiert, ist.

6. Verfahren nach einem der Ansprüche 1-5, wobei
(a) die Isoprenoidverbindung ein Monoterpen ist oder
(b) die Isoprenoidverbindung ein Limonen ist oder
(c) die Isoprenoidverbindung ein Linanool ist oder
(d) die Isoprenoidverbindung ein Isoprenmonomer ist.

7. Verfahren zum Herstellen einer Isoprenoidverbindung, umfassend:
1') Zuführen von Sauerstoff in eine flüssige Phase, die den eine Isoprenoidverbindung bildenden Mikroorganismus in einem System enthält, welches eine Gasphase und die flüssige Phase umfasst, um den eine Isoprenoidverbindung bildenden Mikroorganismus in Anwesenheit von gelöstem Sauerstoff in einer ausreichenden Konzentration wachsen zu lassen,
2') Verringern der Konzentration von gelöstem Sauerstoff in der flüssigen Phase, um die Bildung der Isoprenoidverbindung durch den eine Isoprenoidverbindung bildenden Mikroorganismus in der flüssigen Phase zu induzieren,
3') Kultivieren des eine Isoprenoidverbindung bildenden Mikroorganismus in der flüssigen Phase, um die Isoprenoidverbindung zu bilden, und
4') Sammeln der Isoprenoidverbindung, wobei der eine Isoprenoidverbindung bildende Mikroorganismus die Fähigkeit hat, die Isoprenoidverbindung in Abhängigkeit von einem Promotor zu bilden, der umgekehrt abhängig von der Konzentration von gelöstem Sauerstoff ist, und wobei der eine Isoprenoidverbindung bildende Mikroorganismus ein Gen enthält, das für ein Isoprenoidverbindungs-Syntheseenzym codiert, wobei das Gen unter der Kontrolle des Promotors steht.

8. Verfahren gemäß Anspruch 7, wobei eine Sauerstoffkonzentration in der Gasphase abhängig von der Konzentration von gelöstem Sauerstoff in der flüssigen Phase gesteuert wird und wobei die Konzentration von gelöstem Sauerstoff in der flüssigen Phase verringert wird, um einen Ausbruch des Isoprens in der Gasphase zu vermeiden.

9. Verfahren gemäß Anspruch 7 oder 8, wobei die Konzentration von gelöstem Sauerstoff in der Flüssigphase 0,34 ppm oder weniger in 2') und 3') beträgt.

10. Verfahren gemäß Anspruch 1, wobei das wachstumsfördernde Mittel eine Phosphorverbindung ist.

11. Verfahren gemäß Anspruch 10, wobei der Promotor ein durch Phosphormangel induzierbarer Promotor ist.

12. Verfahren gemäß Anspruch 11, wobei der durch Phosphormangel induzierbare Promotor ein Promotor des Gens, das für eine saure Phosphatase codiert, oder ein Promotor des Gens, das für einen Phosphor-Aufnahmeträger codiert, ist.

13. Verfahren gemäß Anspruch 11 oder 12, wobei die Bildung des Isoprenoids durch den isoprenoidbildenden Mikroorganismus in Anwesenheit von Gesamtphosphor in einer Konzentration von 50 mg/L oder weniger induziert wird.

14. Verfahren gemäß einem der Ansprüche 7 bis 13, wobei die Isoprenoid-Bildungsrate in der Induktionsphase 600 ppm/vvm/h oder weniger, vorzugsweise 80 ppm/vvm/h oder weniger, beträgt.

15. Verfahren gemäß einem der Ansprüche 7 bis 14, wobei die Isoprenoidverbindung ein Isopren ist.

16. Verfahren gemäß einem der Ansprüche 1 bis 15, wobei der eine Isoprenoidverbindung bildende Mikroorganismus die Fähigkeit besitzt, Dimethylallyldiphosphat über einen Methylerythritolphosphatweg zu synthetisieren.

17. Verfahren gemäß einem der Ansprüche 1 bis 16, wobei der eine Isoprenoidverbindung bildende Mikroorganismus die Fähigkeit besitzt, Dimethylallyldiphosphat über einen Mevalonatweg zu synthetisieren.

18. Verfahren gemäß einem der Ansprüche 1 bis 17, wobei der eine Isoprenoidverbindung bildende Mikroorganismus ein Mikroorganismus ist, der zur Familie der *Enterobacteriaceae* gehört.

19. Verfahren gemäß Anspruch 18, wobei der eine Isoprenoidverbindung bildende Mikroorganismus ein Mikroorganismus, der zur Gattung *Pantoea, Enterobacter* oder *Escherichia* gehört, ist.

20. Verfahren gemäß Anspruch 19, wobei der eine Isoprenoidverbindung bildende Mikroorganismus *Pantoea ananatis, Enterobacter aerogenes* oder *Escherichia coli* ist.

21. Verfahren zum Herstellen eines Isoprenpolymers, umfassend:
(I) Bilden des Isoprenmonomers durch das Verfahren gemäß einem der Ansprüche 6 d) oder 15 und
(II) Polymerisieren des Isoprenmonomers, um das Isoprenpolymer zu bilden.

22. Verfahren zum Herstellen einer Gummizusammensetzung, umfassend:
(A) Herstellen eines Isoprenpolymers durch das Verfahren nach Anspruch 21 und
(B) Mischen des Isoprenpolymers mit einer oder mehreren Komponenten der Gummizusammensetzung.

23. Verfahren zum Herstellen eines Reifens, umfassend:
(i) Herstellen einer Gummimischung durch das Verfahren nach Anspruch 22 und
(ii) Aufbringen der Kautschukmischung, um einen Reifen herzustellen.

## Revendications

1. Procédé de production d'un composé isoprénoïde comprenant :
1) la culture d'un microorganisme formateur de composé isoprénoïde en présence d'un agent promoteur de croissance à une concentration suffisante pour faire croître le microorganisme formateur de composé isoprénoïde ;
2) la diminution de la concentration suffisante de l'agent promoteur de croissance pour induire la formation du composé isoprénoïde par le microorganisme formateur de composé isoprénoïde ; et
3) la culture du microorganisme formateur de composé isoprénoïde pour former le composé isoprénoïde, le microorganisme formateur de composé isoprénoïde ayant la capacité de former le composé isoprénoïde en fonction d'un promoteur qui est inversement dépendant de l'agent promoteur de croissance, et le microorganisme formateur de composé isoprénoïde contenant un gène codant pour une enzyme de synthèse du composé isoprénoïde, le gène étant sous le contrôle du promoteur.

2. Procédé selon la revendication 1, dans lequel l'agent de croissance est l'oxygène.

3. Procédé selon la revendication 2, dans lequel le microorganisme formateur de composé isoprénoïde est un microorganisme aérobie.

4. Procédé selon l'une quelconque des revendications 2 ou 3, dans lequel le promoteur est un promoteur inductible par voie microaérobie.

5. Procédé selon la revendication 4, dans lequel le promoteur inductible par voie microaérobie est un promoteur du gène codant pour une lactate déshydrogénase, ou un promoteur du gène codant pour une α-acétolactate décarboxylase.

6. Procédé selon l'une quelconque des revendications 1 à 5, dans lequel
a) le composé isoprénoïde est un monoterpène ou
b) le composé isoprénoïde est un limonène ou
c) le composé isoprénoïde est un linanool ou
d) le composé isoprénoïde est un monomère d'isoprène.

7. Procédé de production d'un composé isoprénoïde, comprenant :
1') l'apport d'oxygène dans une phase liquide contenant le microorganisme formateur de composé isoprénoïde dans un système comprenant une phase gazeuse et la phase liquide pour faire croître le microorganisme formateur de composé isoprénoïde en présence d'oxygène dissous à une concentration suffisante ;
2') la diminution de la concentration en oxygène dissous dans la phase liquide pour induire la formation du composé isoprénoïde par le microorganisme formateur de composé isoprénoïde dans la phase liquide ;
3') la culture du microorganisme formateur de composé isoprénoïde en phase liquide pour former le composé isoprénoïde ; et
4') la collecte du composé isoprénoïde, le microorganisme formateur de composé isoprénoïde ayant la capacité de former le composé isoprénoïde en fonction d'un promoteur qui dépend inversement de la concentration en oxygène dissous, et le microorganisme formateur de composé isoprénoïde contenant un gène codant pour une enzyme de synthèse du composé isoprénoïde, le gène étant sous le contrôle du promoteur.

8. Procédé selon la revendication 7, dans lequel une concentration en oxygène dans la phase gazeuse est commandée en fonction de la concentration en oxygène dissous dans la phase liquide, et dans lequel la concentration en oxygène dissous dans la phase liquide est diminuée pour éviter l'éclatement de l'isoprène dans la phase gazeuse.

9. Procédé selon la revendication 7 ou 8, dans lequel la concentration en oxygène dissous dans la phase liquide est de 0,34 ppm ou moins pendant 2') et 3').

10. Procédé selon la revendication 1, dans lequel l'agent promoteur de croissance est un composé phosphoré.

11. Procédé selon la revendication 10, dans lequel le promoteur est un promoteur inductible par une carence en phosphore.

12. Procédé selon la revendication 11, dans lequel le promoteur inductible par une carence en phosphore est un promoteur du gène codant pour une acide phosphatase, ou un promoteur du gène codant pour un support d'absorption du phosphore.

13. Procédé selon la revendication 11 ou 12, dans lequel la formation de l'isoprénoïde par le microorganisme formateur d'isoprénoïde est induite en présence d'un phosphore total à une concentration de 50 mg/L ou moins.

14. Procédé selon l'une des revendications 7 à 13, dans lequel la vitesse de formation d'isoprénoïde pendant la phase d'induction est de 600 ppm/vvm/h ou moins, de préférence 80 ppm/vvm/h ou moins.

15. Procédé selon l'une quelconque des revendications 7 à 14, dans lequel le composé isoprénoïde est un isoprène.

16. Procédé selon l'une quelconque des revendications 1 à 15, dans lequel le microorganisme formateur de composé isoprénoïde a la capacité de synthétiser le diphosphate de diméthylallyle par une voie au phosphate de méthylérythritol.

17. Procédé selon l'une quelconque des revendications 1 à 16, dans lequel le microorganisme formateur de composé isoprénoïde a la capacité de synthétiser le diphosphate de diméthylallyle par une voie au mévalonate.

18. Procédé selon l'une quelconque des revendications 1 à 17, dans lequel le microorganisme formateur de composé isoprénoïde est un microorganisme appartenant à la famille *Enterobacteriaceae.*

19. Procédé selon la revendication 18, dans lequel le microorganisme formateur de composé isoprénoïde est un microorganisme appartenant au genre *Pantoea, Enterobacter* ou *Escherichia.*

20. Procédé selon la revendication 19, dans lequel le microorganisme formateur de composé isoprénoïde est *Pantoea ananatis, Enterobacter aerogenes* ou *Escherichia coli.*

21. Procédé de production d'un polymère d'isoprène comprenant :
(I) la formation du monomère d'isoprène par le procédé selon l'une quelconque des revendications 6 d) ou 15 ; et
(II) la polymérisation du monomère d'isoprène pour former le polymère d'isoprène.

22. Procédé de production d'une composition de caoutchouc, comprenant :
(A) la préparation d'un polymère d'isoprène par le procédé selon la revendication 21 ; et
(B) le mélange dudit polymère d'isoprène avec un ou plusieurs constituants de composition de caoutchouc.

23. Procédé de production d'un pneu, comprenant :
(i) la production d'une composition de caoutchouc par le procédé selon la revendication 22 ; et
(ii) l'emploi de la composition de caoutchouc pour fabriquer un pneu.


### SEQUENCE LISTING

<110> Ajinomoto Co., Inc.
<120> METHOD OF PRODUCING ISOPRENOID COMPOUND
<130> EP112994FZ268pau
<140> EP15863232.3
   <141> 2015-11-27
<150> PCT/JP2015/083498
   <151> 2015-11-27
<150> RU2015141007
   <151> 2015-09-25
<150> RU2014148144
   <151> 2014-11-28
<160> 117
<170> PatentIn version 3.5
<210> 1
   <211> 1783
   <212> DNA
   <213> Enterobacter aerogenes
<400> 1
   ataatgtggt gctgctgcaa accatgcgcg gtttcttcga gcttctgcaa tcctcggtga     60
   agcagagccg ccagcgcatg tatctggtgc cgccggtgtt cgcgcgtctg accgaacaac    120
   atcaggcggt aatggaagcg atcttcgccg gcgacgcgga aggcgcccgc caggcgatga    180
   tggcccacct cggttttgtc cacgccacca ttaaacgttt tgatgaagac caggcccgcc    240
   aggcgcgtat tacccgcctg cccggtgacc acaatgaaaa ttccagggag aactcgtgat    300
   gattatttcc gccgccagcg actatcgcgc cgccgcacag cgtatcctgc caccatttct    360
   gttccactat atcgacggcg gcgcctacgc cgaacatacc ctgcgccgca acgttgaaga    420
   cctctccgac gtggcgctcc gccagcgcat tctgcgcaat atgtcggatc tgagcctgga    480
   gaccacgtta ttcaacgaaa agctggcgat gccaacggcg ctggcgccgg tcgggctgtg    540
   cggcatgtac gcgcgccgcg gcgaagtgca ggcagcgggc gcggcggacg acaaaggcat    600
   cccgtttacc ctctctaccg tttccgtttg cccgattgaa gaggtcgccc cgaccatcaa    660
   acgcccgatg tggttccagc tgtatgtcct gcgcgatcgc ggatttatgc gcaatgcact    720
   ggagcgcgcc aaagccgcag gctgctccac gctggtcttt accgtggata tgccgacccc    780
   cggcgcgcgc tatcgcgacg cccattctgg catgagcggc ccaaacgccg ccatgcgccg    840
   ctactggcag gccgtcactc atccgcagtg ggcatgggac gtcggtctca acggccgccc    900
   gcacgacctg ggcaatatct ccgcctacct cggcaaaccg accgggcttg aggattacat    960
   cggctggctg gcgaataatt tcgatccgtc gatttcatgg aaagacctgg agtggatccg   1020
   cgacttctgg gacggcccaa tggtgatcaa agggatcctc gacccggagg acgcccgcga   1080
   tgcggtgcgc ttcggcgctg acggcattgt cgtctccaac cacggcggcc gccagcttga   1140
   cggcgtgctc tcttccgccc gcgccctgcc cgccattgcc gatgcggtta aaggcgacat   1200
   cactattctc gccgacagcg gtatccgcaa cggtctcgac gtggtgcgga tgatcgccct   1260
   gggcgccgac agcgtgctgc tgggccgcgc gtatctgtac gcgctggcga cccacggcaa   1320
   gcagggggtg gcgaatctgc tcaaccttat cgagaaagag atgaaagtgg cgatgacgct   1380
   gaccggggcg aaatccatca gcgaaatcag ccgcgattcg ctggtgcaga atgccgaagc   1440
   gctgcagacc tttgatgcgc tcaagcaggg taacgccgcg taattccccg cgttttccct   1500
   tacgccgccc ctctccctca gggtgagggg cccgtccggc tggaatcggt tttctccctc   1560
   tccctctggg agagggtcgg ggtgagggtt caacaaatat ctgctatcct gccccccgct   1620
   attaaggggg cagtatgctg aacatcgtgt tattcgaacc agaaatcccg ccgaacaccg   1680
   gcaatatcat ccgcctgtgc gccaacaccg gcttcaattt acatatcatt gagccaatgg   1740
   gctttacctg ggacgataaa cgcctgagac gcgccgggtt gga                      1783
<210> 2
   <211> 89
   <212> DNA
   <213> Artificial Sequence
<220>
   <223> Primer for amplifying lambda attL-TetR- lambda attR-Ptac-KDyI
<400> 2
   ccacgcacgg attacccgcc tgcccggtga gcataatgag cattcgaggg agaaaaacgc 60
   tgaagcctgc ttttttatac taagttggc 89
<210> 3
   <211> 90
   <212> DNA
   <213> Artificial Sequence
<220>
   <223> Primer for amplifying lambda attL-TetR- lambda attR-Ptac-KDyI
<400> 3
   acagcgccga acggtcccct ctccctgagg gagagggtta gggtgagggg gcgcaaacga 60
   ttatagcatt ctatgaattt gcctgtcatt 90
<210> 4
   <211> 26
   <212> DNA
   <213> Artificial Sequence
<220>
   <223> Primer for confirming delta lld:: lambda attL-TetR-lambda attR-Ptac-KDyI
<400> 4
   catgaaacga ttcgatgaag atcagg 26
<210> 5
   <211> 26
   <212> DNA
   <213> Artificial Sequence
<220>
   <223> Primer for confirming delta lld:: lambda attL-TetR-lambda attR-Ptac-KDyI
<400> 5
   ctggttcgta aagtacgatg tttagc 26
<210> 6
   <211> 2319
   <212> DNA
   <213> Enterobacter aerogenes
<400> 6
   gcatgccgct gatgccatcc acgccgtcga ttagcttatg tagaccgtga aacaacatta 60
   agccgccaac ggcaagtcgt aacaagagtt taccggcatc ttcatgcgac agcgttttat 120
   ttactgcgtt taacaatccc ttaaccattt gatgtgcttc ctgttttaca ccctgtgtga 180
   tcccagagta tgcacaatat tccaccaaca aaaccagtga aaaataaggg gcaattggca 240
   acgtaacctg tagtttcatc taagcttgat agcgttatca caaaaaggag atggaaaacc 300
   atgaaacaga ccgtagcgtc atatattgcc aaaacgcttg aacaggccgg cgtgaaacgt 360
   atttggggag tgaccgggga ttccctcaac ggcttgagcg acagtttaaa ccgcatgggg 420
   accatcgaat ggatgccgac ccgccatgaa gaggttgccg cctttgccgc cggtgccgaa 480
   gcccaactca ccggcgagct ggcggtgtgc gccggttcct gcggaccggg caaccttcat 540
   ctgattaacg gtttgtttga ttgccatcgt aaccacgtac cggttctggc tatcgccgcc 600
   catattccgt cgagcgaaat cggcagcggt tatttccagg aaacgcatcc gcaggagctg 660
   ttccgcgaat gtagccacta ctgcgagctg gtctccacac cggagcaaat tccgcaagtt 720
   ctggctatcg ccatgcgtaa ggcggtgatt aaccgcggcg tgtctgtggt ggtcctgccc 780
   ggcgacgtgg cgctgaaacc ggccccggaa agcgccagca gccactggta tcacgctccg 840
   cagcccacgg tcactccggc ggatgaagag ctgcacaagc tggcgcagct gatccgctac 900
   tcgagtaata ttgccctgat gtgcggcagc ggctgcgccg gcgcgcacca ggagctggtg 960
   gagtttgcgg caaaaattaa ggcccctgtc gtccatgccc tgcgcgggaa agagcacgtc 1020
   gaatacgaca atccttacga tgttggcatg accgggctta tcggcttctc ttccggtttc 1080
   cataccatga tgaacgccga cacgctgatc ctgctcggca cccagttccc ctatcgcgcg 1140
   ttctacccta ccgacgccaa aattattcag atcgacatca accccggtag catcggcgcg 1200
   catagtaagg tcgacatggc gctggtgggc gatatcaaat cgacactgaa agccctgctg 1260
   ccgctgctgg aagagaaaac cgatcgtcgc ttcctcgata aagcgctgga ggattatcgc 1320
   gaagcgcgta aagggcttga cgatctcgcc aaacccagcg aaaaagcgct ccatccccag 1380
   tatctggcgc agcagattag ccgctttgcc gacgacgacg cgatctttac ctgcgacgtc 1440
   ggtaccccca ccgtctgggc ggcacgctat ctgcagatga acggcaaacg ccgcctgttg 1500
   ggttcgttta accacggctc gatggccaac gccatgccgc aggctattgg cgccaaagcg 1560
   accgcgccga atcgccaggt catcgccatg tgcggcgatg gcggtttcag catgctgatg 1620
   ggggattttc tatcgctggc gcagatgaag ctgccggtga aaatcgttat ttttaataac 1680
   agcattctcg gctttgtagc gatggagatg aaagccggcg gctatctgac cgacggtacc 1740
   gagttgcacg acaccaattt cgcccgcatt gccgaagcct gcgggatcaa aggaatacgc 1800
   gtcgagaaag cttctgaagt tgatgaggcg ctgcagaccg cctttagcac cgacggtccg 1860
   gtattagtcg atgtagtggt agcgaaggaa gagctggcga tcccgccgca aattaagctg 1920
   gagcaggcca aaggatttag cctgtatatg ctgcgggcga ttatcagcgg acgcggcgat 1980
   gaagtgatcg aactggcgaa aaccaactgg ctcaggtaaa acaatgctat caccctgaat 2040
   cagaacaagg atatgccgtg atagatcttc gcagtgatac cgtaactcgc cccgggcgcg 2100
   ccatgatgga ggccatgatg gccgccccgg tcggggacga tgtgtatggc gacgacccaa 2160
   ccgtcaatga actccagcgc tatgccgctg agctcgcagg caaagaggcg gcgctgtttc 2220
   tgcctaccgg cacccaggcc aatctcgttg gcctgcttag ccactgccag cgcggcgaag 2280
   agtacatcgt cggccagggc gcgcataact atctgtatg 2319
<210> 7
   <211> 90
   <212> DNA
   <213> Artificial Sequence
<220>
   <223> Primer for amplifying PMK gene from E.coli MG1655 Ptac-KDyI strain
<400> 7
   tatcactgcg aagatctatc acggcatatc cttgttctga ttcagggtga tagcattgtt 60
   ttatttatca agataagttt ccggatcttt 90
<210> 8
   <211> 56
   <212> DNA
   <213> Artificial Sequence
<220>
   <223> Primer for amplifying PMK gene from E.coli MG1655 Ptac-KDyI strain
<400> 8
   cggataacaa tttcacacaa ggagactgcc atgtcagagt tgagagcctt cagtgc 56
<210> 9
   <211> 89
   <212> DNA
   <213> Artificial Sequence
<220>
   <223> Primer for amplifying lambda attL-Kmr- lambda attR-Ptac cassette
<400> 9
   acgtaacctg tagtttcatc taagcttgat agcgttatca caaaaaggag atggaaaacc 60
   tgaagcctgc ttttttatac taagttggc 89
<210> 10
   <211> 56
   <212> DNA
   <213> Artificial Sequence
<220>
   <223> Primer for amplifying lambda attL-Kmr- lambda attR-Ptac cassette
<400> 10
   gcactgaagg ctctcaactc tgacatggca gtctccttgt gtgaaattgt tatccg 56
<210> 11
   <211> 22
   <212> DNA
   <213> Artificial Sequence
<220>
   <223> Primer for confirming delta poxB:: lambda attL-Kmr-lambda attR-Ptac-PMK
<400> 11
   gtcgattagc ttatgtagac cg 22
<210> 12
   <211> 21
   <212> DNA
   <213> Artificial Sequence
<220>
   <223> Primer for confirming delta poxB:: lambda attL-Kmr-lambda attR-Ptac-PMK
<400> 12
   tggagttcat tgacggttgg g 21
<210> 13
   <211> 2883
   <212> DNA
   <213> Enterobacter aerogenes
<400> 13
   gccagcggct ttgaacacag catcgctaat atgtttatga tcccgatggg tatcgtaatt     60
   cgcaactttg caagcccgga attctggacc gctatcggtt caactccgga gagtttttct    120
   cacttaaccg ttatgaactt catcactgat aacctgattc cagtcactat cggaaacatc    180
   atcggcggtg gtttgttggt tgggttgaca tactgggtca tttacctgcg tggcgacgat    240
   catcattaat ggttgtctca ggcagtaaat aaaaaatcca cttaagaagg taggtgttac    300
   atgtccgagc ttaatgaaaa gttagccaca gcctgggaag gttttgcgaa aggtgactgg    360
   cagaacgaag tcaacgtacg tgactttatc cagaaaaact acaccccata tgaaggtgac    420
   gaatccttcc tggctggcgc aactgatgcg accaccaagc tgtgggacag cgtaatggaa    480
   ggcgttaaac aggaaaaccg cactcacgcg cctgttgatt tcgacacttc cctcgcatcc    540
   accatcactt ctcacgacgc gggctacatc gagaaagcgc tcgagaaaat cgttggtctg    600
   caaactgaag ccccgctgaa acgtgcgatt atcccgttcg gcggtatcaa aatggttgaa    660
   ggttcctgca aagcgtacaa tcgcgaactg gacccgatgc tgaaaaaaat cttcaccgag    720
   taccgtaaaa ctcacaacca gggcgttttc gacgtatata ccccggacat cctgcgctgc    780
   cgtaaatccg gcgtactgac cggtctgccg gatgcttacg gccgtggtcg tatcatcggt    840
   gactatcgtc gcgttgcgct gtacggtatc gacttcctga tgaaagacaa attcgcccag    900
   ttcaactcgc tgcaggcgaa actggaaagc ggcgaagatc tggaagcaac catccgtctg    960
   cgtgaagaaa ttgctgaaca gcatcgcgcg ctgggtcaga tcaaagagat ggcggctaaa   1020
   tatggctacg acatctccgg tccggcgact accgctcagg aagctattca gtggacctac   1080
   ttcggttacc tggccgccgt taaatctcag aacggcgcgg caatgtcctt cggtcgtact   1140
   tccagcttcc tggatatcta catcgaacgt gacctgcagg cgggtaaaat caccgagcaa   1200
   gacgcgcagg aaatggttga ccacctggtc atgaaactgc gtatggttcg cttcctgcgt   1260
   accccggaat atgatgaact gttctccggc gacccgattt gggcaacgga atctatcggt   1320
   ggtatgggcg ttgacggccg tactctggta accaaaaaca gcttccgctt cctgaacacc   1380
   ctgtacacca tggggccgtc tccggagccg aacatcacta tcctgtggtc tgaaaaactg   1440
   ccgctgagct ttaagaaatt cgccgctaaa gtatccatcg atacctcttc tctgcagtac   1500
   gagaacgatg acctgatgcg cccggacttc aacaacgacg attacgctat cgcatgctgc   1560
   gtaagcccga tgattgttgg taaacaaatg cagttcttcg gcgctcgcgc taacctcgcg   1620
   aaaaccatgc tgtatgctat caacggcggc gttgatgaaa aactgaaaat gcaggttggt   1680
   ccgaaatctg aaccgatcaa aggcgacgtc ctgaacttcg acgaagttat ggagcgcatg   1740
   gatcacttca tggactggct ggctaaacag tacgtcaccg cgctgaacat cattcattac   1800
   atgcatgaca agtacagcta cgaagcctct ctgatggcgc tgcacgaccg tgacgttatc   1860
   cgcaccatgg cgtgtggtat cgcaggtctg tccgttgctg ctgactccct gtctgctatc 1920
   aaatatgcga aagttaaacc gattcgtgac gaagacggtc tggctgttga cttcgaaatc 1980
   gaaggcgaat acccgcagtt tggtaacaac gatgctcgcg tcgatgacat ggccgttgac 2040
   ctggttgaac gtttcatgaa gaaaattcag aaactgcaca cctaccgcaa cgctatcccg 2100
   actcagtccg ttctgaccat cacttctaac gtcgtgtatg gtaagaaaac cggtaacacc 2160
   ccagatggtc gtcgcgctgg cgcgccgttc ggaccaggtg ctaacccgat gcacggccgt 2220
   gaccagaaag gtgctgtagc ctctctgact tccgttgcta aactgccgtt tgcttacgct 2280
   aaagatggta tctcttacac cttctctatc gtgccgaacg cgctgggtaa agatgacgaa 2340
   gttcgtaaga ccaacctggc gggcctgatg gatggttact tccaccacga agcgtccatc 2400
   gaaggtggtc agcacctgaa cgtgaacgtc atgaaccgcg aaatgctgct cgacgcgatg 2460
   gaaaacccgg aaaaatatcc gcagctgacc attcgtgtat ctggctacgc ggtacgtttt 2520
   aactccctga ctaaagaaca gcagcaggat gttattaccc gtaccttcac tcagaccatg 2580
   taattccctg tctgactgaa aaagcgtaca ataaaggccc cacatcagtg gggccttttt 2640
   aacacgtgat tccctgcccc agcctgcttt gccagttatc tatactttgg gtacctgtca 2700
   aaacagactt aacacagccg gtttgagctg tgcatcacag gccctggagg gccgaacccg 2760
   gagatatcac cgcaatgtca actattggtc gtattcactc ctttgaatcc tgtggcaccg 2820
   tcgatggccc aggcatccgc tttattacct tcttccaggg ctgcctgatg cgctgccttt 2880
   act 2883
<210> 14
   <211> 60
   <212> DNA
   <213> Artificial Sequence
<220>
   <223> Primer for amplifying MVD gene from E.coli MG1655 Ptac-KDyI strain
<400> 14
   cggataacaa tttcacacaa ggagactgcc atgaccgttt acacagcatc cgttaccgca 60
<210> 15
   <211> 90
   <212> DNA
   <213> Artificial Sequence
<220>
   <223> Primer for amplifying MVD gene from E.coli MG1655 Ptac-KDyI strain
<400> 15
   gttaaaaagg ccccactgat gtggggcctt tattgtacgc tttttcagtc agacagggaa 60
   ttattccttt ggtagaccag tctttgcgtc 90
<210> 16
   <211> 89
   <212> DNA
   <213> Artificial Sequence
<220>
   <223> Primer for amplifying lambda attL-Kmr- lambda attR-Ptac cassett
<400> 16
   catcattaat ggttgtctca ggcagtaaat aaaaaatcca cttaagaagg taggtgttac 60
   tgaagcctgc ttttttatac taagttggc 89
<210> 17
   <211> 60
   <212> DNA
   <213> Artificial Sequence
<220>
   <223> Primer for amplifying lambda attL-Kmr- lambda attR-Ptac cassette
<400> 17
   tgcggtaacg gatgctgtgt aaacggtcat ggcagtctcc ttgtgtgaaa ttgttatccg 60
<210> 18
   <211> 20
   <212> DNA
   <213> Artificial Sequence
<220>
   <223> Primer for amplifying delta pflB:: lambda attL-Kmr-lambda attR-Ptac-MVD
<400> 18
   ggctttgaac acagcatcgc 20
<210> 19
   <211> 18
   <212> DNA
   <213> Artificial Sequence
<220>
   <223> Primer for amplifying delta pflB:: lambda attL-Kmr-lambda attR-Ptac-MVD
<400> 19
   ctgttttgac aggtaccc 18
<210> 20
   <211> 1341
   <212> DNA
   <213> Enterobacter aerogenes
<400> 20
   atatccgcag ctgaccattc gtgtatctgg ctacgcggta cgttttaact ccctgactaa      60
   agaacagcag caggatgtta ttacccgtac cttcactcag accatgtaat tccctgtctg     120
   actgaaaaag cgtacaataa aggccccaca tcagtggggc ctttttaaca cgtgattccc     180
   tgccccagcc tgctttgcca gttatctata ctttgggtac ctgtcaaaac agacttaaca     240
   cagccggttt gagctgtgca tcacaggccc tggagggccg aacccggaga tatcaccgca     300
   atgtcaacta ttggtcgtat tcactccttt gaatcctgtg gcaccgtcga tggcccaggc     360
   atccgcttta ttaccttctt ccagggctgc ctgatgcgct gcctttactg ccataaccgc     420
   gacacctggg atacccatgg cggtaaagaa atcaccgttg aagaattgat gaaagaagtg     480
   gtgacctatc gccactttat gaatgcctcg ggcggcggcg tcaccgcctc cggcggcgag     540
   gcgatcctgc aggctgaatt tgtccgcgac tggttccgcg cctgtaaaaa agaaggtatc     600
   cacacctgtc tggataccaa cggcttcgta cgccgttacg atccggtgat tgacgagctg     660
   ctggaagtca ccgatctggt gatgcttgac ctcaagcaga tgaacgatga aattcaccag     720
   aacctggtcg gcgtttctaa ccaccgtacg ctggaattcg cccagtattt gtcgaagaaa     780
   gggattaacg tgtggatccg ctacgtggtg gttcccggct ggtctgatga tgacgattcc     840
   gcacatcgtc tgggtgagtt tacccgcgat atgggtaacg tcgagaaaat cgaactcctg     900
   ccctaccatg agctgggtaa acacaaatgg gtggcaatgg gcgaagagta caaacttgac     960
   ggcgtacacc caccgaagaa aaagaccatg gagcgggtaa aaggcatcct ggagcaatat    1020
   ggccataagg tgatgtacta aaccggcagc gggccggagg tactctcacc acggcccgca    1080
   actataatag tatcaatccc gccgataacg cctcatcatg agcgcgatac cgtctgcccg    1140
   cagaaatatt cattaatcaa aacggactac gcgcggcctt cgccgcgcgc cagattggtt    1200
   acgcgtgtgc caccggcgtc ggatgatgcc cggctttgcg cagcagggtc agcagataga    1260
   taaacgatac gctggcgatc atgataaaca gcagattatc cgagaagttc tgcatcaaca    1320
   tcgccgtcag ggtcggcccc a                                              1341
<210> 21
   <211> 81
   <212> DNA
   <213> Artificial Sequence
<220>
   <223> Primer for amplifying yIDI gene from E. coli MG1655 Ptac-KDyI strain
<400> 21
   cggataacaa tttcacacaa ggagactgcc atgaccgcgg ataacaacag catgccccat 60
   ggtgcagtat ctagttacgc c 81
<210> 22
   <211> 90
   <212> DNA
   <213> Artificial Sequence
<220>
   <223> Primer for amplifying yIDI gene from E. coli MG1655 Ptac-KDyI strai
<400> 22
   cgggattgat actattatag ttgcgggccg tggtgagagt acctccggcc cgctgccggt 60
   ttatagcatt ctatgaattt gcctgtcatt 90
<210> 23
   <211> 89
   <212> DNA
   <213> Artificial Sequence
<220>
   <223> Primer for confirming lambda attL-Kmr-lambda attR-Ptac cassette
<400> 23
   cagccggttt gagctgtgca tcacaggccc tggagggccg aacccggaga tatcaccgca 60
   tgaagcctgc ttttttatac taagttggc 89
<210> 24
   <211> 81
   <212> DNA
   <213> Artificial Sequence
<220>
   <223> Primer for confirming lambda attL-Kmr-lambda attR-Ptac cassett
<400> 24
   ggcgtaacta gatactgcac catggggcat gctgttgtta tccgcggtca tggcagtctc 60
   cttgtgtgaa attgttatcc g 81
<210> 25
   <211> 22
   <212> DNA
   <213> Artificial Sequence
<220>
   <223> Primer for confirming delta pflA:: lambda attL-Kmr-lambda attR-Ptac-yID
<400> 25
   cagttatcta tactttgggt ac 22
<210> 26
   <211> 23
   <212> DNA
   <213> Artificial Sequence
<220>
   <223> Primer for confirming delta pflA:: lambda attL-Kmr-lambda attR-Ptac-yIDI
<400> 26
   tttgattaat gaatatttct gcg 23
<210> 27
   <211> 30
   <212> DNA
   <213> Artificial Sequence
<220>
   <223> Primer for amplifying DNA fragment containing promoter region of bud operon and ORF region of BudR
<400> 27
   gagccacttc ctcgttcaac aaatataaga 30
<210> 28
   <211> 30
   <212> DNA
   <213> Artificial Sequence
<220>
   <223> Primer for amplifying DNA fragment containing promoter region of bud operon and ORF region of BudR
<400> 28
   ttagaacatc tctaaaaatc gcttcaccgt 30
<210> 29
   <211> 979
   <212> DNA
   <213> Artificial Sequence
<220>
   <223> DNA fragment containing promoter region of bud operon (1st to 106th positions) and ORF region of BudR (107th to 979th positions)
<400> 29
   gagccacttc ctcgttcaac aaatataaga aaggttaaat aaaggttgac ccgattcagc 60
   tcacagttcc aatatagaaa ccatgctggt ttgagacgtt ttcgatatgg aacttcgtta 120
   tctacgctat tttgtcgccg tcgcccggac gcggcacttc acccgggcag cgaaagaact 180
   gggtatctcg cagccaccgt taagtcagca aattcagcgg cttgagcgag aggttggcac 240
   tccgctgttg cgtcggctaa cccgcggggt ggagctgacc gaagccgggg agtcctttta 300
   tgaagatgcc tgccaaatcc tcgcgctgag cgatgcggcg ctggaaaagg ccaagggcat 360
   tgcccgcgga atgaacggca gcctgtcgtt aggcattacc agttctgatg ctttccatcc 420
   acaaatcttc accttgctgc accgttttca gctcgatcac ccaggcgtcg tcctccatca 480
   gcgggagggc aacatggcaa atttgatggc ggcgctgagc gaggcggaga tcgatatcgc 540
   ctttgtccga ttgccgtgtg aaagcagtaa ggcgtttaac ttgcgtatca ttgatgaaga 600
   gccaatggtc attgcgctgc cgcgcgataa tccattgtca gcggaaccga cgctggcgct 660
   ggaacagttg cgggacgtcg cgccgatcct cttcccgcgc gaagtggcgc cgggtttgta 720
   tgagctggtg ttcaatagct gcctgcgcgc cggtatcgat atgaaccgcg ccaggcagtc 780
   gtcgcagatt tcatcgtcgc tgagtatggt taacgccgga tttggtttcg cgctggtgcc 840
   gcagtcgatg acctgtatcg cgctgcccaa cgtcagctat caatcgatac aggggacgcc 900
   ggtcaagacc gatattgcca tcgcctggcg gcgttttgag cgctcgcgca cggtgaagcg 960
   atttttagag atgttctaa 979
<210> 30
   <211> 45
   <212> DNA
   <213> Artificial Sequence
<220>
   <223> Primer for amplifying DNA fragment of pMW-Para-mvaES-Ttrp in which arabinose promoter is deleted
<400> 30
   acgaggaagt ggctcatgaa aaccgtggtt attatcgatg cgctg 45
<210> 31
   <211> 45
   <212> DNA
   <213> Artificial Sequence
<220>
   <223> Primer for amplifying DNA fragment of pMW-Para-mvaES-Ttrp in which arabinose promoter is deleted
<400> 31
   ttagagatgt tctaaactgg ccgtcgtttt acaacgtcgt gactg 45
<210> 32
   <211> 803
   <212> PRT
   <213> Enterococcus faecalis
<400> 32
   Met Lys Thr Val Val Ile Ile Asp Ala Leu Arg Thr Pro Ile Gly Lys
   1               5                   10                  15
   Tyr Lys Gly Ser Leu Ser Gln Val Ser Ala Val Asp Leu Gly Thr His
               20                  25                  30
   Val Thr Thr Gln Leu Leu Lys Arg His Ser Thr Ile Ser Glu Glu Ile
           35                  40                  45
   Asp Gln Val Ile Phe Gly Asn Val Leu Gln Ala Gly Asn Gly Gln Asn
       50                  55                  60
   Pro Ala Arg Gln Ile Ala Ile Asn Ser Gly Leu Ser His Glu Ile Pro
   65                  70                  75                  80
   Ala Met Thr Val Asn Glu Val Cys Gly Ser Gly Met Lys Ala Val Ile
                   85                  90                  95
   Leu Ala Lys Gln Leu Ile Gln Leu Gly Glu Ala Glu Val Leu Ile Ala
               100                 105                 110
   Gly Gly Ile Glu Asn Met Ser Gln Ala Pro Lys Leu Gln Arg Phe Asn
           115                 120                 125
   Tyr Glu Thr Glu Ser Tyr Asp Ala Pro Phe Ser Ser Met Met Tyr Asp
       130                 135                 140
   Gly Leu Thr Asp Ala Phe Ser Gly Gln Ala Met Gly Leu Thr Ala Glu
   145                 150                 155                 160
   Asn Val Ala Glu Lys Tyr His Val Thr Arg Glu Glu Gln Asp Gln Phe
                   165                 170                 175
   Ser Val His Ser Gln Leu Lys Ala Ala Gln Ala Gln Ala Glu Gly Ile
               180                 185                 190
   Phe Ala Asp Glu Ile Ala Pro Leu Glu Val Ser Gly Thr Leu Val Glu
           195                 200                 205
   Lys Asp Glu Gly Ile Arg Pro Asn Ser Ser Val Glu Lys Leu Gly Thr
       210                 215                 220
   Leu Lys Thr Val Phe Lys Glu Asp Gly Thr Val Thr Ala Gly Asn Ala
   225                 230                 235                 240
   Ser Thr Ile Asn Asp Gly Ala Ser Ala Leu Ile Ile Ala Ser Gln Glu
                   245                 250                 255
   Tyr Ala Glu Ala His Gly Leu Pro Tyr Leu Ala Ile Ile Arg Asp Ser
               260                 265                 270
   Val Glu Val Gly Ile Asp Pro Ala Tyr Met Gly Ile Ser Pro Ile Lys
           275                 280                 285
   Ala Ile Gln Lys Leu Leu Ala Arg Asn Gln Leu Thr Thr Glu Glu Ile
       290                 295                 300
   Asp Leu Tyr Glu Ile Asn Glu Ala Phe Ala Ala Thr Ser Ile Val Val
   305                 310                 315                 320
   Gln Arg Glu Leu Ala Leu Pro Glu Glu Lys Val Asn Ile Tyr Gly Gly
                   325                 330                 335
   Gly Ile Ser Leu Gly His Ala Ile Gly Ala Thr Gly Ala Arg Leu Leu
               340                 345                 350
   Thr Ser Leu Ser Tyr Gln Leu Asn Gln Lys Glu Lys Lys Tyr Gly Val
           355                 360                 365
   Ala Ser Leu Cys Ile Gly Gly Gly Leu Gly Leu Ala Met Leu Leu Glu
       370                 375                 380
   Arg Pro Gln Gln Lys Lys Asn Ser Arg Phe Tyr Gln Met Ser Pro Glu
   385                 390                 395                 400
   Glu Arg Leu Ala Ser Leu Leu Asn Glu Gly Gln Ile Ser Ala Asp Thr
                   405                 410                 415
   Lys Lys Glu Phe Glu Asn Thr Ala Leu Ser Ser Gln Ile Ala Asn His
               420                 425                 430
   Met Ile Glu Asn Gln Ile Ser Glu Thr Glu Val Pro Met Gly Val Gly
           435                 440                 445
   Leu His Leu Thr Val Asp Glu Thr Asp Tyr Leu Val Pro Met Ala Thr
       450                 455                 460
   Glu Glu Pro Ser Val Ile Ala Ala Leu Ser Asn Gly Ala Lys Ile Ala
   465                 470                 475                 480
   Gln Gly Phe Lys Thr Val Asn Gln Gln Arg Leu Met Arg Gly Gln Ile
                   485                 490                 495
   Val Phe Tyr Asp Val Ala Asp Ala Glu Ser Leu Ile Asp Glu Leu Gln
               500                 505                 510
   Val Arg Glu Thr Glu Ile Phe Gln Gln Ala Glu Leu Ser Tyr Pro Ser
           515                 520                 525
   Ile Val Lys Arg Gly Gly Gly Leu Arg Asp Leu Gln Tyr Arg Ala Phe
       530                 535                 540
   Asp Glu Ser Phe Val Ser Val Asp Phe Leu Val Asp Val Lys Asp Ala
   545                 550                 555                 560
   Met Gly Ala Asn Ile Val Asn Ala Met Leu Glu Gly Val Ala Glu Leu
                   565                 570                 575
   Phe Arg Glu Trp Phe Ala Glu Gln Lys Ile Leu Phe Ser Ile Leu Ser
   580 585 590
   Asn Tyr Ala Thr Glu Ser Val Val Thr Met Lys Thr Ala Ile Pro Val
   595 600 605
   Ser Arg Leu Ser Lys Gly Ser Asn Gly Arg Glu Ile Ala Glu Lys Ile
   610 615 620
   Val Leu Ala Ser Arg Tyr Ala Ser Leu Asp Pro Tyr Arg Ala Val Thr
   625 630 635 640
   His Asn Lys Gly Ile Met Asn Gly Ile Glu Ala Val Val Leu Ala Thr
   645 650 655
   Gly Asn Asp Thr Arg Ala Val Ser Ala Ser Cys His Ala Phe Ala Val
   660 665 670
   Lys Glu Gly Arg Tyr Gln Gly Leu Thr Ser Trp Thr Leu Asp Gly Glu
   675 680 685
   Gln Leu Ile Gly Glu Ile Ser Val Pro Leu Ala Leu Ala Thr Val Gly
   690 695 700
   Gly Ala Thr Lys Val Leu Pro Lys Ser Gln Ala Ala Ala Asp Leu Leu
   705 710 715 720
   Ala Val Thr Asp Ala Lys Glu Leu Ser Arg Val Val Ala Ala Val Gly
   725 730 735
   Leu Ala Gln Asn Leu Ala Ala Leu Arg Ala Leu Val Ser Glu Gly Ile
   740 745 750
   Gln Lys Gly His Met Ala Leu Gln Ala Arg Ser Leu Ala Met Thr Val
   755 760 765
   Gly Ala Thr Gly Lys Glu Val Glu Ala Val Ala Gln Gln Leu Lys Arg
   770 775 780
   Gln Lys Thr Met Asn Gln Asp Arg Ala Leu Ala Ile Leu Asn Asp Leu
   785 790 795 800
   Arg Lys Gln
<210> 33
   <211> 2412
   <212> DNA
   <213> Enterococcus faecalis
<400> 33
   atgaaaacag tagttattat tgatgcatta cgaacaccaa ttggaaaata taaaggcagc 60
   ttaagtcaag taagtgccgt agacttagga acacatgtta caacacaact tttaaaaaga 120
   cattccacta tttctgaaga aattgatcaa gtaatctttg gaaatgtttt acaagctgga 180
   aatggccaaa atcccgcacg acaaatagca ataaacagcg gtttgtctca tgaaattccc 240
   gcaatgacgg ttaatgaggt ctgcggatca ggaatgaagg ccgttatttt ggcgaaacaa 300
   ttgattcaat taggagaagc ggaagtttta attgctggcg ggattgagaa tatgtcccaa 360
   gcacctaaat tacaacgttt taattacgaa acagaaagct acgatgcgcc tttttctagt 420
   atgatgtatg atggattaac ggatgccttt agtggtcagg caatgggctt aactgctgaa 480
   aatgtggccg aaaagtatca tgtaactaga gaagagcaag atcaattttc tgtacattca 540
   caattaaaag cagctcaagc acaagcagaa gggatattcg ctgacgaaat agccccatta 600
   gaagtatcag gaacgcttgt ggagaaagat gaagggattc gccctaattc gagcgttgag 660
   aagctaggaa cgcttaaaac agtttttaaa gaagacggta ctgtaacagc agggaatgca 720
   tcaaccatta atgatggggc ttctgctttg attattgctt cacaagaata tgccgaagca 780
   cacggtcttc cttatttagc tattattcga gacagtgtgg aagtcggtat tgatccagcc 840
   tatatgggaa tttcgccgat taaagccatt caaaaactgt tagcgcgcaa tcaacttact 900
   acggaagaaa ttgatctgta tgaaatcaac gaagcatttg cagcaacttc aatcgtggtc 960
   caaagagaac tggctttacc agaggaaaag gtcaacattt atggtggcgg tatttcatta 1020
   ggtcatgcga ttggtgccac aggtgctcgt ttattaacga gtttaagtta tcaattaaat 1080
   caaaaagaaa agaaatatgg agtggcttct ttatgtatcg gcggtggctt aggactcgct 1140
   atgctactag agagacctca gcaaaaaaaa aacagccgat tttatcaaat gagtcctgag 1200
   gaacgcctgg cttctcttct taatgaaggc cagatttctg ctgatacaaa aaaagaattt 1260
   gaaaatacgg ctttatcttc gcagattgcc aatcatatga ttgaaaatca aatcagtgaa 1320
   acagaagtgc cgatgggcgt tggcttacat ttaacagtgg acgaaactga ttatttggta 1380
   ccaatggcga cagaagagcc ctcagttatt gcggctttga gtaatggtgc aaaaatagca 1440
   caaggattta aaacagtgaa tcaacaacgc ttaatgcgtg gacaaatcgt tttttacgat 1500
   gttgcagatc ccgagtcatt gattgataaa ctacaagtaa gagaagcgga agtttttcaa 1560
   caagcagagt taagttatcc atctatcgtt aaacggggcg gcggcttaag agatttgcaa 1620
   tatcgtactt ttgatgaatc atttgtatct gtcgactttt tagtagatgt taaggatgca 1680
   atgggggcaa atatcgttaa cgctatgttg gaaggtgtgg ccgagttgtt ccgtgaatgg 1740
   tttgcggagc aaaagatttt attcagtatt ttaagtaatt atgccacgga gtcggttgtt 1800
   acgatgaaaa cggctattcc agtttcacgt ttaagtaagg ggagcaatgg ccgggaaatt 1860
   gctgaaaaaa ttgttttagc ttcacgctat gcttcattag atccttatcg ggcagtcacg   1920
   cataacaaag gaatcatgaa tggcattgaa gctgtagttt tagctacagg aaatgataca   1980
   cgcgctgtta gcgcttcttg tcatgctttt gcggtgaagg aaggtcgcta ccaaggcttg   2040
   actagttgga cgctggatgg cgaacaacta attggtgaaa tttcagttcc gcttgcttta   2100
   gccacggttg gcggtgccac aaaagtctta cctaaatctc aagcagctgc tgatttgtta   2160
   gcagtgacgg atgcaaaaga actaagtcga gtagtagcgg ctgttggttt ggcacaaaat   2220
   ttagcggcgt tacgggcctt agtctctgaa ggaattcaaa aaggacacat ggctctacaa   2280
   gcacgttctt tagcgatgac ggtcggagct actggtaaag aagttgaggc agtcgctcaa   2340
   caattaaaac gtcaaaaaac gatgaaccaa gaccgagcca tggctatttt aaatgattta   2400
   agaaaacaat aa                                                       2412
<210> 34
   <211> 2412
   <212> DNA
   <213> Artificial Sequence
<220>
   <223> DNA having modified codons, which encodes mvaE derived from Enterococcus faecalis
<400> 34
   atgaaaaccg tggttattat cgatgcgctg cgcacgccga ttggtaaata taaaggcagc     60
   ctgtctcaag tgagcgccgt tgatctgggt acgcatgtga ccacgcagct gctgaaacgt    120
   cacagcacca tctctgaaga aattgatcag gtgatctttg gtaacgttct gcaagccggt    180
   aatggtcaga atccggcacg tcagattgca atcaacagtg gcctgagcca tgaaattccg    240
   gcgatgaccg tgaatgaagt ttgcggtagc ggcatgaaag cggttattct ggccaaacag    300
   ctgatccagc tgggtgaagc ggaagtgctg attgccggcg gtatcgaaaa catgagtcag    360
   gcaccgaaac tgcaacgttt taattatgaa accgaaagct acgatgcccc gttcagctct    420
   atgatgtatg atggcctgac cgatgcattt agcggtcagg cgatgggcct gacggcagaa    480
   aacgtggcgg aaaaatacca tgttacccgc gaagaacagg atcagttttc tgttcacagt    540
   cagctgaaag cggcccaggc ccaggcagaa ggtattttcg ccgatgaaat cgcaccgctg    600
   gaagtgtctg gtacgctggt tgaaaaagat gaaggcattc gtccgaatag tagcgtggaa    660
   aaactgggca ccctgaaaac ggtgttcaaa gaagatggca ccgttacggc gggcaatgca    720
   agcaccatca atgatggtgc gagtgccctg attatcgcga gccaggaata tgcagaagcg    780
   catggcctgc cgtacctggc cattatccgc gattctgtgg aagttggtat tgatccggca    840
   tatatgggca ttagtccgat caaagcgatt cagaaactgc tggcccgtaa ccagctgacc    900
   accgaagaaa ttgatctgta cgaaatcaat gaagcgtttg cagcgaccag tattgtggtt    960
   cagcgcgaac tggccctgcc ggaagaaaaa gttaacattt atggcggtgg catcagcctg 1020
   ggtcacgcaa ttggtgccac cggtgcacgt ctgctgacca gtctgagcta tcagctgaat 1080
   cagaaagaga aaaaatacgg tgtggcaagc ctgtgtattg gtggcggtct gggtctggcc 1140
   atgctgctgg aacgtccgca gcagaagaaa aactctcgtt tttaccagat gagtccggaa 1200
   gaacgtctgg ccagtctgct gaacgaaggc cagattagcg cagataccaa aaaagaattc 1260
   gaaaatacgg cactgtctag tcagatcgcg aaccatatga ttgaaaatca gatcagcgaa 1320
   accgaagtgc cgatgggtgt tggcctgcac ctgaccgtgg atgaaacgga ttatctggtt 1380
   ccgatggcga cggaagaacc gagcgttatt gccgcactgt ctaacggtgc aaaaatcgcg 1440
   cagggcttta aaaccgtgaa tcagcagcgt ctgatgcgcg gccagattgt gttctacgat 1500
   gttgcggatc cggaaagcct gatcgataaa ctgcaagtgc gcgaagccga agtttttcag 1560
   caggcagaac tgagctatcc gtctattgtg aaacgtggcg gtggcctgcg cgatctgcaa 1620
   taccgtacct ttgatgaaag tttcgtgagc gttgatttcc tggtggatgt taaagatgcc 1680
   atgggtgcaa acatcgtgaa tgcgatgctg gaaggcgttg ccgaactgtt tcgtgaatgg 1740
   ttcgcggaac agaaaatcct gttttctatc ctgagtaact acgcgaccga aagcgtggtt 1800
   accatgaaaa cggccattcc tgtgagccgc ctgtctaaag gtagtaatgg ccgtgaaatt 1860
   gcggaaaaaa tcgttctggc gagccgctat gcctctctgg atccgtaccg tgccgtgacc 1920
   cataacaaag gtattatgaa tggcatcgaa gcagtggttc tggcgaccgg taacgatacc 1980
   cgtgccgtgt ctgcaagttg ccatgcattc gcagttaaag aaggtcgtta tcagggcctg 2040
   accagctgga cgctggatgg tgaacagctg atcggcgaaa tttctgtgcc gctggccctg 2100
   gcaaccgtgg gtggcgcgac gaaagttctg ccgaaaagcc aggcggccgc agatctgctg 2160
   gcggtgaccg atgcaaaaga actgtctcgc gtggttgcgg ccgttggtct ggcacagaat 2220
   ctggcagcgc tgcgtgcgct ggtgtctgaa ggtattcaga aaggccacat ggcactgcaa 2280
   gcccgtagtc tggccatgac cgtgggtgca acgggcaaag aagtggaagc agttgcgcag 2340
   cagctgaaac gccagaaaac catgaaccag gatcgtgcca tggcaatcct gaatgatctg 2400
   cgcaaacagt aa 2412
<210> 35
   <211> 383
   <212> PRT
   <213> Enterococcus faecalis
<400> 35
   Met Thr Ile Gly Ile Asp Lys Ile Ser Phe Phe Val Pro Pro Tyr Tyr
   1 5 10 15
   Ile Asp Met Thr Ala Leu Ala Glu Ala Arg Asn Val Asp Pro Gly Lys
               20                  25                  30
   Phe His Ile Gly Ile Gly Gln Asp Gln Met Ala Val Asn Pro Ile Ser
           35                  40                  45
   Gln Asp Ile Val Thr Phe Ala Ala Asn Ala Ala Glu Ala Ile Leu Thr
       50                  55                  60
   Lys Glu Asp Lys Glu Ala Ile Asp Met Val Ile Val Gly Thr Glu Ser
   65                  70                  75                  80
   Ser Ile Asp Glu Ser Lys Ala Ala Ala Val Val Leu His Arg Leu Met
                   85                  90                  95
   Gly Ile Gln Pro Phe Ala Arg Ser Phe Glu Ile Lys Glu Ala Cys Tyr
               100                 105                 110
   Gly Ala Thr Ala Gly Leu Gln Leu Ala Lys Asn His Val Ala Leu His
           115                 120                 125
   Pro Asp Lys Lys Val Leu Val Val Ala Ala Asp Ile Ala Lys Tyr Gly
       130                 135                 140
   Leu Asn Ser Gly Gly Glu Pro Thr Gln Gly Ala Gly Ala Val Ala Met
   145                 150                 155                 160
   Leu Val Ala Ser Glu Pro Arg Ile Leu Ala Leu Lys Glu Asp Asn Val
                   165                 170                 175
   Met Leu Thr Gln Asp Ile Tyr Asp Phe Trp Arg Pro Thr Gly His Pro
               180                 185                 190
   Tyr Pro Met Val Asp Gly Pro Leu Ser Asn Glu Thr Tyr Ile Gln Ser
           195                 200                 205
   Phe Ala Gln Val Trp Asp Glu His Lys Lys Arg Thr Gly Leu Asp Phe
       210                 215                 220
   Ala Asp Tyr Asp Ala Leu Ala Phe His Ile Pro Tyr Thr Lys Met Gly
   225                 230                 235                 240
   Lys Lys Ala Leu Leu Ala Lys Ile Ser Asp Gln Thr Glu Ala Glu Gln
                   245                 250                 255
   Glu Arg Ile Leu Ala Arg Tyr Glu Glu Ser Ile Ile Tyr Ser Arg Arg
               260                 265                 270
   Val Gly Asn Leu Tyr Thr Gly Ser Leu Tyr Leu Gly Leu Ile Ser Leu
           275                 280                 285
   Leu Glu Asn Ala Thr Thr Leu Thr Ala Gly Asn Gln Ile Gly Leu Phe
       290                 295                 300
   Ser Tyr Gly Ser Gly Ala Val Ala Glu Phe Phe Thr Gly Glu Leu Val
   305                 310                 315                 320
   Ala Gly Tyr Gln Asn His Leu Gln Lys Glu Thr His Leu Ala Leu Leu
                   325                 330                 335
   Asp Asn Arg Thr Glu Leu Ser Ile Ala Glu Tyr Glu Ala Met Phe Ala
               340                 345                 350
   Glu Thr Leu Asp Thr Asp Ile Asp Gln Thr Leu Glu Asp Glu Leu Lys
           355                 360                 365
   Tyr Ser Ile Ser Ala Ile Asn Asn Thr Val Arg Ser Tyr Arg Asn
       370                 375                 380
<210> 36
   <211> 1152
   <212> DNA
   <213> Enterococcus faecalis
<400> 36
   atgacaattg ggattgataa aattagtttt tttgtgcccc cttattatat tgatatgacg 60
   gcactggctg aagccagaaa tgtagaccct ggaaaatttc atattggtat tgggcaagac 120
   caaatggcgg tgaacccaat cagccaagat attgtgacat ttgcagccaa tgccgcagaa 180
   gcgatcttga ccaaagaaga taaagaggcc attgatatgg tgattgtcgg gactgagtcc 240
   agtatcgatg agtcaaaagc ggccgcagtt gtcttacatc gtttaatggg gattcaacct 300
   ttcgctcgct ctttcgaaat caaggaagct tgttacggag caacagcagg cttacagtta 360
   gctaagaatc acgtagcctt acatccagat aaaaaagtct tggtcgtagc ggcagatatt 420
   gcaaaatatg gcttaaattc tggcggtgag cctacacaag gagctggggc ggttgcaatg 480
   ttagttgcta gtgaaccgcg cattttggct ttaaaagagg ataatgtgat gctgacgcaa 540
   gatatctatg acttttggcg tccaacaggc cacccgtatc ctatggtcga tggtcctttg 600
   tcaaacgaaa cctacatcca atcttttgcc caagtctggg atgaacataa aaaacgaacc 660
   ggtcttgatt ttgcagatta tgatgcttta gcgttccata ttccttacac aaaaatgggc 720
   aaaaaagcct tattagcaaa aatctccgac caaactgaag cagaacagga acgaatttta 780
   gcccgttatg aagaaagtat cgtctatagt cgtcgcgtag gaaacttgta tacgggttca 840
   ctttatctgg gactcatttc ccttttagaa aatgcaacga ctttaaccgc aggcaatcaa 900
   attggtttat tcagttatgg ttctggtgct gtcgctgaat ttttcactgg tgaattagta 960
   gctggttatc aaaatcattt acaaaaagaa actcatttag cactgctgga taatcggaca 1020
   gaactttcta tcgctgaata tgaagccatg tttgcagaaa ctttagacac agacattgat 1080
   caaacgttag aagatgaatt aaaatatagt atttctgcta ttaataatac cgttcgttct 1140
   tatcgaaact aa 1152
<210> 37
   <211> 1152
   <212> DNA
   <213> Artificial Sequence
<220>
   <223> DNA having modified codons, which encodes mvaS derived from Enterococcus faecalis
<400> 37
   atgaccattg gtatcgataa aattagcttt ttcgtgccgc cgtattacat cgatatgacg      60
   gcgctggccg aagcacgtaa cgttgatccg ggcaaatttc atattggcat cggtcaggat     120
   cagatggcgg tgaacccgat ttctcaggat atcgttacct tcgcggccaa tgcagcggaa     180
   gcaattctga cgaaagaaga taaagaagcg attgatatgg tgatcgttgg caccgaaagc     240
   tctatcgatg aaagtaaagc cgcagcggtg gttctgcacc gtctgatggg cattcagccg     300
   tttgcgcgca gcttcgaaat caaagaagcc tgctatggcg cgaccgccgg tctgcaactg     360
   gccaaaaacc atgtggcact gcacccggat aaaaaagttc tggtggttgc cgcagatatt     420
   gcgaaatacg gtctgaatag cggcggtgaa ccgacccagg gtgcaggtgc cgtggcaatg     480
   ctggttgcat ctgaaccgcg tattctggcg ctgaaagaag ataacgtgat gctgacccag     540
   gatatctatg atttttggcg tccgaccggt catccgtacc cgatggtgga tggcccgctg     600
   agtaatgaaa cctatattca gagcttcgcc caggtttggg atgaacataa aaaacgtacg     660
   ggtctggatt ttgcggatta tgatgcactg gcgttccaca ttccgtacac caaaatgggc     720
   aaaaaagcgc tgctggccaa aatcagcgat cagacggaag ccgaacagga acgtattctg     780
   gcacgctatg aagaaagcat cgtgtactct cgtcgcgttg gcaacctgta taccggttct     840
   ctgtacctgg gcctgattag tctgctggaa aacgcgacca cgctgacggc cggcaatcag     900
   atcggtctgt tttcttatgg cagtggtgcc gtggcagaat ttttcaccgg tgaactggtt      960
   gccggctacc agaaccatct gcaaaaagaa acccacctgg ccctgctgga taatcgcacg    1020
   gaactgtcta ttgcagaata tgaagcaatg tttgcggaaa ccctggatac ggatatcgat    1080
   cagaccctgg aagatgaact gaaatatagt attagcgcga tcaacaatac ggtgcgtagt    1140
   taccgcaatt aa                                                        1152
<210> 38
   <211> 40
   <212> DNA
   <213> Artificial Sequence
<220>
   <223> Primer for amplifying a fragment comprising Para composed of araC and ara BAD promoters from E.coli
<400> 38
   tgaattcgag ctcggtaccc actcttcctt tttcaatatt 40
<210> 39
   <211> 40
   <212> DNA
   <213> Artificial Sequence
<220>
   <223> Primer for amplifying a fragment comprising Para composed of araC and ara BAD promoters from E.coli
<400> 39
   ataataacca cggttttcat tttttataac ctccttagag 40
<210> 40
   <211> 40
   <212> DNA
   <213> Artificial Sequence
<220>
   <223> Primer for amplifying a fragment comprising EFmvaE gene
<400> 40
   ctctaaggag gttataaaaa atgaaaaccg tggttattat 40
<210> 41
   <211> 55
   <212> DNA
   <213> Artificial Sequence
<220>
   <223> Primer for amplifying a fragment comprising EFmvaE gene
<400> 41
   ttatcgatac caatggtcat gtttttttac ctcctttact gtttgcgcag atcat 55
<210> 42
   <211> 55
   <212> DNA
   <213> Artificial Sequence
<220>
   <223> Primer for amplifying a fragment comprising EFmvaS gene
<400> 42
   atgatctgcg caaacagtaa aggaggtaaa aaaacatgac cattggtatc gataa 55
<210> 43
   <211> 40
   <212> DNA
   <213> Artificial Sequence
<220>
   <223> Primer for amplifying a fragment comprising EFmvaS gene
<400> 43
   cagcggaact ggcggctccc ttaattgcgg taactacgca 40
<210> 44
   <211> 40
   <212> DNA
   <213> Artificial Sequence
<220>
   <223> Primer for amplifying a fragment comprising Ttrp
<400> 44
   tgcgtagtta ccgcaattaa gggagccgcc agttccgctg 40
<210> 45
   <211> 38
   <212> DNA
   <213> Artificial Sequence
<220>
   <223> Primer for amplifying a fragment comprising Ttrp
<400> 45
   gtcgactcta gaggatccct aatgagaatt agtcaaat 38
<210> 46
   <211> 42
   <212> DNA
   <213> Artificial Sequence
<220>
   <223> Primer (Linker-F)
<400> 46
   agctttaggg ataacagggt aatctcgagc tgcaggcatg ca 42
<210> 47
   <211> 42
   <212> DNA
   <213> Artificial Sequence
<220>
   <223> Primer (Linker-R)
<400> 47
   agcttgcatg cctgcagctc gagattaccc tgttatccct aa 42
<210> 48
   <211> 55
   <212> DNA
   <213> Artificial Sequence
<220>
   <223> Primer (lldD5' CAS)
<400> 48
   tttttaagct ttagggataa cagggtaatc tcgagattta aagcggctgc tttac 55
<210> 49
   <211> 43
   <212> DNA
   <213> Artificial Sequence
<220>
   <223> Primer (lldD3' CAS)
<400> 49
   tttttaagct tgcatgcctg cagtatttaa tagaatcagg tag 43
<210> 50
   <211> 54
   <212> DNA
   <213> Artificial Sequence
<220>
   <223> Primer (phoC5' CAS)
<400> 50
   tttttaagct ttagggataa cagggtaatc tcgagtggat aacctcatgt aaac 54
<210> 51
   <211> 44
   <212> DNA
   <213> Artificial Sequence
<220>
   <223> Primer (phoC3' CAS)
<400> 51
   tttttaagct tgcatgcctg cagttgatgt ctgattatct ctga 44
<210> 52
   <211> 54
   <212> DNA
   <213> Artificial Sequence
<220>
   <223> Primer (pstS5' CAS)
<400> 52
   tttttaagct ttagggataa cagggtaatc tcgagagcct ctcacgcgtg aatc 54
<210> 53
   <211> 44
   <212> DNA
   <213> Artificial Sequence
<220>
   <223> pstS3' CAS
<400> 53
   tttttaagct tgcatgcctg cagaggggag aaaagtcagg ctaa 44
<210> 54
   <211> 33
   <212> DNA
   <213> Artificial Sequence
<220>
   <223> Primer (n67)
<400> 54
   tgcgaagacg tcctcgtgaa gaaggtgttg ctg 33
<210> 55
   <211> 36
   <212> DNA
   <213> Artificial Sequence
<220>
   <223> Primer (n68)
<400> 55
   tgcgaagggc cccgttgtgt ctcaaaatct ctgatg 36
<210> 56
   <211> 70
   <212> DNA
   <213> Artificial Sequence
<220>
   <223> Primer (ampH-attL-phi80)
<400> 56
   atgcgcactc cttacgtact ggctctactg gtttctttgc gaaaggtcat ttttcctgaa 60
   tatgctcaca 70
<210> 57
   <211> 64
   <212> DNA
   <213> Artificial Sequence
<220>
   <223> Primer (ampH-attR-phi80)
<400> 57
   ttaaggaatc gcctggacca tcatcggcga gccgttctga cgtttgttga cagctggtcc 60
   aatg 64
<210> 58
   <211> 68
   <212> DNA
   <213> Artificial Sequence
<220>
   <223> Primer (DampC-phL)
<400> 58
   ctgatgaact gtcacctgaa tgagtgctga tgaaaatata gaaaggtcat ttttcctgaa 60
   tatgctca 68
<210> 59
   <211> 64
   <212> DNA
   <213> Artificial Sequence
<220>
   <223> Primer (DampC-phR)
<400> 59
   attcgccagc ataacgatgc cgctgttgag ctgaggaaca cgtttgttga cagctggtcc 60
   aatg 64
<210> 60
   <211> 18
   <212> DNA
   <213> Artificial Sequence
<220>
   <223> Primer (ampH-t1)
<400> 60
   gcgaagccct ctccgttg 18
<210> 61
   <211> 21
   <212> DNA
   <213> Artificial Sequence
<220>
   <223> Primer (ampH-t2)
<400> 61
   agccagtcag cctcatcagc g 21
<210> 62
   <211> 21
   <212> DNA
   <213> Artificial Sequence
<220>
   <223> Primer (ampC-t1)
<400> 62
   gattcccact tcaccgagcc g 21
<210> 63
   <211> 21
   <212> DNA
   <213> Artificial Sequence
<220>
   <223> Primer (ampC-t2)
<400> 63
   ggcaggtatg gtgctctgac g 21
<210> 64
   <211> 64
   <212> DNA
   <213> Artificial Sequence
<220>
   <223> Primer (crtE-attRphi80)
<400> 64
   atgacggtct gcgcaaaaaa acacgttcat ctcactcgcg cgtttgttga cagctggtcc 60
   aatg 64
<210> 65
   <211> 68
   <212> DNA
   <213> Artificial Sequence
<220>
   <223> Primer (crtZ-attLphi80)
<400> 65
   atgttgtgga tttggaatgc cctgatcgtt ttcgttaccg gaaaggtcat ttttcctgaa 60
   tatgctca 68
<210> 66
   <211> 21
   <212> DNA
   <213> Artificial Sequence
<220>
   <223> Primer (crtZ-test)
<400> 66
   ccgtgtggtt ctgaaagccg a 21
<210> 67
   <211> 21
   <212> DNA
   <213> Artificial Sequence
<220>
   <223> Primer (crtE-test)
<400> 67
   cgttgccgta aatgtatccg t 21
<210> 68
   <211> 27
   <212> DNA
   <213> Artificial Sequence
<220>
   <223> Primer (phL-test)
<400> 68
   ggatgtaaac cataacactc tgcgaac 27
<210> 69
   <211> 25
   <212> DNA
   <213> Artificial Sequence
<220>
   <223> Primer (phR-test)
<400> 69
   gattggtggt tgaattgtcc gtaac 25
<210> 70
   <211> 3471
   <212> DNA
   <213> Artificial Sequence
<220>
   <223> Sequence of the chemically synthesized DNA fragment retaining artificial KDyI operon with optimized codons
<400> 70
   gcatgcagga ggtatgaatg tcagagttgc gtgccttcag tgccccaggg aaagcgttac     60
   tcgctggtgg atatttagtt ttagatacaa aatatgaagc atttgtagtc ggattatcgg    120
   cacgtatgca cgctgtagcc catccttacg gttcattgca agggtctgat aagtttgaag    180
   tgcgtgtgaa aagtaaacaa tttaaagatg gggagtggct gtaccatata agtcctaaaa    240
   gtggcttcat tcctgtttcg ataggcggat ctaagaaccc tttcattgaa aaagttatcg    300
   ctaacgtatt tagctacttt aaacctaaca tggacgacta ctgcaatcgt aacttgttcg    360
   ttattgatat tttctctgat gatgcctacc attctcagga ggatagcgtt accgaacatc    420
   gtggcaaccg ccgtttgagt tttcattcgc accgtattga agaagttccc aaaacagggc    480
   tgggctcctc ggcaggttta gtcacagttt taactacagc tttggcctcc ttttttgtat    540
   cggacctgga aaataatgta gacaaatatc gtgaagttat tcataattta gcacaagttg    600
   ctcattgtca agctcagggt aaaattggaa gcgggtttga tgtagcggcg gcagcatatg    660
   gatctatccg ttatcgccgt ttcccacccg cattaatctc taatttgcca gatattggaa    720
   gtgctactta cggcagtaaa ctggcgcatt tggttgatga agaagactgg aatattacga    780
   ttaaaagtaa ccatttacct tcgggattaa ctttatggat gggcgatatt aagaatggtt    840
   cagaaacagt aaaactggtc cagaaggtaa aaaattggta tgattcgcat atgccagaaa    900
   gcctcaaaat atatacagaa ctcgatcatg caaattctcg ttttatggat ggactctcta    960
   aactcgatcg cttacacgag actcatgacg attacagcga tcagatattt gagtctcttg 1020
   agcgtaatga ctgtacctgt caaaagtatc ctgaaatcac agaagttcgt gatgcagttg 1080
   ccacaattcg tcgttccttt cgtaaaataa ctaaagaatc tggtgccgat atcgaacctc 1140
   ccgtacaaac tagcttattg gatgattgcc agaccttaaa aggagttctt acttgcttaa 1200
   tacctggtgc tggtggttat gacgccattg cagtgattac taagcaagat gttgatcttc 1260
   gtgctcaaac cgctaatgac aaacgttttt ctaaggttca atggctggat gtaactcagg 1320
   ctgactgggg tgttcgtaaa gaaaaagatc cggaaactta tcttgataaa taactgcaga 1380
   ggaggtatga atgaccgttt acacagcatc cgttaccgca cccgtcaaca tcgcaaccct 1440
   taagtattgg gggaaacgtg acacgaagtt gaatctgccc accaattcgt ccatatcagt 1500
   gactttatcg caagatgacc tccgtacgtt gacctctgcg gctactgcac ctgagtttga 1560
   acgcgacact ttgtggttaa atggagaacc acacagcatc gacaatgaac gtactcaaaa 1620
   ttgtctgcgc gacctccgcc aattacgtaa ggaaatggaa tcgaaggacg cctcattgcc 1680
   cacattatct caatggaaac tccacattgt ctccgaaaat aactttccta cagcagctgg 1740
   tttagcttcc tccgctgctg gctttgctgc attggtctct gcaattgcta agttatacca 1800
   attaccacag tcaacttcag aaatatctcg tatagcacgt aaggggtctg gttcagcttg 1860
   tcgttcgttg tttggcggat acgtggcctg ggaaatggga aaagctgaag atggtcatga 1920
   ttccatggca gtacaaatcg cagacagctc tgactggcct cagatgaaag catgtgtcct 1980
   tgttgtcagc gatattaaaa aggatgtgag ttccactcag ggtatgcaat tgaccgtggc 2040
   aacctccgaa ctctttaaag aacgtattga acatgtcgta ccaaagcgtt ttgaagtcat 2100
   gcgtaaagcc attgttgaaa aagatttcgc cacctttgca aaggaaacaa tgatggattc 2160
   caactctttc catgccacat gtttggactc tttccctcca atattctaca tgaatgacac 2220
   ttccaagcgt atcatcagtt ggtgccacac cattaatcag ttttacggag aaacaatcgt 2280
   tgcatacacg tttgatgcag gtccaaatgc tgtgttgtac tacttagctg aaaatgagtc 2340
   gaaactcttt gcatttatct ataaattgtt tggctctgtt cctggatggg acaagaaatt 2400
   tactactgag cagcttgagg ctttcaacca tcaatttgaa tcatctaact ttactgcacg 2460
   tgaattggat cttgagttgc aaaaggatgt tgcccgtgtg attttaactc aagtcggttc 2520
   aggcccacaa gaaacaaacg aatctttgat tgacgcaaag actggtctcc caaaggaata 2580
   aggatccagg aggtatgaat gactgccgac aacaatagta tgccccatgg tgcagtatct 2640
   agttacgcca aattagtgca aaaccaaaca cctgaagaca ttttggaaga gtttcctgaa 2700
   attattccat tacaacaacg tcctaatacc cgctctagtg agacgtcaaa tgacgaaagc 2760
   ggagaaacat gtttttctgg tcatgatgag gagcaaatta agttaatgaa tgaaaattgt 2820
   attgttttgg attgggacga taatgctatt ggtgccggca ccaagaaagt ttgtcattta 2880
   atggaaaata ttgaaaaggg tttacttcat cgtgcattct ccgtctttat tttcaatgaa 2940
   caaggtgaat tacttttaca acaacgtgcc actgaaaaaa taactttccc tgatctttgg 3000
   actaacacat gctgctctca tccactttgt attgatgacg aattaggttt gaagggtaag 3060
   ctcgacgata agattaaggg cgctattact gcggcggtgc gtaaactcga tcatgaatta 3120
   ggtattccag aagatgaaac taagacacgt ggtaagtttc actttttaaa ccgtatccat 3180
   tacatggcac caagcaatga accatggggt gaacatgaaa ttgattacat cctcttttat 3240
   aagatcaacg ctaaagaaaa cttgactgtc aacccaaacg tcaatgaagt tcgtgacttc 3300
   aaatgggttt caccaaatga tttgaaaact atgtttgctg acccaagtta caagtttacg 3360
   ccttggttta agattatttg cgagaattac ttattcaact ggtgggagca attagatgac 3420
   ctttctgaag tggaaaatga ccgtcaaatt catcgtatgc tctaaggtac c 3471
<210> 71
   <211> 68
   <212> DNA
   <213> Artificial Sequence
<220>
   <223> Primer gcd-attL
<400> 71
   ggtcaacatt atggggaaaa actcctcatc ctttagcgtg tgaagcctgc ttttttatac 60
   taagttgg 68
<210> 72
   <211> 68
   <212> DNA
   <213> Artificial Sequence
<220>
   <223> Primer gcd-attR
<400> 72
   ttacttctgg tcgggcagcg cataggcaat cacgtaatcg cgctcaagtt agtataaaaa 60
   agctgaac 68
<210> 73
   <211> 20
   <212> DNA
   <213> Artificial Sequence
<220>
   <223> Primer gcd-t1
<400> 73
   tgacaacaat ctatctgatt 20
<210> 74
   <211> 20
   <212> DNA
   <213> Artificial Sequence
<220>
   <223> Primer gcd-t2
<400> 74
   tgcgcctggt taagctggcg 20
<210> 75
   <211> 574
   <212> PRT
   <213> Actinidia arguta
<400> 75
   Met Ala Ser Phe Asn Arg Phe Cys Val Ser Ser Leu Leu Ala Pro Asn
   1               5                   10                  15
   Asn Ser Pro Gln Ile Ser Asn Ala Pro Arg Ser Thr Ala Val Pro Ser
               20                  25                  30
   Met Pro Thr Thr Gln Lys Trp Ser Ile Thr Glu Asp Leu Ala Phe Ile
           35                  40                  45
   Ser Asn Pro Ser Lys Gln His Asn His Gln Thr Gly Tyr Arg Ile Phe
       50                  55                  60
   Ser Asp Glu Phe Tyr Leu Lys His Glu Asn Lys Leu Lys Asp Val Arg
   65                  70                  75                  80
   Arg Ala Leu Arg Glu Val Glu Glu Thr Pro Leu Glu Gly Leu Val Met
                   85                  90                  95
   Ile Asp Thr Leu Gln Arg Leu Gly Ile Asp Tyr His Phe Gln Gly Glu
               100                 105                 110
   Ile Gly Ala Leu Leu Gln Lys Gln Gln Arg Ile Ser Thr Cys Asp Tyr
           115                 120                 125
   Pro Glu His Asp Leu Phe Glu Val Ser Thr Arg Phe Arg Leu Leu Arg
       130                 135                 140
   Gln Glu Gly His Asn Val Pro Ala Asp Val Phe Asn Asn Phe Arg Asp
   145                 150                 155                 160
   Lys Glu Gly Arg Phe Lys Ser Glu Leu Ser Arg Asp Ile Arg Gly Leu
                   165                 170                 175
   Met Ser Leu Tyr Glu Ala Ser Gln Leu Ser Ile Gln Gly Glu Asp Ile
               180                 185                 190
   Leu Asp Gln Ala Ala Asp Phe Ser Ser Gln Leu Leu Ser Gly Trp Ala
           195                 200                 205
   Thr Asn Leu Asp His His Gln Ala Arg Leu Val Arg Asn Ala Leu Thr
       210                 215                 220
   His Pro Tyr His Lys Ser Leu Ala Thr Phe Met Ala Arg Asn Phe Asn
   225                 230                 235                 240
   Tyr Asp Cys Lys Gly Gln Asn Gly Trp Val Asn Asn Leu Gln Glu Leu
                   245                 250                 255
   Ala Lys Met Asp Leu Thr Met Val Gln Ser Met His Gln Lys Glu Val
               260                 265                 270
   Leu Gln Val Ser Gln Trp Trp Lys Gly Arg Gly Leu Ala Asn Glu Leu
           275                 280                 285
   Lys Leu Val Arg Asn Gln Pro Leu Lys Trp Tyr Met Trp Pro Met Ala
       290                 295                 300
   Ala Leu Thr Asp Pro Arg Phe Ser Glu Glu Arg Val Glu Leu Thr Lys
   305                 310                 315                 320
   Pro Ile Ser Phe Ile Tyr Ile Ile Asp Asp Ile Phe Asp Val Tyr Gly
                   325                 330                 335
   Thr Leu Glu Glu Leu Thr Leu Phe Thr Asp Ala Val Asn Arg Trp Glu
               340                 345                 350
   Leu Thr Ala Val Glu Gln Leu Pro Asp Tyr Met Lys Ile Cys Phe Lys
           355                 360                 365
   Ala Leu Tyr Asp Ile Thr Asn Glu Ile Ala Tyr Lys Ile Tyr Lys Lys
       370                 375                 380
   His Gly Arg Asn Pro Ile Asp Ser Leu Arg Arg Thr Trp Ala Ser Leu
   385                 390                 395                 400
   Cys Asn Ala Phe Leu Glu Glu Ala Lys Trp Phe Ala Ser Gly Asn Leu
                   405                 410                 415
   Pro Lys Ala Glu Glu Tyr Leu Lys Asn Gly Ile Ile Ser Ser Gly Met
               420                 425                 430
   His Val Val Thr Val His Met Phe Phe Leu Leu Gly Gly Cys Phe Thr
   435 440 445
   Glu Glu Ser Val Asn Leu Val Asp Glu His Ala Gly Ile Thr Ser Ser
   450 455 460
   Ile Ala Thr Ile Leu Arg Leu Ser Asp Asp Leu Gly Ser Ala Lys Asp
   465 470 475 480
   Glu Asp Gln Asp Gly Tyr Asp Gly Ser Tyr Leu Glu Cys Tyr Leu Lys
   485 490 495
   Asp His Lys Gly Ser Ser Val Glu Asn Ala Arg Glu Glu Val Ile Arg
   500 505 510
   Met Ile Ser Asp Ala Trp Lys Arg Leu Asn Glu Glu Cys Leu Phe Pro
   515 520 525
   Asn Pro Phe Ser Ala Thr Phe Arg Lys Gly Ser Leu Asn Ile Ala Arg
   530 535 540
   Met Val Pro Leu Met Tyr Ser Tyr Asp Asp Asn His Asn Leu Pro Ile
   545 550 555 560
   Leu Glu Glu His Met Lys Thr Met Leu Tyr Asp Ser Ser Ser
   565 570
<210> 76
   <211> 1725
   <212> DNA
   <213> Actinidia arguta
<400> 76
   atggccagct tcaacaggtt ttgtgtctct tctcttcttg ctccaaacaa cagcccacaa 60
   attagcaatg ctccccgctc caccgctgta ccctctatgc ctaccaccca aaaatggagc 120
   atcaccgaag acctagcatt catttctaat ccctcgaaac aacacaacca tcaaaccgga 180
   tatcgcattt tctctgatga gttttaccta aagcacgaaa acaaattgaa ggacgttagg 240
   agagcgttaa gggaagtgga ggaaacccca ttagaaggtc tggtcatgat cgacaccctc 300
   caacggctag gcattgacta ccacttccag ggggagattg gagccctact acagaaacaa 360
   cagagaatat ctacttgtga ttatcccgag catgatcttt ttgaggtctc tactcgcttt 420
   cggctgttaa ggcaagaagg tcacaatgtg cctgcagatg tgtttaacaa cttcagagac 480
   aaggagggaa ggttcaaatc agaactaagc agagacatca gggggttgat gagtttgtat 540
   gaagcttcac agttaagcat acaaggagaa gacatacttg atcaagccgc agattttagt 600
   tcccaactcc ttagcgggtg ggcgacaaat ctcgatcatc atcaagctag gcttgtgcgt 660
   aatgcactga cacatcccta tcacaagagc ctagcgacat tcatggcaag aaacttcaat 720
   tatgattgca agggccaaaa tggatgggtc aataacttgc aagaactagc aaaaatggac 780
   ttaactatgg ttcagtccat gcatcaaaaa gaagtccttc aagtttccca atggtggaaa 840
   ggcaggggtt tggccaatga attgaagctt gtgagaaatc agccacttaa atggtacatg 900
   tggccaatgg cagccctcac agatccaaga ttctcagagg aaagagttga actcacaaaa 960
   ccaatctctt ttatctatat catagatgac atttttgatg tttatgggac attagaagaa 1020
   ctcactctct tcacagatgc tgtcaataga tgggaactta ctgctgttga gcaactaccc 1080
   gactacatga agatttgctt taaggctctt tatgacatca caaatgaaat cgcctacaag 1140
   atctacaaaa agcatggacg gaaccccata gattctctgc ggagaacgtg ggcaagtttg 1200
   tgcaacgcgt tcttagaaga agcaaaatgg tttgcttctg ggaacttgcc aaaggcagaa 1260
   gagtacttga agaatgggat catcagttca gggatgcatg tggttacggt tcacatgttc 1320
   tttctcttgg gcggttgttt caccgaagaa agtgtcaatc ttgtggatga acatgcggga 1380
   attacatctt ctatagcaac aatccttcgt ctttcggatg acttgggaag tgccaaggat 1440
   gaggatcaag atggctacga tggatcctat ttagaatgct atctgaagga ccacaagggc 1500
   tcttcggtag agaatgcaag agaagaagtt attcgcatga tttcagatgc atggaagcgc 1560
   ctcaacgagg aatgcctatt tccgaatcca ttttcagcaa ctttcaggaa gggttctctt 1620
   aatatcgcaa ggatggttcc tttgatgtac agctatgatg acaatcataa cctcccaatc 1680
   cttgaggagc acatgaagac aatgctctat gatagttctt cttga 1725
<210> 77
   <211> 1650
   <212> DNA
   <213> Artificial Sequence
<220>
   <223> DNA having modified codons, which encodes linalool synthase gene derived from Actinidia arguta (opt_AaLINS)
<400> 77
   atgtccaccg ccgtgccctc tatgcccact acccaaaaat ggtctattac cgaagactta     60
   gcctttatta gcaatcccag caaacaacat aatcaccaaa ccggctaccg gatttttagt    120
   gacgaatttt acctgaaaca tgaaaacaaa ttgaaagatg tgcggcgcgc cttgcgtgaa    180
   gttgaagaaa cccccctgga aggcttggtg atgattgaca ctttacagcg gctgggtatt    240
   gattaccact ttcaaggcga aattggtgcc ttgttacaga aacaacagcg cattagtacc    300
   tgtgactatc ccgaacatga tttgtttgaa gtgagcactc gctttcgtct gttgcgtcaa    360
   gaaggtcaca atgtgcccgc cgacgttttt aataactttc gcgataaaga agggcgtttt    420
   aaatctgaac tgtcccggga tattcgcgga ttgatgtcct tatacgaagc cagtcaactg    480
   agcattcagg gggaagacat tttggatcaa gccgctgact tttccagtca gttactgtct     540
   ggatgggcca ccaatttaga tcatcaccaa gcccgtctgg tgcggaacgc tttgacccat     600
   ccctaccaca aaagtctggc cacttttatg gctcgcaact ttaactacga ttgcaaaggg     660
   caaaacggat gggtgaataa cctgcaggaa ttggccaaaa tggatttaac catggttcaa     720
   agtatgcatc agaaagaagt gctgcaagtt agccagtggt ggaaagggcg gggattggcc     780
   aatgaactga aattggtgcg caaccaaccc ttgaaatggt atatgtggcc catggccgct     840
   ttaaccgatc cccggttttc tgaagaacgc gtggaattga ctaaacccat ttcctttatt     900
   tacattattg atgacatttt tgacgtttat ggcaccttag aagaattaac cctgtttact     960
   gatgccgtga atcggtggga attaactgct gttgaacagc tgcccgacta catgaaaatt    1020
   tgttttaaag ccttgtacga tattaccaac gaaattgctt acaaaattta caaaaaacat    1080
   gggcgcaacc ccattgatag tttacgtcgg acttgggcca gcttatgcaa tgcttttctg    1140
   gaagaagcca aatggtttgc tagtggcaat ttgcccaaag ccgaagaata cctgaaaaac    1200
   gggattatta gctctggaat gcatgtggtt accgtgcaca tgtttttctt gttaggcggt    1260
   tgttttactg aagaatccgt gaatttggtt gatgaacatg ccggcattac ctccagtatt    1320
   gctactattt tgcgtttatc tgatgactta ggttccgcca aagatgaaga ccaagatggc    1380
   tatgacggta gctacttgga atgttacctg aaagatcata aaggtagctc tgtggaaaat     1440
   gcccgtgaag aagttattcg gatgatttcc gatgcttgga aacgcttgaa tgaagaatgc    1500
   ttatttccca accccttttc tgccaccttt cgcaaagggt ccttaaatat tgctcgtatg    1560
   gtgcccctga tgtacagtta cgatgacaac cataacctgc ccattctgga agaacacatg    1620
   aaaaccatgt tgtatgattc cagtagctaa                                     1650
<210> 78
   <211> 590
   <212> PRT
   <213> Coriandrum sativum
<400> 78
   Met Ala Ala Ile Thr Ile Phe Pro Leu Ser Tyr Ser Ile Lys Phe Arg
   1               5                   10                  15
   Arg Ser Ser Pro Cys Asn Pro Lys Asp Val Thr Ala Cys Lys Ser Val
               20                  25                  30
   Ile Lys Ser Val Thr Gly Met Thr Lys Val Pro Val Pro Val Pro Glu
           35                  40                  45
   Pro Ile Val Arg Arg Ser Gly Asn Tyr Lys Pro Cys Met Trp Asp Asn
       50                  55                  60
   Asp Phe Leu Gln Ser Leu Lys Thr Glu Tyr Thr Gly Glu Ala Ile Asn
   65                  70                  75                  80
   Ala Arg Ala Ser Glu Met Lys Glu Glu Val Arg Met Ile Phe Asn Asn
                   85                  90                  95
   Val Val Glu Pro Leu Asn Gln Leu Glu Leu Ile Asp Gln Leu Gln Arg
               100                 105                 110
   Leu Gly Leu Asp Tyr His Phe Arg Asp Glu Ile Asn His Thr Leu Lys
           115                 120                 125
   Asn Val His Asn Gly Gln Lys Ser Glu Thr Trp Glu Lys Asp Leu His
       130                 135                 140
   Ala Thr Ala Leu Glu Phe Arg Leu Leu Arg Gln His Gly His Tyr Ile
   145                 150                 155                 160
   Ser Pro Glu Gly Phe Lys Arg Phe Thr Glu Asn Gly Ser Phe Asn Lys
                   165                 170                 175
   Gly Ile Arg Ala Asp Val Arg Gly Leu Leu Ser Leu Tyr Glu Ala Ser
               180                 185                 190
   Tyr Phe Ser Ile Glu Gly Glu Ser Leu Met Glu Glu Ala Trp Ser Phe
           195                 200                 205
   Thr Ser Asn Ile Leu Lys Glu Cys Leu Glu Asn Thr Ile Asp Leu Asp
       210                 215                 220
   Leu Gln Met Gln Val Arg His Ala Leu Glu Leu Pro Leu Gln Trp Arg
   225                 230                 235                 240
   Ile Pro Arg Phe Asp Ala Lys Trp Tyr Ile Asn Leu Tyr Gln Arg Ser
                   245                 250                 255
   Gly Asp Met Ile Pro Ala Val Leu Glu Phe Ala Lys Leu Asp Phe Asn
               260                 265                 270
   Ile Arg Gln Ala Leu Asn Gln Glu Glu Leu Lys Asp Leu Ser Arg Trp
           275                 280                 285
   Trp Ser Arg Leu Asp Met Gly Glu Lys Leu Pro Phe Ala Arg Asp Arg
       290                 295                 300
   Leu Val Thr Ser Phe Phe Trp Ser Leu Gly Ile Thr Gly Glu Pro His
   305                 310                 315                 320
   His Arg Tyr Cys Arg Glu Val Leu Thr Lys Ile Ile Glu Phe Val Gly
                   325                 330                 335
   Val Tyr Asp Asp Val Tyr Asp Val Tyr Gly Thr Leu Asp Glu Leu Glu
               340                 345                 350
   Leu Phe Thr Asn Val Val Lys Arg Trp Asp Thr Asn Ala Met Lys Glu
           355                 360                 365
   Leu Pro Asp Tyr Met Lys Leu Cys Phe Leu Ser Leu Ile Asn Met Val
       370                 375                 380
   Asn Glu Thr Thr Tyr Asp Ile Leu Lys Asp His Asn Ile Asp Thr Leu
   385                 390                 395                 400
   Pro His Gln Arg Lys Trp Phe Asn Asp Leu Phe Glu Arg Tyr Ile Val
                   405                 410                 415
   Glu Ala Arg Trp Tyr Asn Ser Gly Tyr Gln Pro Thr Leu Glu Glu Tyr
               420                 425                 430
   Leu Lys Asn Gly Phe Val Ser Ile Gly Gly Pro Ile Gly Val Leu Tyr
           435                 440                 445
   Ser Tyr Ile Cys Thr Glu Asp Pro Ile Lys Lys Glu Asp Leu Glu Phe
       450                 455                 460
   Ile Glu Asp Leu Pro Asp Ile Val Arg Leu Thr Cys Glu Ile Phe Arg
   465                 470                 475                 480
   Leu Thr Asp Asp Tyr Gly Thr Ser Ser Ala Glu Leu Lys Arg Gly Asp
                   485                 490                 495
   Val Pro Ser Ser Ile Tyr Cys Tyr Met Ser Asp Thr Gly Val Thr Glu
               500                 505                 510
   Glu Val Ser Arg Lys His Met Met Asn Leu Ile Arg Lys Lys Trp Ala
           515                 520                 525
   Gln Ile Asn Lys Leu Arg Phe Ser Lys Glu Tyr Asn Asn Pro Leu Ser
       530                 535                 540
   Trp Ser Phe Val Asp Ile Met Leu Asn Ile Ile Arg Ala Ala His Phe
   545                 550                 555                 560
   Leu Tyr Asn Thr Gly Asp Asp Gly Phe Gly Val Glu Asp Val Ala Val
                   565                 570                 575
   Glu Ala Thr Leu Val Ser Leu Leu Val Glu Pro Ile Pro Leu
               580                 585                 590
<210> 79
   <211> 1773
   <212> DNA
   <213> Coriandrum sativum
<400> 79
   atggcagcga taactatatt tccactttct tattcgatca aatttaggag atcctcccca      60
   tgcaatccta aagatgtgac agcctgcaag tctgtaatta aatccgtcac tggaatgact     120
   aaggttcctg ttccagtacc agagcctatc gtaaggcgat cagggaacta caaaccttgc     180
   atgtgggaca acgatttctt gcagtctttg aaaactgaat acaccgggga agcaatcaat     240
   gcacgagctt ctgagatgaa ggaagaggtg aggatgatat ttaataatgt ggtcgaacca     300
   ttgaatcagc ttgagctgat tgatcagttg cagagacttg ggttggatta tcattttcgt     360
   gatgaaatca accatacttt gaagaacgta cataatggtc agaagagtga gacttgggag     420
   aaggacttgc atgctactgc tcttgaattt aggcttctta gacaacatgg acattatata     480
   tcccctgagg gcttcaagag atttacagag aatgggagct tcaataaagg tatccgtgca     540
   gatgtccggg gactattaag tttatatgaa gcctcgtact tttctattga aggagagtcc     600
   ctgatggagg aggcttggtc ctttacaagt aacatcctta aagagtgcct cgaaaatact     660
   attgatttgg atctccagat gcaagtgaga catgctttgg aacttccact acaatggagg     720
   atcccgagat ttgatgcaaa gtggtacata aatttgtatc aaagaagtgg tgacatgatc     780
   ccagcggttc tggaatttgc aaagttggac ttcaacatta ggcaagcgtt gaaccaagaa     840
   gagcttaaag atttgtcgag gtggtggagt agattagaca tgggagagaa acttcccttt     900
   gccagagata ggttggtaac atcatttttc tggagtttgg ggattactgg cgagcctcat     960
   cacagatatt gcagagaggt tttaaccaaa ataatagagt ttgttggtgt atacgatgat    1020
   gtttatgatg tatatggtac acttgatgaa cttgaactct ttacaaatgt cgtgaagagg    1080
   tgggatacaa atgcaatgaa agagctccca gactacatga agttgtgctt cctgtcattg    1140
   atcaacatgg tcaatgaaac gacttacgac atcctcaagg accataacat cgatacttta    1200
   ccacaccaaa gaaaatggtt caatgattta ttcgagcgtt acatagtgga ggcgaggtgg    1260
   tataacagtg gataccagcc aacactagaa gaatacttga aaaatggatt tgtgtcaata    1320
   ggaggcccca ttggagtgct ttactcttac atctgtactg aggatccaat caagaaagaa    1380
   gatttagagt ttatcgagga ccttcctgat atagtacgat tgacatgtga aatttttcgg    1440
   ttaactgatg attatggaac atcttcggct gagttaaaga gaggagatgt tccatcttct    1500
   atatattgct acatgtcgga tactggtgtt acggaagaag tttcccgtaa gcacatgatg    1560
   aacttgatca ggaagaagtg ggcacaaatt aacaaactca gattttcaaa ggagtataat    1620
   aatcctttat cgtggtcttt tgttgatatt atgttgaata taatcagggc agcccatttt    1680
   ttgtataata ctggagacga tggctttggt gttgaagatg ttgcagttga agctacatta    1740
   gtttcgcttc ttgtcgagcc cattcctctc taa                                 1773
<210> 80
   <211> 1659
   <212> DNA
   <213> Artificial Sequence
<220>
   <223> DNA having modified codons, which encodes linalool synthase gene derived from Coriandrum sativum (opt_CsLINS)
<400> 80
   atgactaaag tgcccgtgcc cgtgcccgaa cccattgtgc ggcggagcgg taactataaa      60
   ccctgtatgt gggataacga ttttctgcaa agcttaaaaa ccgaatatac tggcgaagcc     120
   attaatgccc gtgcttctga aatgaaagaa gaagtgcgga tgatttttaa caacgtggtt     180
   gaacccctga accaattgga actgattgat caactgcagc gcctggggtt ggactaccat     240
   tttcgtgatg aaattaacca taccttgaaa aacgtgcaca acggacaaaa atccgaaacc     300
   tgggaaaaag atttacacgc cactgctctg gaatttcgtt tgttacggca gcatggccac     360
   tatattagcc ccgaaggttt taaacggttt accgaaaatg ggtcttttaa caaaggcatt     420
   cgggccgatg tgcggggcct gttgtccctg tacgaagcta gctacttttc tattgaaggt     480
   gaaagtttga tggaagaagc ctggtccttt actagtaaca ttttgaaaga atgtctggaa     540
   aacaccattg atttagacct gcaaatgcag gtgcgccatg ccttggaatt acccctgcaa     600
   tggcgcattc cccgttttga tgctaaatgg tacattaacc tgtaccagcg cagtggggac     660
   atgattcccg ccgtgttgga atttgctaaa ctggatttta acattcgtca agccttgaac     720
   caggaagaat taaaagacct gagccgctgg tggtctcgtc tggatatggg cgaaaaattg     780
   ccctttgctc gggatcgctt ggtgacttcc tttttctgga gtttaggcat taccggtgaa     840
   ccccatcacc ggtactgtcg cgaagttctg accaaaatta ttgaatttgt gggggtttac     900
   gatgacgtgt atgacgttta cggaaccttg gatgaattgg aactgtttac taacgtggtt     960
   aaacgttggg acaccaacgc catgaaagaa ttacccgatt atatgaaact gtgctttctg    1020
   tccttgatta atatggtgaa cgaaaccact tacgatattc tgaaagacca taacattgat    1080
   accttgcccc accaacgcaa atggtttaac gatctgtttg aacggtacat tgtggaagcc    1140
   cgctggtata atagtggtta ccagcccacc ctggaagaat acttgaaaaa tgggtttgtg    1200
   tccattggcg gtcccattgg agttttgtac agttacattt gtactgaaga ccccatcaaa    1260
   aaagaagatt tggaatttat tgaagattta cccgacattg tgcgtctgac ctgcgaaatt 1320
   tttcggctga ccgatgacta tggcacttcc agtgccgaat tgaaacgggg tgacgttccc 1380
   agctctattt attgctacat gagcgatacc ggtgtgactg aagaagtttc tcggaaacat 1440
   atgatgaacc tgattcgcaa aaaatgggcc caaattaaca aactgcggtt tagcaaagaa 1500
   tataataacc ccttgtcctg gagttttgtg gatattatgc tgaacattat tcgcgccgct 1560
   cattttctgt acaacactgg ggatgacggg tttggagttg aagatgtggc cgttgaagct 1620
   accttagtga gtttactggt tgaacccatt cccttataa 1659
<210> 81
   <211> 900
   <212> DNA
   <213> Escherichia coli
<400> 81
   atggactttc cgcagcaact cgaagcctgc gttaagcagg ccaaccaggc gctgagccgt 60
   tttatcgccc cactgccctt tcagaacact cccgtggtcg aaaccatgca gtatggcgca 120
   ttattaggtg gtaagcgcct gcgacctttc ctggtttatg ccaccggtca tatgttcggc 180
   gttagcacaa acacgctgga cgcacccgct gccgccgttg agtgtatcca cgcttactca 240
   ttaattcatg atgatttacc ggcaatggat gatgacgatc tgcgtcgcgg tttgccaacc 300
   tgccatgtga agtttggcga agcaaacgcg attctcgctg gcgacgcttt acaaacgctg 360
   gcgttctcga ttttaagcga tgccgatatg ccggaagtgt cggaccgcga cagaatttcg 420
   atgatttctg aactggcgag cgccagtggt attgccggaa tgtgcggtgg tcaggcatta 480
   gatttagacg cggaaggcaa acacgtacct ctggacgcgc ttgagcgtat tcatcgtcat 540
   aaaaccggcg cattgattcg cgccgccgtt cgccttggtg cattaagcgc cggagataaa 600
   ggacgtcgtg ctctgccggt actcgacaag tatgcagaga gcatcggcct tgccttccag 660
   gttcaggatg acatcctgga tgtggtggga gatactgcaa cgttgggaaa acgccagggt 720
   gccgaccagc aacttggtaa aagtacctac cctgcacttc tgggtcttga gcaagcccgg 780
   aagaaagccc gggatctgat cgacgatgcc cgtcagtcgc tgaaacaact ggctgaacag 840
   tcactcgata cctcggcact ggaagcgcta gcggactaca tcatccagcg taataaataa 900
<210> 82
   <211> 900
   <212> DNA
   <213> Artificial Sequence
<220>
   <223> DNA having modified codons, which encodes farnesyl diphosphate synthase gene derived from Escherichia coli (ispA gene)
<400> 82
   atggattttc cccagcagct ggaagcctgc gtgaaacagg ccaaccaggc cctgagccgc      60
   tttatcgccc ccctgccctt tcagaacacc cccgtggtgg aaaccatgca gtacggcgcc     120
   ctgctgggcg gcaaacgcct gcgccccttt ctggtgtacg ccaccggcca catgtttggc     180
   gtgagcacca acaccctgga tgcccccgcc gccgccgtgg aatgcatcca cgcctacttt     240
   ctgatccacg atgatctgcc cgccatggat gatgatgatc tgcgccgcgg cctgcccacc     300
   tgccacgtga aatttggcga agccaacgcc atcctggccg gcgatgccct gcagaccctg     360
   gcctttagca tcctgagcga tgccgatatg cccgaagtga gcgatcgcga tcgcatcagc     420
   atgatcagcg aactggccag cgccagcggc atcgccggca tgtgcggcgg ccaggccctg     480
   gatctggatg ccgaaggcaa acacgtgccc ctggatgccc tggaacgcat ccaccgccac     540
   aaaaccggcg ccctgatccg cgccgccgtg cgcctgggcg ccctgagcgc cggcgataaa     600
   ggccgccgcg ccctgcccgt gctggataaa tacgccgaaa gcatcggcct ggcctttcag     660
   gtgcaggatg atatcctgga tgtggtgggc gataccgcca ccctgggcaa acgccagggc     720
   gccgatcagc agctgggcaa aagcacctac cccgccctgc tgggcctgga acaggcccgc     780
   aaaaaagccc gcgatctgat cgatgatgcc cgccagagcc tgaaacagct ggccgaacag     840
   agcctggata ccagcgccct ggaagccctg gccgattaca tcatccagcg caacaaatag     900
<210> 83
   <211> 37
   <212> DNA
   <213> Artificial Sequence
<220>
   <223> Primer
<400> 83
   acgttgttgc cattgccctg ttgacaatta atcatcg 37
<210> 84
   <211> 37
   <212> DNA
   <213> Artificial Sequence
<220>
   <223> Primer
<400> 84
   atgacttggt tgagtttagc tactggaatc atacaac 37
<210> 85
   <211> 29
   <212> DNA
   <213> Artificial Sequence
<220>
   <223> Primer
<400> 85
   tgtgaaatta gctactggaa tcatacaac 29
<210> 86
   <211> 45
   <212> DNA
   <213> Artificial Sequence
<220>
   <223> Primer
<400> 86
   gtagctaatt tcacacagga gactgccatg gattttcccc agcag 45
<210> 87
   <211> 37
   <212> DNA
   <213> Artificial Sequence
<220>
   <223> Primer
<400> 87
   atgacttggt tgagtctatt tgttgcgctg gatgatg 37
<210> 88
   <211> 28
   <212> DNA
   <213> Artificial Sequence
<220>
   <223> Primer
<400> 88
   tgtgaaatta taagggaatg ggttcaac 28
<210> 89
   <211> 45
   <212> DNA
   <213> Artificial Sequence
<220>
   <223> Primer
<400> 89
   ccttataatt tcacacagga gactgccatg gattttcccc agcag 45
<210> 90
   <211> 634
   <212> PRT
   <213> Picea sitchensis
<400> 90
   Met Ser Pro Val Ser Ala Ile Pro Leu Ala Tyr Lys Leu Cys Leu Pro
   1               5                   10                  15
   Arg Ser Leu Ile Ser Ser Ser Arg Glu Leu Asn Pro Leu His Ile Thr
               20                  25                  30
   Ile Pro Asn Leu Gly Met Cys Arg Arg Gly Lys Ser Met Ala Pro Ala
           35                  40                  45
   Ser Met Ser Met Ile Leu Thr Ala Ala Val Ser Asp Asp Asp Arg Val
       50                  55                  60
   Gln Arg Arg Arg Gly Asn Tyr His Ser Asn Leu Trp Asp Asp Asp Phe
   65                  70                  75                  80
   Ile Gln Ser Leu Ser Thr Pro Tyr Gly Glu Pro Ser Tyr Arg Glu Ser
                   85                  90                  95
   Ala Glu Arg Leu Lys Gly Glu Ile Lys Lys Met Phe Arg Ser Met Ser
               100                 105                 110
   Lys Glu Asp Glu Glu Leu Ile Thr Pro Leu Asn Asp Leu Ile Gln Arg
           115                 120                 125
   Leu Trp Met Val Asp Ser Val Glu Arg Leu Gly Ile Asp Arg His Phe
       130                 135                 140
   Lys Asn Glu Ile Lys Ser Ala Leu Asp Tyr Val Tyr Ser Tyr Trp Asn
   145                 150                 155                 160
   Glu Lys Gly Ile Gly Cys Gly Arg Asp Ser Val Val Ala Asp Leu Asn
                   165                 170                 175
   Ser Thr Ala Leu Gly Phe Arg Thr Leu Arg Leu His Gly Tyr Asn Val
               180                 185                 190
   Ser Ser Glu Val Leu Lys Val Phe Glu Asp Gln Asn Gly Gln Phe Ala
           195                 200                 205
   Cys Ser Pro Ser Lys Thr Glu Gly Glu Ile Arg Ser Ala Leu Asn Leu
       210                 215                 220
   Tyr Arg Ala Ser Leu Ile Ala Phe Pro Gly Glu Lys Val Met Glu Asp
   225                 230                 235                 240
   Ala Glu Ile Phe Ser Ser Arg Tyr Leu Lys Glu Ala Val Gln Lys Ile
                   245                 250                 255
   Pro Asp Cys Ser Leu Ser Gln Glu Ile Ala Tyr Ala Leu Glu Tyr Gly
               260                 265                 270
   Trp His Thr Asn Met Pro Arg Leu Glu Ala Arg Asn Tyr Met Asp Val
   275 280 285
   Phe Gly His Pro Ser Ser Pro Trp Leu Lys Lys Asn Lys Thr Gln Tyr
   290 295 300
   Met Asp Gly Glu Lys Leu Leu Glu Leu Ala Lys Leu Glu Phe Asn Ile
   305 310 315 320
   Phe His Ser Leu Gln Gln Glu Glu Leu Gln Tyr Ile Ser Arg Trp Trp
   325 330 335
   Lys Asp Ser Gly Leu Pro Lys Leu Ala Phe Ser Arg His Arg His Val
   340 345 350
   Glu Tyr Tyr Thr Leu Gly Ser Cys Ile Ala Thr Asp Pro Lys His Arg
   355 360 365
   Ala Phe Arg Leu Gly Phe Val Lys Thr Cys His Leu Asn Thr Val Leu
   370 375 380
   Asp Asp Ile Tyr Asp Thr Phe Gly Thr Met Asp Glu Ile Glu Leu Phe
   385 390 395 400
   Thr Glu Ala Val Arg Arg Trp Asp Pro Ser Glu Thr Glu Ser Leu Pro
   405 410 415
   Asp Tyr Met Lys Gly Val Tyr Met Val Leu Tyr Glu Ala Leu Thr Glu
   420 425 430
   Met Ala Gln Glu Ala Glu Lys Thr Gln Gly Arg Asp Thr Leu Asn Tyr
   435 440 445
   Ala Arg Lys Ala Trp Glu Ile Tyr Leu Asp Ser Tyr Ile Gln Glu Ala
   450 455 460
   Lys Trp Ile Ala Ser Gly Tyr Leu Pro Thr Phe Gln Glu Tyr Phe Glu
   465 470 475 480
   Asn Gly Lys Ile Ser Ser Ala Tyr Arg Ala Ala Ala Leu Thr Pro Ile
   485 490 495
   Leu Thr Leu Asp Val Pro Leu Pro Glu Tyr Ile Leu Lys Gly Ile Asp
   500 505 510
   Phe Pro Ser Arg Phe Asn Asp Leu Ala Ser Ser Phe Leu Arg Leu Arg
   515 520 525
   Gly Asp Thr Arg Cys Tyr Lys Ala Asp Arg Ala Arg Gly Glu Glu Ala
       530                 535                 540
   Ser Cys Ile Ser Cys Tyr Met Lys Asp Asn Pro Gly Ser Thr Glu Glu
   545                 550                 555                 560
   Asp Ala Leu Asn His Ile Asn Ser Met Ile Asn Glu Ile Ile Lys Glu
                   565                 570                 575
   Leu Asn Trp Glu Leu Leu Arg Pro Asp Ser Asn Ile Pro Met Pro Ala
               580                 585                 590
   Arg Lys His Ala Phe Asp Ile Thr Arg Ala Leu His His Leu Tyr Lys
           595                 600                 605
   Tyr Arg Asp Gly Phe Ser Val Ala Thr Lys Glu Thr Lys Ser Leu Val
       610                 615                 620
   Ser Arg Met Val Leu Glu Pro Val Thr Leu
   625                 630
<210> 91
   <211> 1905
   <212> DNA
   <213> Picea sitchensis
<400> 91
   atgtctcctg tttctgccat accgttggct tacaaattgt gcctgcccag atcgttgatc     60
   agttctagtc gtgagcttaa tcctctccat ataacaatcc caaatcttgg aatgtgcagg    120
   cgagggaaat caatggcacc agcttccatg agcatgattt tgaccgccgc cgtctctgat    180
   gatgaccgtg tacaaagacg cagaggcaat tatcactcga acctctggga cgatgatttc    240
   atacagtctc tttcaacgcc ttatggggaa ccttcttatc gggaaagtgc tgagagactt    300
   aaaggggaaa taaagaagat gttcagatca atgtcaaagg aggatgaaga attaattact    360
   cccctcaatg atctcattca acgactttgg atggtcgata gcgtcgaacg tttggggatc    420
   gatagacatt tcaaaaatga gataaaatca gcgctggatt atgtttacag ttattggaat    480
   gaaaaaggca ttggatgtgg gagagatagt gttgttgctg atctcaactc cactgccttg    540
   gggtttcgaa ctcttcgcct acacggatac aatgtctcct cagaggtttt gaaagttttt    600
   gaagaccaaa acggacagtt tgcatgctct cccagtaaaa cagaagggga gatcagaagc    660
   gctcttaact tatatcgggc ttccctcatt gcctttcctg gggagaaagt tatggaagac    720
   gctgaaatct tctcttcaag atatttgaaa gaagccgtgc aaaagattcc ggactgcagt    780
   ctttcacaag agatagccta tgctttggaa tatggttggc acacaaatat gccaagattg    840
   gaagcaagga attacatgga cgtatttgga catcctagta gcccatggct caagaagaat     900
   aagacgcaat atatggacgg cgagaaactt ttagaactag caaaattgga gttcaatatc     960
   tttcactcct tgcaacagga ggagttacaa tatatctcca gatggtggaa agattcgggt    1020
   ttgcctaaac tggccttcag tcggcatcgt cacgtggaat actacacttt ggggtcttgc    1080
   attgcgactg accccaaaca tcgtgcattc agactgggct ttgtcaaaac gtgtcatctt    1140
   aacacggttc tggacgatat ctacgacaca ttcggaacga tggacgaaat cgaactcttc    1200
   acagaagcag tcaggagatg ggatccgtcg gagacagaga gccttccaga ctatatgaaa    1260
   ggagtgtaca tggtactcta cgaagcccta actgaaatgg ctcaagaggc ggagaaaaca    1320
   caaggccgag acacgctcaa ctatgctcga aaggcttggg agatttatct tgattcgtat    1380
   attcaagaag caaagtggat cgccagtggt tatctgccaa catttcagga atactttgag    1440
   aacgggaaaa ttagctctgc ttatcgcgca gcggcattga cacccatcct cacattggac    1500
   gtaccgcttc ctgaatacat cttgaaggga attgattttc catcgagatt caatgatttg    1560
   gcatcttcct tccttcgact aagaggtgac acacgctgct acaaggcgga tagggcccgt    1620
   ggagaagaag cttcgtgcat atcttgttat atgaaagaca atcctggatc aacggaggaa    1680
   gatgctctca atcatatcaa ctccatgatc aatgaaataa tcaaagaatt aaattgggaa    1740
   ttactaagac ctgatagcaa tattccaatg cctgcgagga aacatgcttt tgacataact    1800
   agagctctcc accacctcta taaataccga gatgggttca gcgttgccac taaggaaacg    1860
   aaaagtctgg tcagcagaat ggtccttgaa cctgtgactt tgtaa                    1905
<210> 92
   <211> 1716
   <212> DNA
   <213> Artificial Sequence
<220>
   <223> DNA having modified codons, which encodes limonene synthase gene derived from Picea sitchensis (opt_PsLMS)
<400> 92
   atgcagcgcc gtcgcggcaa ttaccacagc aacctgtggg acgatgactt tatccagagt 60
   ctgagcaccc cgtatggtga acccagttac cgtgaaagcg cggagcgcct gaaaggcgag 120
   attaaaaaga tgtttcgcag tatgagcaag gaagatgaag agctgatcac gccgctgaat 180
   gacctgattc agcgcctgtg gatggtcgat agcgttgagc gtctgggcat cgaccgccat 240
   tttaaaaatg aaattaagag tgccctggat tacgtctata gctactggaa cgaaaaaggc 300
   atcggttgtg gccgcgatag tgttgtggcg gacctgaata gcaccgccct gggttttcgt 360
   acgctgcgcc tgcacggcta caatgttagt agcgaggtgc tgaaagtctt tgaagatcag 420
   aacggccagt ttgcgtgcag tccgagcaag accgaaggcg agatccgtag tgccctgaac 480
   ctgtatcgcg cgagcctgat tgcctttccc ggtgaaaaag ttatggaaga cgcggagatt 540
   tttagtagcc gctacctgaa agaagccgtg cagaagatcc ccgattgtag tctgagccag 600
   gagattgcgt atgccctgga atacggctgg cataccaata tgcctcgtct ggaagcccgc 660
   aactatatgg acgtttttgg tcaccccagt agcccttggc tgaaaaagaa taagacgcag 720
   tacatggatg gcgaaaaact gctggagctg gcgaagctgg aatttaacat ctttcatagc 780
   ctgcagcagg aagagctgca gtacattagt cgttggtgga aagacagcgg cctgcctaag 840
   ctggccttta gccgtcatcg ccacgtggaa tactataccc tgggtagctg tatcgcgacg 900
   gatccgaaac atcgtgcctt tcgcctgggc tttgtgaaga cctgccacct gaatacggtc 960
   ctggatgaca tctatgatac ctttggcacg atggacgaaa ttgagctgtt taccgaagcg 1020
   gtccgtcgct gggatccgag tgaaacggag agcctgcccg actacatgaa aggtgtttat 1080
   atggtgctgt acgaagccct gaccgagatg gcgcaggaag ccgagaaaac ccagggccgt 1140
   gacacgctga attatgcgcg caaggcctgg gagatttatc tggatagtta catccaggaa 1200
   gcgaaatgga ttgccagcgg ttatctgccg acgtttcagg aatactttga gaacggcaag 1260
   atcagtagcg cctatcgtgc ggccgccctg acccctattc tgaccctgga tgtgccgctg 1320
   cccgaataca ttctgaaagg catcgatttt ccgagccgtt ttaatgacct ggccagtagc 1380
   tttctgcgtc tgcgcggtga tacccgctgc tataaggccg accgtgcccg cggcgaagag 1440
   gcgagttgta ttagctgcta catgaaggat aatcccggca gtacggaaga ggacgccctg 1500
   aaccatatca acagcatgat caacgaaatc atcaaggagc tgaactggga actgctgcgc 1560
   cctgatagca acatccctat gccggcgcgt aaacacgcct ttgacattac ccgcgcgctg 1620
   catcacctgt ataagtaccg tgatggcttt agcgttgcca ccaaagaaac gaagagtctg 1680
   gtcagccgca tggttctgga acccgtgacg ctgtaa 1716
<210> 93
   <211> 637
   <212> PRT
   <213> Abies grandis
<400> 93
   Met Ala Leu Leu Ser Ile Val Ser Leu Gln Val Pro Lys Ser Cys Gly
   1               5                   10                  15
   Leu Lys Ser Leu Ile Ser Ser Ser Asn Val Gln Lys Ala Leu Cys Ile
               20                  25                  30
   Ser Thr Ala Val Pro Thr Leu Arg Met Arg Arg Arg Gln Lys Ala Leu
           35                  40                  45
   Val Ile Asn Met Lys Leu Thr Thr Val Ser His Arg Asp Asp Asn Gly
       50                  55                  60
   Gly Gly Val Leu Gln Arg Arg Ile Ala Asp His His Pro Asn Leu Trp
   65                  70                  75                  80
   Glu Asp Asp Phe Ile Gln Ser Leu Ser Ser Pro Tyr Gly Gly Ser Ser
                   85                  90                  95
   Tyr Ser Glu Arg Ala Glu Thr Val Val Glu Glu Val Lys Glu Met Phe
               100                 105                 110
   Asn Ser Ile Pro Asn Asn Arg Glu Leu Phe Gly Ser Gln Asn Asp Leu
           115                 120                 125
   Leu Thr Arg Leu Trp Met Val Asp Ser Ile Glu Arg Leu Gly Ile Asp
       130                 135                 140
   Arg His Phe Gln Asn Glu Ile Arg Val Ala Leu Asp Tyr Val Tyr Ser
   145                 150                 155                 160
   Tyr Trp Lys Glu Lys Glu Gly Ile Gly Cys Gly Arg Asp Ser Thr Phe
                   165                 170                 175
   Pro Asp Leu Asn Ser Thr Ala Leu Ala Leu Arg Thr Leu Arg Leu His
               180                 185                 190
   Gly Tyr Asn Val Ser Ser Asp Val Leu Glu Tyr Phe Lys Asp Glu Lys
           195                 200                 205
   Gly His Phe Ala Cys Pro Ala Ile Leu Thr Glu Gly Gln Ile Thr Arg
       210                 215                 220
   Ser Val Leu Asn Leu Tyr Arg Ala Ser Leu Val Ala Phe Pro Gly Glu
   225                 230                 235                 240
   Lys Val Met Glu Glu Ala Glu Ile Phe Ser Ala Ser Tyr Leu Lys Lys
                   245                 250                 255
   Val Leu Gln Lys Ile Pro Val Ser Asn Leu Ser Gly Glu Ile Glu Tyr
               260                 265                 270
   Val Leu Glu Tyr Gly Trp His Thr Asn Leu Pro Arg Leu Glu Ala Arg
           275                 280                 285
   Asn Tyr Ile Glu Val Tyr Glu Gln Ser Gly Tyr Glu Ser Leu Asn Glu
       290                 295                 300
   Met Pro Tyr Met Asn Met Lys Lys Leu Leu Gln Leu Ala Lys Leu Glu
   305 310 315 320
   Phe Asn Ile Phe His Ser Leu Gln Leu Arg Glu Leu Gln Ser Ile Ser
   325 330 335
   Arg Trp Trp Lys Glu Ser Gly Ser Ser Gln Leu Thr Phe Thr Arg His
   340 345 350
   Arg His Val Glu Tyr Tyr Thr Met Ala Ser Cys Ile Ser Met Leu Pro
   355 360 365
   Lys His Ser Ala Phe Arg Met Glu Phe Val Lys Val Cys His Leu Val
   370 375 380
   Thr Val Leu Asp Asp Ile Tyr Asp Thr Phe Gly Thr Met Asn Glu Leu
   385 390 395 400
   Gln Leu Phe Thr Asp Ala Ile Lys Arg Trp Asp Leu Ser Thr Thr Arg
   405 410 415
   Trp Leu Pro Glu Tyr Met Lys Gly Val Tyr Met Asp Leu Tyr Gln Cys
   420 425 430
   Ile Asn Glu Met Val Glu Glu Ala Glu Lys Thr Gln Gly Arg Asp Met
   435 440 445
   Leu Asn Tyr Ile Gln Asn Ala Trp Glu Ala Leu Phe Asp Thr Phe Met
   450 455 460
   Gln Glu Ala Lys Trp Ile Ser Ser Ser Tyr Leu Pro Thr Phe Glu Glu
   465 470 475 480
   Tyr Leu Lys Asn Ala Lys Val Ser Ser Gly Ser Arg Ile Ala Thr Leu
   485 490 495
   Gln Pro Ile Leu Thr Leu Asp Val Pro Leu Pro Asp Tyr Ile Leu Gln
   500 505 510
   Glu Ile Asp Tyr Pro Ser Arg Phe Asn Glu Leu Ala Ser Ser Ile Leu
   515 520 525
   Arg Leu Arg Gly Asp Thr Arg Cys Tyr Lys Ala Asp Arg Ala Arg Gly
   530 535 540
   Glu Glu Ala Ser Ala Ile Ser Cys Tyr Met Lys Asp His Pro Gly Ser
   545 550 555 560
   Ile Glu Glu Asp Ala Leu Asn His Ile Asn Ala Met Ile Ser Asp Ala
   565 570 575
   Ile Arg Glu Leu Asn Trp Glu Leu Leu Arg Pro Asp Ser Lys Ser Pro
   580 585 590
   Ile Ser Ser Lys Lys His Ala Phe Asp Ile Thr Arg Ala Phe His His
   595 600 605
   Val Tyr Lys Tyr Arg Asp Gly Tyr Thr Val Ser Asn Asn Glu Thr Lys
   610 615 620
   Asn Leu Val Met Lys Thr Val Leu Glu Pro Leu Ala Leu
   625 630 635
<210> 94
   <211> 1914
   <212> DNA
   <213> Abies grandis
<400> 94
   atggctctcc tttctatcgt atctttgcag gttcccaaat cctgcgggct gaaatcgttg 60
   atcagttcca gcaatgtgca gaaggctctc tgtatctcta cagcagtccc aacactcaga 120
   atgcgtaggc gacagaaagc tctggtcatc aacatgaaat tgaccactgt atcccatcgt 180
   gatgataatg gtggtggtgt actgcaaaga cgcatagccg atcatcatcc caacctgtgg 240
   gaagatgatt tcatacaatc attgtcctca ccttatgggg gatcttcgta cagtgaacgt 300
   gctgagacag tcgttgagga agtaaaagag atgttcaatt caataccaaa taatagagaa 360
   ttatttggtt cccaaaatga tctccttaca cgcctttgga tggtggatag cattgaacgt 420
   ctggggatag atagacattt ccaaaatgag ataagagtag ccctcgatta tgtttacagt 480
   tattggaagg aaaaggaagg cattgggtgt ggcagagatt ctacttttcc tgatctcaac 540
   tcgactgcct tggcgcttcg aactcttcga ctgcacggat acaatgtgtc ttcagatgtg 600
   ctggaatact tcaaagatga aaaggggcat tttgcctgcc ctgcaatcct aaccgaggga 660
   cagatcacta gaagtgttct aaatttatat cgggcttccc tggtcgcctt tcccggggag 720
   aaagttatgg aagaggctga aatcttctcg gcatcttatt tgaaaaaagt cttacaaaag 780
   attccggtct ccaatctttc aggagagata gaatatgttt tggaatatgg ttggcacacg 840
   aatttgccga gattggaagc aagaaattat atcgaggtct acgagcagag cggctatgaa 900
   agcttaaacg agatgccata tatgaacatg aagaagcttt tacaacttgc aaaattggag 960
   ttcaatatct ttcactcttt gcaactaaga gagttacaat ctatctccag atggtggaaa 1020
   gaatcaggtt cgtctcaact gacttttaca cggcatcgtc acgtggaata ctacactatg 1080
   gcatcttgca tttctatgtt gccaaaacat tcagctttca gaatggagtt tgtcaaagtg   1140
   tgtcatcttg taacagttct cgatgatata tatgacactt ttggaacaat gaacgaactc   1200
   caacttttta cggatgcaat taagagatgg gatttgtcaa cgacaaggtg gcttccagaa   1260
   tatatgaaag gagtgtacat ggacttgtat caatgcatta atgaaatggt ggaagaggct   1320
   gagaagactc aaggccgaga tatgctcaac tatattcaaa atgcttggga agccctattt   1380
   gataccttta tgcaagaagc aaagtggatc tccagcagtt atctcccaac gtttgaggag   1440
   tacttgaaga atgcaaaagt tagttctggt tctcgcatag ccacattaca acccattctc   1500
   actttggatg taccacttcc tgattacata ctgcaagaaa ttgattatcc atccagattc   1560
   aatgagttag cttcgtccat ccttcgacta cgaggtgaca cgcgctgcta caaggcggat   1620
   agggcccgtg gagaagaagc ttcagctata tcgtgttata tgaaagacca tcctggatca   1680
   atagaggaag atgctctcaa tcatatcaac gccatgatca gtgatgcaat cagagaatta   1740
   aattgggagc ttctcagacc ggatagcaaa agtcccatct cttccaagaa acatgctttt   1800
   gacatcacca gagctttcca tcatgtctac aaatatcgag atggttacac tgtttccaac   1860
   aacgaaacaa agaatttggt gatgaaaacc gttcttgaac ctctcgcttt gtaa         1914
<210> 95
   <211> 1713
   <212> DNA
   <213> Artificial Sequence
<220>
   <223> DNA having modified codons, which encodes limonene synthase gene derived from Abies grandis (opt_AgLMS)
<400> 95
   atgcagcgtc gcatcgcgga tcatcacccc aatctgtggg aagatgactt tattcagagc      60
   ctgagtagcc cttatggtgg cagtagctac agtgaacgcg ccgagaccgt tgtggaagag     120
   gttaaggaaa tgtttaacag catccccaac aaccgtgagc tgtttggcag tcagaacgat     180
   ctgctgacgc gcctgtggat ggtggatagc atcgaacgtc tgggtattga ccgccacttt     240
   cagaatgaaa tccgcgttgc gctggactac gtgtatagct actggaaaga aaaagaaggc     300
   attggctgtg gtcgcgatag cacctttcct gacctgaata gtacggccct ggccctgcgt     360
   accctgcgtc tgcatggcta taacgtcagt agcgatgttc tggaatactt taaagacgag     420
   aagggccact ttgcctgccc cgcgatcctg accgagggtc agattacgcg tagcgttctg     480
   aatctgtatc gcgccagtct ggtggcgttt cctggcgaaa aagtcatgga agaggccgag     540
   atctttagcg cgagttacct gaaaaaggtc ctgcagaaga tccctgttag caacctgagt     600
   ggcgaaattg agtatgtgct ggaatacggt tggcatacca atctgccgcg tctggaagcc     660
   cgcaactata ttgaagtcta cgagcagagc ggctatgaaa gtctgaatga gatgccgtac     720
   atgaacatga aaaagctgct gcagctggcg aaactggaat ttaatatctt tcacagcctg     780
   cagctgcgtg aactgcagag cattagtcgt tggtggaagg agagcggtag tagccagctg     840
   acctttacgc gtcatcgcca cgtggaatac tatacgatgg ccagctgtat cagtatgctg     900
   cctaaacata gcgcgtttcg catggaattt gtcaaggttt gccacctggt gaccgtcctg     960
   gatgacatct acgatacctt tggcacgatg aatgaactgc agctgtttac ggatgccatt    1020
   aaacgttggg acctgagcac cacccgttgg ctgcccgaat acatgaaggg cgtttatatg    1080
   gacctgtacc agtgtattaa cgaaatggtg gaagaggccg agaaaaccca gggtcgcgat    1140
   atgctgaatt acatccagaa cgcctgggaa gcgctgtttg acacctttat gcaggaagcc    1200
   aagtggatta gtagcagtta tctgcccacg tttgaagagt acctgaaaaa tgccaaagtc    1260
   agcagcggta gccgtattgc caccctgcag ccgattctga cgctggatgt tccgctgccc    1320
   gactatattc tgcaggaaat cgattacccc agccgtttta acgagctggc cagcagtatt    1380
   ctgcgtctgc gcggcgatac gcgctgttat aaagcggacc gtgcccgcgg tgaagaggcc    1440
   agcgcgatca gttgctacat gaaggatcat ccgggcagca ttgaagagga cgcgctgaat    1500
   cacattaacg ccatgatcag tgatgcgatt cgtgaactga attgggagct gctgcgccct    1560
   gacagcaaaa gtccgatcag cagtaaaaag catgcctttg atattacccg tgcgtttcat    1620
   cacgtctata agtaccgcga cggctacacc gttagcaata acgaaacgaa aaacctggtg    1680
   atgaagacgg tcctggaacc cctggcgctg taa                                 1713
<210> 96
   <211> 599
   <212> PRT
   <213> Mentha spicata
<400> 96
   Met Ala Leu Lys Val Leu Ser Val Ala Thr Gln Met Ala Ile Pro Ser
   1               5                   10                  15
   Asn Leu Thr Thr Cys Leu Gln Pro Ser His Phe Lys Ser Ser Pro Lys
               20                  25                  30
   Leu Leu Ser Ser Thr Asn Ser Ser Ser Arg Ser Arg Leu Arg Val Tyr
           35                  40                  45
   Cys Ser Ser Ser Gln Leu Thr Thr Glu Arg Arg Ser Gly Asn Tyr Asn
       50                  55                  60
   Pro Ser Arg Trp Asp Val Asn Phe Ile Gln Ser Leu Leu Ser Asp Tyr
   65                  70                  75                  80
   Lys Glu Asp Lys His Val Ile Arg Ala Ser Glu Leu Val Thr Leu Val
                   85                  90                  95
   Lys Met Glu Leu Glu Lys Glu Thr Asp Gln Ile Arg Gln Leu Glu Leu
               100                 105                 110
   Ile Asp Asp Leu Gln Arg Met Gly Leu Ser Asp His Phe Gln Asn Glu
           115                 120                 125
   Phe Lys Glu Ile Leu Ser Ser Ile Tyr Leu Asp His His Tyr Tyr Lys
       130                 135                 140
   Asn Pro Phe Pro Lys Glu Glu Arg Asp Leu Tyr Ser Thr Ser Leu Ala
   145                 150                 155                 160
   Phe Arg Leu Leu Arg Glu His Gly Phe Gln Val Ala Gln Glu Val Phe
                   165                 170                 175
   Asp Ser Phe Lys Asn Glu Glu Gly Glu Phe Lys Glu Ser Leu Ser Asp
               180                 185                 190
   Asp Thr Arg Gly Leu Leu Gln Leu Tyr Glu Ala Ser Phe Leu Leu Thr
           195                 200                 205
   Glu Gly Glu Thr Thr Leu Glu Ser Ala Arg Glu Phe Ala Thr Lys Phe
       210                 215                 220
   Leu Glu Glu Lys Val Asn Glu Gly Gly Val Asp Gly Asp Leu Leu Thr
   225                 230                 235                 240
   Arg Ile Ala Tyr Ser Leu Asp Ile Pro Leu His Trp Arg Ile Lys Arg
                   245                 250                 255
   Pro Asn Ala Pro Val Trp Ile Glu Trp Tyr Arg Lys Arg Pro Asp Met
               260                 265                 270
   Asn Pro Val Val Leu Glu Leu Ala Ile Leu Asp Leu Asn Ile Val Gln
           275                 280                 285
   Ala Gln Phe Gln Glu Glu Leu Lys Glu Ser Phe Arg Trp Trp Arg Asn
       290                 295                 300
   Thr Gly Phe Val Glu Lys Leu Pro Phe Ala Arg Asp Arg Leu Val Glu
   305                 310                 315                 320
   Cys Tyr Phe Trp Asn Thr Gly Ile Ile Glu Pro Arg Gln His Ala Ser
                   325                 330                 335
   Ala Arg Ile Met Met Gly Lys Val Asn Ala Leu Ile Thr Val Ile Asp
               340                 345                 350
   Asp Ile Tyr Asp Val Tyr Gly Thr Leu Glu Glu Leu Glu Gln Phe Thr
           355                 360                 365
   Asp Leu Ile Arg Arg Trp Asp Ile Asn Ser Ile Asp Gln Leu Pro Asp
       370                 375                 380
   Tyr Met Gln Leu Cys Phe Leu Ala Leu Asn Asn Phe Val Asp Asp Thr
   385                 390                 395                 400
   Ser Tyr Asp Val Met Lys Glu Lys Gly Val Asn Val Ile Pro Tyr Leu
                   405                 410                 415
   Arg Gln Ser Trp Val Asp Leu Ala Asp Lys Tyr Met Val Glu Ala Arg
               420                 425                 430
   Trp Phe Tyr Gly Gly His Lys Pro Ser Leu Glu Glu Tyr Leu Glu Asn
           435                 440                 445
   Ser Trp Gln Ser Ile Ser Gly Pro Cys Met Leu Thr His Ile Phe Phe
       450                 455                 460
   Arg Val Thr Asp Ser Phe Thr Lys Glu Thr Val Asp Ser Leu Tyr Lys
   465                 470                 475                 480
   Tyr His Asp Leu Val Arg Trp Ser Ser Phe Val Leu Arg Leu Ala Asp
                   485                 490                 495
   Asp Leu Gly Thr Ser Val Glu Glu Val Ser Arg Gly Asp Val Pro Lys
               500                 505                 510
   Ser Leu Gln Cys Tyr Met Ser Asp Tyr Asn Ala Ser Glu Ala Glu Ala
           515                 520                 525
   Arg Lys His Val Lys Trp Leu Ile Ala Glu Val Trp Lys Lys Met Asn
       530                 535                 540
   Ala Glu Arg Val Ser Lys Asp Ser Pro Phe Gly Lys Asp Phe Ile Gly
   545                 550                 555                 560
   Cys Ala Val Asp Leu Gly Arg Met Ala Gln Leu Met Tyr His Asn Gly
                   565                 570                 575
   Asp Gly His Gly Thr Gln His Pro Ile Ile His Gln Gln Met Thr Arg
               580                 585                 590
   Thr Leu Phe Glu Pro Phe Ala
   595
<210> 97
   <211> 1800
   <212> DNA
   <213> Mentha spicata
<400> 97
   atggctctca aagtgttaag tgttgcaact caaatggcga ttcctagcaa cctaacgaca 60
   tgtcttcaac cctcacactt caaatcttct ccaaaactgt tatctagcac taacagtagt 120
   agtcggtctc gcctccgtgt gtattgctcc tcctcgcaac tcactactga aagacgatcc 180
   ggaaactaca acccttctcg ttgggatgtc aacttcatcc aatcgcttct cagtgactat 240
   aaggaggaca aacacgtgat tagggcttct gagctggtca ctttggtgaa gatggaactg 300
   gagaaagaaa cggatcaaat tcgacaactt gagttgatcg atgacttgca gaggatgggg 360
   ctgtccgatc atttccaaaa tgagttcaaa gaaatcttgt cctctatata tctcgaccat 420
   cactattaca agaacccttt tccaaaagaa gaaagggatc tctactccac atctcttgca 480
   tttaggctcc tcagagaaca tggttttcaa gtcgcacaag aggtattcga tagtttcaag 540
   aacgaggagg gtgagttcaa agaaagcctt agcgacgaca ccagaggatt gttgcaactg 600
   tatgaagctt cctttctgtt gacggaaggc gaaaccacgc tcgagtcagc gagggaattc 660
   gccaccaaat ttttggagga aaaagtgaac gagggtggtg ttgatggcga ccttttaaca 720
   agaatcgcat attctttgga catccctctt cattggagga ttaaaaggcc aaatgcacct 780
   gtgtggatcg aatggtatag gaagaggccc gacatgaatc cagtagtgtt ggagcttgcc 840
   atactcgact taaatattgt tcaagcacaa tttcaagaag agctcaaaga atccttcagg 900
   tggtggagaa atactgggtt tgttgagaag ctgcccttcg caagggatag actggtggaa 960
   tgctactttt ggaatactgg gatcatcgag ccacgtcagc atgcaagtgc aaggataatg 1020
   atgggcaaag tcaacgctct gattacggtg atcgatgata tttatgatgt ctatggcacc 1080
   ttagaagaac tcgaacaatt cactgacctc attcgaagat gggatataaa ctcaatcgac 1140
   caacttcccg attacatgca actgtgcttt cttgcactca acaacttcgt cgatgataca 1200
   tcgtacgatg ttatgaagga gaaaggcgtc aacgttatac cctacctgcg gcaatcgtgg 1260
   gttgatttgg cggataagta tatggtagag gcacggtggt tctacggcgg gcacaaacca 1320
   agtttggaag agtatttgga gaactcatgg cagtcgataa gtgggccctg tatgttaacg 1380
   cacatattct tccgagtaac agattcgttc acaaaggaga ccgtcgacag tttgtacaaa 1440
   taccacgatt tagttcgttg gtcatccttc gttctgcggc ttgctgatga tttgggaacc 1500
   tcggtggaag aggtgagcag aggggatgtg ccgaaatcac ttcagtgcta catgagtgac 1560
   tacaatgcat cggaggcgga ggcgcggaag cacgtgaaat ggctgatagc ggaggtgtgg   1620
   aagaagatga atgcggagag ggtgtcgaag gattctccat tcggcaaaga ttttatagga   1680
   tgtgcagttg atttaggaag gatggcgcag ttgatgtacc ataatggaga tgggcacggc   1740
   acacaacacc ctattataca tcaacaaatg accagaacct tattcgagcc ctttgcatga   1800
<210> 98
   <211> 1635
   <212> DNA
   <213> Artificial Sequence
<220>
   <223> DNA having modified codons, which encodes limonene synthase gene derived from Mentha spicata (opt_MsLMS)
<400> 98
   atggaacgcc gtagcggcaa ttataacccc agtcgttggg atgttaattt tattcagagc     60
   ctgctgagtg attacaaaga ggacaagcac gtgatccgcg cgagcgaact ggttaccctg    120
   gtgaaaatgg aactggagaa ggaaacggat cagatccgtc agctggaact gattgatgac    180
   ctgcagcgca tgggcctgag cgaccatttt cagaatgagt ttaaggaaat cctgagcagt    240
   atctacctgg atcatcacta ctacaagaac ccgtttccca aggaagagcg cgacctgtat    300
   agcacgagtc tggcctttcg tctgctgcgc gagcatggct ttcaggtggc gcaggaagtc    360
   tttgatagct ttaaaaacga agagggtgaa tttaaggaaa gcctgagtga tgacacccgc    420
   ggcctgctgc agctgtacga agccagtttt ctgctgacgg agggtgaaac cacgctggag    480
   agcgcccgcg aatttgcgac caaatttctg gaagagaagg ttaatgaagg cggtgtggat    540
   ggtgacctgc tgacccgtat cgcctacagc ctggatattc cgctgcactg gcgtatcaaa    600
   cgccctaacg cgccggtctg gattgagtgg tatcgtaagc gccccgatat gaatcctgtt    660
   gtgctggaac tggccatcct ggacctgaac attgtgcagg cgcagtttca ggaagagctg    720
   aaagagagct ttcgttggtg gcgcaatacg ggctttgtcg aaaagctgcc gtttgcccgt    780
   gatcgcctgg ttgagtgtta cttttggaat accggtatta tcgaaccccg tcagcatgcc    840
   agcgcgcgca ttatgatggg caaagtcaac gcgctgatta cggttatcga tgacatttac    900
   gacgtttatg gcaccctgga agagctggaa cagtttacgg atctgatccg ccgttgggac    960
   atcaatagca ttgatcagct gcctgactac atgcagctgt gctttctggc gctgaataac   1020
   tttgttgatg acaccagtta cgatgtgatg aaagaaaagg gtgtcaacgt tatcccgtat   1080
   ctgcgtcaga gctgggtgga tctggccgac aaatacatgg tcgaagcgcg ctggttttat   1140
   ggcggtcaca agcccagcct ggaagagtat ctggaaaata gttggcagag cattagtggc   1200
   ccttgtatgc tgacccacat tttctttcgc gttacggata gctttaccaa agaaacggtg   1260
   gatagtctgt acaagtatca tgacctggtg cgttggagca gttttgtcct gcgcctggcc   1320
   gatgacctgg gtacgagcgt cgaagaggtt tcacgcggcg atgtgcccaa aagcctgcag   1380
   tgttacatga gcgactataa tgcgagtgag gccgaagcgc gtaaacacgt caagtggctg   1440
   attgccgagg tttggaaaaa gatgaacgcg gaacgcgtga gcaaagatag tccttttggc   1500
   aaggacttta ttggttgtgc ggttgatctg ggtcgtatgg cgcagctgat gtaccataat   1560
   ggcgacggtc atggcaccca gcaccctatt atccatcagc agatgacccg cacgctgttt   1620
   gaaccgtttg cctaa                                                    1635
<210> 99
   <211> 608
   <212> PRT
   <213> Citrus unshiu
<400> 99
   Met Ser Ser Cys Ile Asn Pro Ser Thr Leu Ala Thr Ser Val Asn Gly
   1               5                   10                  15
   Phe Lys Cys Leu Pro Leu Ala Thr Asn Arg Ala Ala Ile Arg Ile Met
               20                  25                  30
   Ala Lys Asn Lys Pro Val Gln Cys Leu Val Ser Thr Lys Tyr Asp Asn
           35                  40                  45
   Leu Thr Val Asp Arg Arg Ser Ala Asn Tyr Gln Pro Ser Ile Trp Asp
       50                  55                  60
   His Asp Phe Leu Gln Ser Leu Asn Ser Asn Tyr Thr Asp Glu Thr Tyr
   65                  70                  75                  80
   Lys Arg Arg Ala Glu Glu Leu Lys Gly Lys Val Lys Thr Ala Ile Lys
                   85                  90                  95
   Asp Val Thr Glu Pro Leu Asp Gln Leu Glu Leu Ile Asp Asn Leu Gln
               100                 105                 110
   Arg Leu Gly Leu Ala Tyr His Phe Glu Pro Glu Ile Arg Asn Ile Leu
           115                 120                 125
   Arg Asn Ile His Asn His Asn Lys Asp Tyr Asn Trp Arg Lys Glu Asn
       130                 135                 140
   Leu Tyr Ala Thr Ser Leu Glu Phe Arg Leu Leu Arg Gln His Gly Tyr
   145                 150                 155                 160
   Pro Val Ser Gln Glu Val Phe Ser Gly Phe Lys Asp Asp Lys Val Gly
                   165                 170                 175
   Phe Ile Cys Asp Asp Phe Lys Gly Ile Leu Ser Leu His Glu Ala Ser
   180 185 190
   Tyr Tyr Ser Leu Glu Gly Glu Ser Ile Met Glu Glu Ala Trp Gln Phe
   195 200 205
   Thr Ser Lys His Leu Lys Glu Met Met Ile Thr Ser Asn Ser Lys Glu
   210 215 220
   Glu Asp Val Phe Val Ala Glu Gln Ala Lys Arg Ala Leu Glu Leu Pro
   225 230 235 240
   Leu His Trp Lys Lys Val Pro Met Leu Glu Ala Arg Trp Phe Ile His
   245 250 255
   Val Tyr Glu Lys Arg Glu Asp Lys Asn His Leu Leu Leu Glu Leu Ala
   260 265 270
   Lys Leu Glu Phe Asn Thr Leu Gln Ala Ile Tyr Gln Glu Glu Leu Lys
   275 280 285
   Asp Ile Ser Gly Trp Trp Lys Asp Thr Gly Leu Gly Glu Lys Leu Ser
   290 295 300
   Phe Ala Arg Asn Arg Leu Val Ala Ser Phe Leu Trp Ser Met Gly Ile
   305 310 315 320
   Ala Phe Glu Pro Gln Phe Ala Tyr Cys Arg Arg Val Leu Thr Ile Ser
   325 330 335
   Ile Ala Leu Ile Thr Val Ile Asp Asp Ile Tyr Asp Val Tyr Gly Thr
   340 345 350
   Leu Asp Glu Leu Glu Ile Phe Thr Asp Ala Val Ala Arg Trp Asp Ile
   355 360 365
   Asn Tyr Ala Leu Lys His Leu Pro Gly Tyr Met Lys Met Cys Phe Leu
   370 375 380
   Ala Leu Tyr Asn Phe Val Asn Glu Phe Ala Tyr Tyr Val Leu Lys Gln
   385 390 395 400
   Gln Asp Phe Asp Met Leu Leu Ser Ile Lys His Ala Trp Leu Gly Leu
   405 410 415
   Ile Gln Ala Tyr Leu Val Glu Ala Lys Trp Tyr His Ser Lys Tyr Thr
               420                 425                 430
   Pro Lys Leu Glu Glu Tyr Leu Glu Asn Gly Leu Val Ser Ile Thr Gly
           435                 440                 445
   Pro Leu Ile Ile Thr Ile Ser Tyr Leu Ser Gly Thr Asn Pro Ile Ile
       450                 455                 460
   Lys Lys Glu Leu Glu Phe Leu Glu Ser Asn Pro Asp Ile Val His Trp
   465                 470                 475                 480
   Ser Ser Lys Ile Phe Arg Leu Gln Asp Asp Leu Gly Thr Ser Ser Asp
                   485                 490                 495
   Glu Ile Gln Arg Gly Asp Val Pro Lys Ser Ile Gln Cys Tyr Met His
               500                 505                 510
   Glu Thr Gly Ala Ser Glu Glu Val Ala Arg Glu His Ile Lys Asp Met
           515                 520                 525
   Met Arg Gln Met Trp Lys Lys Val Asn Ala Tyr Thr Ala Asp Lys Asp
       530                 535                 540
   Ser Pro Leu Thr Arg Thr Thr Ala Glu Phe Leu Leu Asn Leu Val Arg
   545                 550                 555                 560
   Met Ser His Phe Met Tyr Leu His Gly Asp Gly His Gly Val Gln Asn
                   565                 570                 575
   Gln Glu Thr Ile Asp Val Gly Phe Thr Leu Leu Phe Gln Pro Ile Pro
               580                 585                 590
   Leu Glu Asp Lys Asp Met Ala Phe Thr Ala Ser Pro Gly Thr Lys Gly
           595                 600                 605
<210> 100
   <211> 1827
   <212> DNA
   <213> Citrus unshiu
<400> 100
   atgtcttctt gcattaatcc ctcaaccttg gctacctctg taaatggttt caaatgtctt      60
   cctcttgcaa caaatagagc agccatcaga atcatggcaa aaaataagcc agtccaatgc     120
   cttgtcagca ccaaatatga taatttgaca gttgatagga gatcagcaaa ctaccaacct     180
   tcaatttggg accatgattt tttgcagtca ctgaatagca actatacgga tgaaacatac     240
   aaaagacgag cagaagagct gaagggaaaa gtgaagacag cgattaagga tgtaaccgag     300
   cctctggatc agttggagct gattgataat ttgcaaagac ttggattggc ttatcatttt     360
   gagcctgaga ttcggaacat attgcgtaat atccacaacc ataataaaga ttataattgg     420
   agaaaagaaa atctgtatgc aacctccctt gaattcagac ttcttagaca acatggctat     480
   cctgtttctc aagaggtttt cagtggtttt aaagacgaca aggtaggctt catttgtgat     540
   gatttcaagg gaatactgag cttgcatgaa gcctcgtatt acagcttaga aggagaaagc     600
   atcatggagg aggcctggca attcaccagt aagcatctta aagaaatgat gatcaccagc     660
   aacagcaagg aagaggatgt atttgtagca gaacaagcga agcgggcgct ggagctccct     720
   ctgcattgga aaaaagtgcc tatgttagag gcaaggtggt tcatacacgt ttatgagaaa     780
   agagaggaca agaaccacct tttacttgag ctcgctaagt tggagtttaa cactttgcag     840
   gcaatttacc aggaagaact taaagacatt tcagggtggt ggaaggatac aggtcttgga     900
   gagaaattga gctttgcgag gaacaggttg gtagcgtcct tcttatggag catggggatc     960
   gcgtttgagc ctcaattcgc ctactgcagg agagtgctca caatctcgat agccctaatt    1020
   acagtgattg atgacattta tgatgtctat ggaacattgg atgaacttga gatattcact    1080
   gatgctgttg cgaggtggga catcaattat gctttgaagc accttccggg ctatatgaaa    1140
   atgtgttttc ttgcccttta caactttgtt aatgaatttg cttattacgt tctcaaacaa    1200
   caggattttg atatgcttct gagcataaaa catgcatggc ttggcttaat acaagcctac    1260
   ttggtggagg cgaaatggta ccatagcaag tacacaccga aactggaaga atacttggaa    1320
   aatggattgg tatcaataac gggcccttta attataacga tttcatatct ttctggtaca    1380
   aatccaatca ttaagaagga actggaattt ctagaaagta atccagatat agttcactgg    1440
   tcatccaaga ttttccgtct gcaagatgat ttgggaactt catcggacga gatacagaga    1500
   ggggatgttc cgaaatcaat ccagtgttac atgcatgaaa ctggtgcctc ggaggaagtt    1560
   gctcgtgaac acatcaagga tatgatgaga cagatgtgga agaaggtgaa tgcatacaca    1620
   gccgataaag actctccctt gactcgaaca actgctgagt tcctcttgaa tcttgtgcga    1680
   atgtcccatt ttatgtatct acatggagat gggcatggtg ttcaaaacca agagactatc    1740
   gatgtcggct ttacattgct ttttcagccc attcccttgg aggacaaaga catggctttc    1800
   acagcatctc ctggcaccaa aggctga                                        1827
<210> 101
   <211> 1677
   <212> DNA
   <213> Artificial Sequence
<220>
   <223> DNA having modified codons, which encodes limonene synthase gene derived from Citrus unshiu (opt_CuLMS)
<400> 101
   atggaccgcc gtagcgccaa ctatcagccg agtatttggg atcatgactt tctgcagagc     60
   ctgaatagta actacaccga tgaaacgtat aaacgccgtg cggaagagct gaaaggcaag    120
   gttaaaaccg ccatcaagga tgtgacggaa ccgctggacc agctggagct gattgataat    180
   ctgcagcgcc tgggcctggc gtatcatttt gaacccgaga ttcgcaatat cctgcgtaac    240
   atccataatc acaacaagga ttacaattgg cgcaaagaaa acctgtatgc cacgagcctg    300
   gagtttcgtc tgctgcgtca gcatggctac cccgtgagcc aggaagtctt tagtggtttt    360
   aaggatgaca aagtcggctt tatctgcgat gactttaaag gtattctgag cctgcatgaa    420
   gcgagctact atagtctgga aggcgagagc atcatggaag aggcctggca gtttaccagt    480
   aagcacctga aggaaatgat gatcacgagc aacagtaaag aagaggacgt ttttgttgcg     540
   gaacaggcca agcgtgccct ggagctgcct ctgcattgga aaaaggtccc gatgctggaa    600
   gcccgctggt ttatccatgt ttatgaaaag cgtgaggata aaaatcacct gctgctggaa    660
   ctggcgaaac tggagtttaa caccctgcag gccatctacc aggaagagct gaaggacatt    720
   agcggttggt ggaaagatac gggcctgggt gaaaagctga gttttgcgcg caatcgtctg    780
   gttgccagct ttctgtggag tatgggcatt gcgtttgaac cgcagtttgc ctactgtcgc    840
   cgtgtgctga ccattagcat cgcgctgatt acggtcatcg atgacattta cgacgtttat    900
   ggtaccctgg atgaactgga gatctttacg gacgccgtgg cgcgctggga tattaactac    960
   gccctgaaac acctgcccgg ctatatgaag atgtgctttc tggcgctgta caattttgtg   1020
   aacgaatttg cctactatgt cctgaaacag caggattttg acatgctgct gagcatcaag   1080
   catgcgtggc tgggcctgat tcaggcgtac ctggtcgaag ccaaatggta ccacagcaag   1140
   tataccccta aactggaaga gtatctggag aatggtctgg ttagcatcac cggccccctg  1200
   attatcacga ttagctacct gagtggcacg aatcctatta tcaaaaagga actggagttt   1260
   ctggaaagca acccggacat cgtgcattgg agcagtaaaa tttttcgcct gcaggatgac   1320
   ctgggtacca gcagtgacga aatccagcgc ggcgatgtcc ccaagagcat tcagtgttac   1380
   atgcatgaaa ccggtgccag tgaagaggtg gcccgtgagc acattaaaga tatgatgcgt   1440
   cagatgtgga aaaaggtcaa tgcgtatacc gccgataaag acagccctct gacccgtacc   1500
   accgccgaat ttctgctgaa tctggttcgt atgagtcact ttatgtacct gcatggcgac   1560
   ggtcacggcg ttcagaacca ggaaaccatc gatgtgggct ttacgctgct gtttcagccg   1620
   attcccctgg aggataaaga catggcgttt accgccagcc cgggtacgaa gggctaa      1677
<210> 102
   <211> 606
   <212> PRT
   <213> Citrus limon
<400> 102
   Met Ser Ser Cys Ile Asn Pro Ser Thr Leu Val Thr Ser Val Asn Ala
   1               5                   10                  15
   Phe Lys Cys Leu Pro Leu Ala Thr Asn Lys Ala Ala Ile Arg Ile Met
               20                  25                  30
   Ala Lys Tyr Lys Pro Val Gln Cys Leu Ile Ser Ala Lys Tyr Asp Asn
           35                  40                  45
   Leu Thr Val Asp Arg Arg Ser Ala Asn Tyr Gln Pro Ser Ile Trp Asp
       50                  55                  60
   His Asp Phe Leu Gln Ser Leu Asn Ser Asn Tyr Thr Asp Glu Ala Tyr
   65                  70                  75                  80
   Lys Arg Arg Ala Glu Glu Leu Arg Gly Lys Val Lys Ile Ala Ile Lys
                   85                  90                  95
   Asp Val Ile Glu Pro Leu Asp Gln Leu Glu Leu Ile Asp Asn Leu Gln
               100                 105                 110
   Arg Leu Gly Leu Ala His Arg Phe Glu Thr Glu Ile Arg Asn Ile Leu
           115                 120                 125
   Asn Asn Ile Tyr Asn Asn Asn Lys Asp Tyr Asn Trp Arg Lys Glu Asn
       130                 135                 140
   Leu Tyr Ala Thr Ser Leu Glu Phe Arg Leu Leu Arg Gln His Gly Tyr
   145                 150                 155                 160
   Pro Val Ser Gln Glu Val Phe Asn Gly Phe Lys Asp Asp Gln Gly Gly
                   165                 170                 175
   Phe Ile Cys Asp Asp Phe Lys Gly Ile Leu Ser Leu His Glu Ala Ser
               180                 185                 190
   Tyr Tyr Ser Leu Glu Gly Glu Ser Ile Met Glu Glu Ala Trp Gln Phe
           195                 200                 205
   Thr Ser Lys His Leu Lys Glu Val Met Ile Ser Lys Asn Met Glu Glu
       210                 215                 220
   Asp Val Phe Val Ala Glu Gln Ala Lys Arg Ala Leu Glu Leu Pro Leu
   225                 230                 235                 240
   His Trp Lys Val Pro Met Leu Glu Ala Arg Trp Phe Ile His Ile Tyr
                   245                 250                 255
   Glu Arg Arg Glu Asp Lys Asn His Leu Leu Leu Glu Leu Ala Lys Met
   260 265 270
   Glu Phe Asn Thr Leu Gln Ala Ile Tyr Gln Glu Glu Leu Lys Glu Ile
   275 280 285
   Ser Gly Trp Trp Lys Asp Thr Gly Leu Gly Glu Lys Leu Ser Phe Ala
   290 295 300
   Arg Asn Arg Leu Val Ala Ser Phe Leu Trp Ser Met Gly Ile Ala Phe
   305 310 315 320
   Glu Pro Gln Phe Ala Tyr Cys Arg Arg Val Leu Thr Ile Ser Ile Ala
   325 330 335
   Leu Ile Thr Val Ile Asp Asp Ile Tyr Asp Val Tyr Gly Thr Leu Asp
   340 345 350
   Glu Leu Glu Ile Phe Thr Asp Ala Val Glu Arg Trp Asp Ile Asn Tyr
   355 360 365
   Ala Leu Lys His Leu Pro Gly Tyr Met Lys Met Cys Phe Leu Ala Leu
   370 375 380
   Tyr Asn Phe Val Asn Glu Phe Ala Tyr Tyr Val Leu Lys Gln Gln Asp
   385 390 395 400
   Phe Asp Leu Leu Leu Ser Ile Lys Asn Ala Trp Leu Gly Leu Ile Gln
   405 410 415
   Ala Tyr Leu Val Glu Ala Lys Trp Tyr His Ser Lys Tyr Thr Pro Lys
   420 425 430
   Leu Glu Glu Tyr Leu Glu Asn Gly Leu Val Ser Ile Thr Gly Pro Leu
   435 440 445
   Ile Ile Thr Ile Ser Tyr Leu Ser Gly Thr Asn Pro Ile Ile Lys Lys
   450 455 460
   Glu Leu Glu Phe Leu Glu Ser Asn Pro Asp Ile Val His Trp Ser Ser
   465 470 475 480
   Lys Ile Phe Arg Leu Gln Asp Asp Leu Gly Thr Ser Ser Asp Glu Ile
   485 490 495
   Gln Arg Gly Asp Val Pro Lys Ser Ile Gln Cys Tyr Met His Glu Thr
   500 505 510
   Gly Ala Ser Glu Glu Val Ala Arg Gln His Ile Lys Asp Met Met Arg
   515 520 525
   Gln Met Trp Lys Lys Val Asn Ala Tyr Thr Ala Asp Lys Asp Ser Pro
   530 535 540
   Leu Thr Gly Thr Thr Thr Glu Phe Leu Leu Asn Leu Val Arg Met Ser
   545 550 555 560
   His Phe Met Tyr Leu His Gly Asp Gly His Gly Val Gln Asn Gln Glu
   565 570 575
   Thr Ile Asp Val Gly Phe Thr Leu Leu Phe Gln Pro Ile Pro Leu Glu
   580 585 590
   Asp Lys His Met Ala Phe Thr Ala Ser Pro Gly Thr Lys Gly
   595 600 605
<210> 103
   <211> 1821
   <212> DNA
   <213> Citrus limon
<400> 103
   atgtcttctt gcattaatcc ctcaaccttg gttacctctg taaatgcttt caaatgtctt     60
   cctcttgcaa caaataaagc agccatcaga atcatggcca aatataagcc agtccaatgc    120
   cttatcagcg ccaaatatga taatttgaca gttgatagga gatcagcaaa ctaccaacct    180
   tcaatttggg accacgattt tttgcagtca ttgaatagca actatacgga tgaagcatac    240
   aaaagacgag cagaagagct gaggggaaaa gtgaagatag cgattaagga tgtaatcgag    300
   cctctggatc agttggagct gattgataac ttgcaaagac ttggattggc tcatcgtttt    360
   gagactgaga ttaggaacat attgaataat atctacaaca ataataaaga ttataattgg    420
   agaaaagaaa atctgtatgc aacctccctt gaattcagac tacttagaca acatggctat    480
   cctgtttctc aagaggtttt caatggtttt aaagacgacc agggaggctt catttgtgat    540
   gatttcaagg gaatactgag cttgcatgaa gcttcgtatt acagcttaga aggagaaagc    600
   atcatggagg aggcctggca atttactagt aaacatctta aagaagtgat gatcagcaag    660
   aacatggaag aggatgtatt tgtagcagaa caagcgaagc gtgcactgga gctccctctg    720
   cattggaaag tgccaatgtt agaggcaagg tggttcatac acatttatga gagaagagag    780
   gacaagaacc accttttact tgagctcgct aagatggagt ttaacacttt gcaggcaatt    840
   taccaggaag aactaaaaga aatttcaggg tggtggaagg atacaggtct tggagagaaa    900
   ttgagctttg cgaggaacag gttggtagcg tccttcttat ggagcatggg gatcgcgttt    960
   gagcctcaat tcgcctactg caggagagtg ctcacaatct cgatagccct aattacagtg   1020
   attgatgaca tttatgatgt ctatggaaca ttggatgaac ttgagatatt cactgatgct   1080
   gttgagaggt gggacatcaa ttatgctttg aagcaccttc cgggctatat gaaaatgtgt   1140
   tttcttgcgc tttacaactt tgttaatgaa tttgcttatt acgttctcaa acaacaggat   1200
   tttgatttgc ttctgagcat aaaaaatgca tggcttggct taatacaagc ctacttggtg   1260
   gaggcgaaat ggtaccatag caagtacaca ccgaaactgg aagaatactt ggaaaatgga   1320
   ttggtatcaa taacgggccc tttaattata acgatttcat atctttctgg tacaaatcca   1380
   atcattaaga aggaactgga atttctagaa agtaatccag atatagttca ctggtcatcc   1440
   aagattttcc gtctgcaaga tgatttggga acttcatcgg acgagataca gagaggggat   1500
   gttccgaaat caatccagtg ttacatgcat gaaactggtg cctcagagga agttgctcgt   1560
   caacacatca aggatatgat gagacagatg tggaagaagg tgaatgcata cacagccgat   1620
   aaagactctc ccttgactgg aacaactact gagttcctct tgaatcttgt gagaatgtcc   1680
   cattttatgt atctacatgg agatgggcat ggtgttcaaa accaagagac tatcgatgtc   1740
   ggttttacat tgctttttca gcccattccc ttggaggaca aacacatggc tttcacagca   1800
   tctcctggca ccaaaggctg a                                             1821
<210> 104
   <211> 1671
   <212> DNA
   <213> Artificial Sequence
<220>
   <223> DNA having modified codons, which encodes limonene synthase gene derived from Citrus limon (opt_ClIMS)
<400> 104
   atggaccgcc gtagcgcgaa ttaccagccc agtatttggg atcatgactt tctgcagagc      60
   ctgaacagta attacacgga cgaagcgtat aagcgccgtg ccgaagagct gcgcggtaaa     120
   gttaagattg cgatcaaaga tgtgatcgaa ccgctggacc agctggagct gattgataac     180
   ctgcagcgcc tgggcctggc ccatcgtttt gaaaccgaga tccgcaacat cctgaacaac     240
   atctacaaca acaacaagga ttacaactgg cgcaaggaaa atctgtacgc cacgagcctg     300
   gagtttcgcc tgctgcgtca gcacggttat cccgtgagtc aggaagtctt taacggcttt     360
   aaggatgacc agggcggttt tatctgcgat gactttaaag gtattctgag cctgcatgaa     420
   gcgagctact atagtctgga aggcgagagt atcatggaag aggcctggca gtttaccagc     480
   aaacacctga aggaagttat gattagtaag aacatggaag aggatgtttt tgtggccgaa     540
   caggcgaagc gtgccctgga gctgcctctg cattggaaag tgccgatgct ggaagcccgc     600
   tggtttattc atatctatga acgccgtgag gacaagaacc acctgctgct ggaactggcg     660
   aaaatggagt ttaatacgct gcaggccatc taccaggaag agctgaaaga aattagcggt     720
   tggtggaagg ataccggcct gggcgagaaa ctgagttttg cgcgcaaccg tctggttgcc     780
   agctttctgt ggagtatggg catcgcgttt gaaccccagt ttgcctactg tcgccgtgtg     840
   ctgacgatta gcatcgcgct gattaccgtc atcgatgaca tttacgacgt ttatggtacg     900
   ctggatgaac tggagatctt taccgacgcg gttgaacgct gggatattaa ttacgccctg     960
   aagcacctgc ctggctatat gaaaatgtgc tttctggcgc tgtacaactt tgtgaatgaa    1020
   tttgcctact atgtcctgaa gcagcaggat tttgacctgc tgctgagcat caaaaacgcc    1080
   tggctgggcc tgattcaggc gtacctggtc gaagccaaat ggtaccatag caaatatacc    1140
   cctaagctgg aagagtatct ggaaaatggt ctggttagca tcacgggccc cctgattatc    1200
   accattagct acctgagtgg cacgaaccct attatcaaaa aggaactgga gtttctggaa    1260
   agcaatccgg acatcgtgca ctggagcagt aagatttttc gtctgcagga tgacctgggt    1320
   acgagcagtg acgaaatcca gcgcggcgat gtccctaaaa gcattcagtg ttacatgcat    1380
   gagacgggtg ccagtgaaga ggtggcccgt cagcacatta aggatatgat gcgccagatg    1440
   tggaaaaagg tcaatgcgta tacggccgat aaagacagcc cgctgaccgg caccacgacc    1500
   gaatttctgc tgaacctggt gcgcatgagc cattttatgt acctgcatgg cgacggtcac    1560
   ggcgttcaga atcaggaaac gatcgatgtg ggctttaccc tgctgtttca gccgattccc    1620
   ctggaggata agcacatggc gtttacggcc agcccgggta ccaaaggcta a             1671
<210> 105
   <211> 67
   <212> DNA
   <213> Artificial Sequence
<220>
   <223> primer for amplifying opt_PsLMS
<400> 105
   gtgtggaatc gtgagcggat aacaatttca cacaaggaga ctgccatgca gcgccgtcgc 60
   ggcaatt 67
<210> 106
   <211> 36
   <212> DNA
   <213> Artificial Sequence
<220>
   <223> primer for amplifying opt_PsLMS
<400> 106
   gtctcctgtg tgaaattaca gcgtcacggg ttccag 36
<210> 107
   <211> 75
   <212> DNA
   <213> Artificial Sequence
<220>
   <223> primer for amplifying tac promoter region
<400> 107
   cgttgttgcc attgctgcac cctgttgaca attaatcatc ggctcgtata atgtgtggaa 60
   tcgtgagcgg ataac 75
<210> 108
   <211> 23
   <212> DNA
   <213> Artificial Sequence
<220>
   <223> primer for amplifying ispA*
<400> 108
   tttcacacag gagactgcca tgg 23
<210> 109
   <211> 42
   <212> DNA
   <213> Artificial Sequence
<220>
   <223> primer for amplifying ispA*
<400> 109
   atgacttggt tgagtctatt tgttgcgctg gatgatgtaa tc 42
<210> 110
   <211> 67
   <212> DNA
   <213> Artificial Sequence
<220>
   <223> primer for amplifying opt_AgLMS
<400> 110
   gtgtggaatc gtgagcggat aacaatttca cacaaggaga ctgccatgca gcgtcgcatc 60
   gcggatc 67
<210> 111
   <211> 34
   <212> DNA
   <213> Artificial Sequence
<220>
   <223> primer for amplifying opt_AgLMS
<400> 111
   gtctcctgtg tgaaattaca gcgccagggg ttcc 34
<210> 112
   <211> 72
   <212> DNA
   <213> Artificial Sequence
<220>
   <223> primer for amplifying opt_MsLMS
<400> 112
   gtgtggaatc gtgagcggat aacaatttca cacaaggaga ctgccatgga acgccgtagc 60
   ggcaattata ac 72
<210> 113
   <211> 38
   <212> DNA
   <213> Artificial Sequence
<220>
   <223> primer for amplifying opt_MsLMS
<400> 113
   gtctcctgtg tgaaattagg caaacggttc aaacagcg 38
<210> 114
   <211> 66
   <212> DNA
   <213> Artificial Sequence
<220>
   <223> primer for amplifying opt_CuLMS
<400> 114
   gtgtggaatc gtgagcggat aacaatttca cacaaggaga ctgccatgga ccgccgtagc 60
   gccaac 66
<210> 115
   <211> 35
   <212> DNA
   <213> Artificial Sequence
<220>
   <223> primer for amplifying opt_CuLMS
<400> 115
   gtctcctgtg tgaaattagc ccttcgtacc cgggc 35
<210> 116
   <211> 69
   <212> DNA
   <213> Artificial Sequence
<220>
   <223> primer for amplifying opt_ClIMS
<400> 116
   gtgtggaatc gtgagcggat aacaatttca cacaaggaga ctgccatgga ccgccgtagc 60
   gcgaattac 69
<210> 117
   <211> 35
   <212> DNA
   <213> Artificial Sequence
<220>
   <223> primer for amplifying opt_ClLMS
<400> 117
   gtctcctgtg tgaaattagc ctttggtacc cgggc 35